(19) Europäisches Patentamt
European Patent Office
Office européen des brevets

(11) Publication number: **0 299 533 B1**

(12) # EUROPEAN PATENT SPECIFICATION

(45) Date of publication of patent specification: **07.04.93**

(51) Int. Cl.5: **C07D 257/04**, C07D 257/06, C07D 405/12, C07D 409/12, C07D 403/12, A23L 1/236

(21) Application number: **88111508.3**

(22) Date of filing: **18.07.88**

(54) **High potency sweetening agents.**

(30) Priority: **17.07.87 US 74742**
**02.10.87 US 104620**
**12.07.88 US 216738**

(43) Date of publication of application:
**18.01.89 Bulletin 89/03**

(45) Publication of the grant of the patent:
**07.04.93 Bulletin 93/14**

(84) Designated Contracting States:
**AT BE CH DE ES FR GB GR IT LI LU NL SE**

(56) References cited:
**EP-A- 0 034 925**
**EP-A- 0 107 597**
**EP-A- 0 195 730**
**GB-A- 1 587 258**
**US-A- 3 294 551**
**US-A- 3 615 700**
**US-A- 4 673 582**
**US-A- 4 680 300**

**CHEMICAL AND PHARMACEUTICAL BULLETIN, vol. 32, no. 12, 1984, pages 4893-4906; K. SHIMADA et al.: "Synthesis and gastric antisecretory activity of N-cyano-N-(phenyl-pyridinylmethyl)guanidine derivatives"**

(73) Proprietor: **THE NUTRASWEET COMPANY (a Delaware corporation)**
**1751 Lake Cook Road**
**Deerfield Illinois 60015(US)**

(72) Inventor: **Mazur, Robert**
**523 Maple Avenue**
**Wilmette/Cook, IL 60091(US)**
Inventor: **Owens, William H.**
**1214 East Valley Lane**
**Arlington Heights/Cook, IL 60004(US)**
Inventor: **Klade, Carrie Ann**
**4544 North Sayre, Apt. 2C,**
**Norridge/Cook, IL 60656(US)**
Inventor: **Madigan, Darold**
**908 Wisconsin Avenue**
**Elk Grove Village/Cook, IL 60007(US)**
Inventor: **Muller, George W.**
**1915 Smith Road**
**Northbrook/Cook, IL 60062(US)**

(74) Representative: **Beil, Hans Chr., Dr. et al**
**Beil, Wolff und Beil, Rechtsanwälte Adelonstrasse 58 Postfach 80 01 40**
**W-6230 Frankfurt am Main 80 (DE)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

EP 0 299 533 B1

**JOURNAL OF ORGANIC CHEMISTRY, vol. 51, 1986, pages 1882-1884; C.A. MARYANOFF et al.: "A convenient synthesis of guanidines from thioureas"**

**CHEMICAL ABSTRACTS, vol. 63, no. 4, 16 August 1965, columns 4281h-4282a, Columbus, Ohio, US; M. TREMBLAY: "Infrared spectra of nitrogen-containing compounds"**

## Description

### CROSS-REFERENCE TO RELATED APPLICATION

This application is a Continuation-In-Part (CIP) of Serial No. 104,620 filed October 2, 1987, which was a CIP of Serial No. 074,742 filed 17 July 1987 which is incorporated herein by reference.

### BACKGROUND

The present invention relates to novel guanidine compounds useful as sweetening agents. Additionally, the present invention relates to sweetening compositions, food products containing the present guanidines, methods of sweetening foods, novel intermediates and methods of preparing the novel guanidines.

Certain guanidine derivatives are known in the art as sweeteners. See, for example, Yuki and Inoue (Nippon Kagaku Kaishi, no. 11, 2140-43 (1974)), Chemical Abstracts, Vol. 82, no. 140061p (1975) describes N-((4-chlorophenylamino)iminomethyl)-$\beta$-alanine (Chemical Substance Index, vol. 76-85, 1972-1976, p. 1067cs), European Patent Application 0,107,597 published May 2, 1984 (U.S. Patent No. 4,645,678), U.S. Patent 4,673,582 and European Patent Application 0,195,730 published September 24, 1986. A problem associated with prior art guanidine sweeteners, especially glycine derivatives, is their lack of stability due to cyclization.

U.S. Patent 3,615,700 discloses $\alpha$-tetrazolyl 6 (and 5,6) substituted tryptamine compounds wherein the d-enantiomers are useful as nonnutritive sweeteners.

U.S. Patent 4,680,300 discloses methods of preparing guanidines which are useful as anti-inflammatory agents.

It has been discovered that the present guanidines are more stable than prior art compounds which have a carboxyl moiety in place of the present tetrazole moiety. The presence of the tetrazole moiety prevents cyclization of the molecule which occurs in the prior art molecules having a carboxyl moiety in the tetrazole position.

### SUMMARY OF THE INVENTION

Briefly, in accordance with the present invention, substituted guanidines, containing a tetrazole moiety, are useful as sweetening agents. The present guanidines are added to food products in amounts sufficient to sweeten the food to a desired level. Typical food products include soft drinks, juices, condiments, candies, baked goods, chewing gum and pharmaceuticals.

The present guanidines are prepared by reacting a 5-substituted tetrazole with an isothiourea or carbodiimide intermediate. The resulting product is recovered and used in food applications to replace sucrose. The present guanidines can be combined with other sweeteners and bulking agents.

Of particular interest in the practice of the present invention, (1) 1′-N-[N-cyclooctylamino-(4-cyanophenylimino)methyl]-5-aminomethyltetrazole, (2) 1′-N-[N-1-naphthylamino-(4-cyanophenylimino)-methyl]-5-aminomethyltetrazole, (3) 1′-N-[N-benzenesulfonylamino-(4-cyanophenylimino)methyl]-5-aminomethyltetrazole, (4) 1′-N-[N-(S)-phenethylamino-(3,5-dimethylphenylimino) methyl]-5-aminomethyltetrazole, (5) 1′-N-[N-(S)-phenethylamino-(3,5-dichlorophenylimino)methyl]-5-aminomethyltetrazole, (6) 1′-N-[N-endo-norbornylamino(4-cyanophenylimino)methyl]-5-aminomethyl tetrazole, (7) 1′-N-[N′-2-Aminodecalyl-(4-Cyanophenylimino)methyl]-5-Aminomethyltetrazole, (8) 1′-N-[N′-(R, S-$\alpha$-Cyclohexylbenzyl)(4-Cyanophenylimino)methyl]5-Aminomethyltetrazole, (9) 1′-N-[N′-Benzhydryl(4-Cyanophenylimino)methyl]5-Aminomethyltetrazole or mixtures of these compounds are employed as a sweetening agent in foods especially in carbonated soft drinks.

### BRIEF DESCRIPTION OF DRAWING

Figure 1 illustrates the stability (90° C) of Compound 1 and the stability (70° C) of the glycine analog of Compound 1 as described in Example 43.

DETAILED DESCRIPTION OF THE INVENTION

The present substituted guanidines are represented by the following formula:

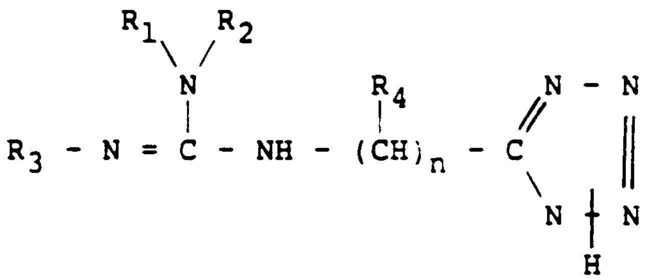 (I)

wherein $R_3$ is an optionally substituted heterocyclic moiety, as defined in the passage immediately after the next definition of $R^4$, or an optionally substituted phenyl of the formula

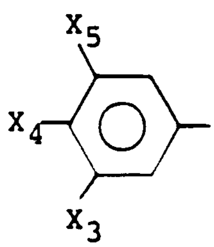

wherein $X_3$, $X_4$ and $X_5$ are the same or different and are selected from the group consisting of

H,
Br,
$CF_3$,
$CF_2CF_3$,
$CH_2CF_3$,
$C_1$-$C_4$ alkyl,
$CH=NOCH_3$,
$CH=NOH$,
CHO,
$CH_2OCH_3$,
$CH_2OH$,
Cl,
CN,
$COCF_3$,
$COC_1$-$C_3$ alkyl,
$CONH_2$,
$CONHC_1$-$C_3$ alkyl,
$CON(C_1$-$C_3$ alkyl$)_2$,
$COOC_1$-$C_3$ alkyl,
COOH,
F,
I,
$NH_2$,
$NHC_1$-$C_3$ alkyl,
$N(C_1$-$C_3$ alkyl$)_2$,
NHCHO,
$NHCOCH_3$,
$NHCONH_2$,
$NHSO_2CH_3$,
$NO_2$,

$OC_1$-$C_3$ alkyl,
$OCOCH_3$,
OH,
$SC_1$-$C_3$ alkyl,
$SOC_1$-$C_3$ alkyl,
$SO_2C_1$-$C_3$ alkyl,
$SO_2NH_2$,
$SO_2NHC_1$-$C_3$ alkyl,
$SO_2N(C_1$-$C_3$ alkyl$)_2$, and
$SO_3H$,

or where substituents at $X_4$ and $X_5$ form a fused ring;
wherein $R_1$ is hydrogen, a $C_1$ to $C_4$ saturated, unsaturated, acyclic, cyclic or mixed hydrocarbyl or modified hydrocarbyl group and wherein, in the modified hydrocarbyl group,

1 to 2 atoms of carbon may be replaced by 1 to 2 of the same or different heteroatoms selected from the group consisting of N, O, S, Cl, Br and I,

and 1 to 3 atoms of hydrogen may be replaced by 1 to 3 atoms of fluorine, oxygen, or nitrogen;

wherein $R_2$ is hydrogen, CN, $NO_2$, a $C_1$ to $C_{20}$ saturated, unsaturated, acyclic, cyclic or mixed hydrocarbyl or modified hydrocarbyl group and wherein, in the modified hydrocarbyl group,

1 to 4 atoms of carbon may be replaced by 1 to 4 of the same or different heteroatoms selected from the group consisting of N, O, S, Cl, Br and I,

and 1 to 5 atoms of hydrogen may be replaced by 1 to 5 atoms of fluorine, oxygen, or nitrogen;

wherein $R_1$ and $R_2$ can be fused;
n is 0 or 1;
$R_4$ is H or $C_1$-$C_6$ alkyl with proviso that $R_4$ can only be alkyl on a single carbon atom when n = 2 or 3.

Suitable heterocyclic moieties for $R_3$ are optionally substituted pyridines, thiazoles, indoles, quinolines, naphthyridines, cinnolines, pteridines, thiophenes, benzothiophenes, naphthothiophenes, thianthrenes, furans, pyrans, isobenzofurans, chromenes, xanthenes, phenoxathins, pyrroles, indoles, isoindoles, indolizines, pyridazines, pyrimidines, pyrazines, pyrazoles, imidazoles, pyrroles, indazoles, purines, quinolizines, isoquinolines, quinolines, phthalazines, quinoxalines, quinazolines, carbazoles, carbolines, phenanthridines, acridines, pyrimidines, phenanthrolines, phenazines, phenarsazines, isothiazoles, phenothiazines, isoxazoles, thiazoles, furazans and heterocyclics of the following formulas:

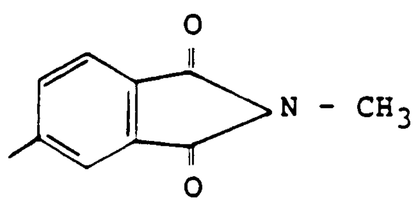

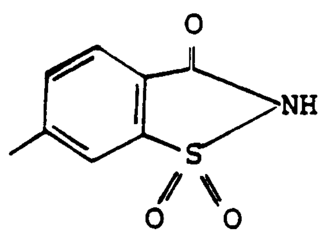

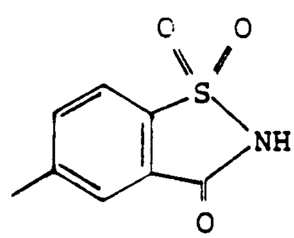

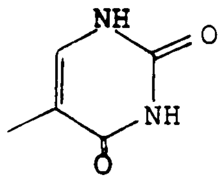

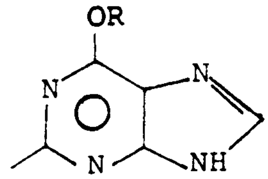

wherein R is H or $C_1$-$C_6$ alkyl. The heterocyclic moieties may be optionally substituted with one or more substituents, such as, for example, $C_1$-$C_6$ alkyl, halogen, $NO_2$, CN, trihalomethyl, dihalomethyl, hydroxyl or hydroxyalkyl.

The present guanidines include tautomeric forms and physiologically acceptable salts of the compounds of Formula I, above. Preferred compounds include those wherein $X_3$, $X_5$ and $R_4$ are hydrogen, n equals 1, and $X_4$ is CN or $NO_2$.

It should be considered that by the term "modified hydrocarbyl" it is contemplated, as one example, that where $R_2$ is a $C_2$ modified hydrocarbyl group that one atom of carbon may be replaced by an atom of nitrogen so that the $C_2$ hydrocarbyl moiety may be replaced by the -CN cyano moiety. As a further example, it is contemplated that where $R_2$ is a $C_3$ modified hydrocarbyl group that one atom of carbon may be replaced by a nitrogen atom and two atoms of carbon may be replaced by two atoms of oxygen so that a $C_3$ hydrocarbyl moiety may be replaced by the $-NO_2$ nitro moiety. Other replacements of hydrocarbyl carbons by the same or different heteroatoms selected from the group consisting of N, O, S, Cl, Br, and I are similarly contemplated by the invention.

Preferably, the present guanidine is one wherein $R_3$ is a substituted phenyl and $R_1$ is H or $CH_3$. Preferred guanidines also include those wherein (a) $X_3$ and $X_5$ are selected from the group consisting of H, Br, $CF_3$, $CH_3$, Cl and F and (b) $X_4$ is H or CN. Also preferred are guanidines wherein $X_3$ and $X_4$ form a fused ring, such as 6-Indazolyl.

Preferred $R_2$ substituents include
normal alk(en) (yn)yl $C_2$-$C_{13}$,
branched alk(en) (yn)yl $C_3$-$C_{13}$,
cycloalk(en)yl $C_3$-$C_{13}$,
alk(en)yl cycloalk(en)yl $C_4$-$C_{13}$,
cycloalk(en)yl alk(en)yl $C_4$-$C_{13}$,
alk(en)yl cycloalk(en)yl alk(en)yl $C_5$-$C_{13}$,
alk(en)yl bicycloalk(en)yl $C_7$-$C_{13}$,
fused bicycloalk(en)yl $C_7$-$C_{13}$,
alk(en)yl fused bicycloalk(en)yl $C_8$-$C_{13}$,
fused bicycloalk(en)yl alk(en)yl $C_8$-$C_{13}$,
alkenyl fused bicycloalk(en)yl alk(en)yl $C_9$-$C_{13}$,
fused tricycloalk(en)yl $C_{10}$-$C_{13}$,
alk(en)yl fused tricycloalk(en)yl $C_{11}$-$C_{13}$,
fused tricycloalk(en)yl alk(en)yl $C_{11}$-$C_{13}$ or
alk(en)yl fused tricycloalk(en)yl alk(en)yl $C_{11}$-$C_{13}$.

Particularly preferred are those guanidines wherein $R_2$ is selected from the group consisting of cyclooctyl, benzyl, cyclononyl, phenyl, alpha-phenethyl, cycloheptyl, cyclohexyl, benzhydryl, tetralyl, and decalyl.

Preferred guanidines also include those listed above wherein $R_2$ is a modified hydrocarbyl group wherein up to four carbon atoms may be replaced by the same or different heteroatoms selected from a group consisting of S to replace C or $CH_2$, N to replace CH, NH and O to replace $CH_2$ and Cl, Br and I to replace $CH_3$ and wherein up to 5 atoms of hydrogen may be substituted by fluorine atoms. Also preferred are those sweetening agents wherein $R_2$ is selected from the group consisting of $CH(CH_3)C_6H_5$, alkyl substituted S-phenylethyl, diphenylmethyl, pyridinyl, pyridinyl methyl, piperidyl, homopiperidyl, indolyl, indolinyl, isoindolinyl, quinolyl, isoquinolyl, pyrazinyl, pyrimidyl, indazolyl, quinoxalinyl, quinazolinyl, purinyl, $OCH_2C_6H_5$, pyranyl, tetrahydropyranyl, benzofuranyl, methoxyphenyl, methyloxycarbonylphenyl, 3,4-methylenedioxyphenyl, morpholinyl, benzoxazolyl, acetamidophenyl, cyano, nitro, thienyl, thienyl methyl, tetrahydro-3-thiophene, endo-norbornyl, exo-norbornyl, benzothienyl, 2,2,4,4-tetramethylthiocyclobut-3-yl, thiazolyl, isothiazolyl, $SO_2C_6H_5$, alkyl substituted $-SO_2C_6H_5$ ($SO_2C_6H_2$(2,4,6-trimethyl), $SO_2C_6H_2$(2,4,6-triisopropyl)), $SO_2$c-$C_6H_{11}$, $SO_2$c-$C_7H_{13}$, 6-oxo-cis-hydrindane, chlorophenyl, fluorophenyl, and trifluoromethylphenyl.

Other preferred compounds include those having the formula

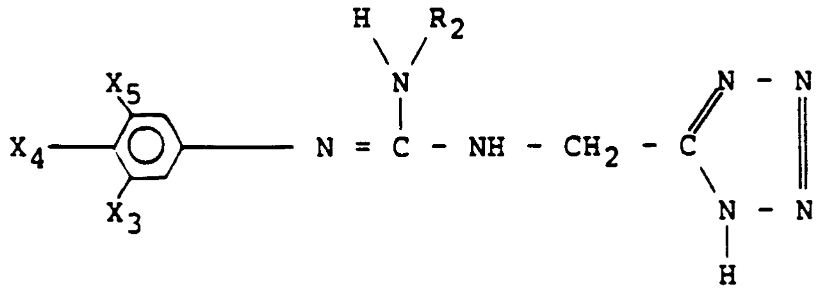

(II)

wherein $X_3$, $X_4$, $X_5$ and $R_2$ are as described above. Particularly preferred compounds are those of Formula II wherein $X_3$ and $X_5$ are H and $X_4$ is CN or wherein $X_3$ and $X_5$ are selected from the group consisting of $CF_3$, $CH_3$, Cl and F and wherein $X_4$ is selected from the group consisting of H and CN.

Most preferred are such compounds wherein $R_1$ is selected from the group consisting of H and $CH_3$; $R_3$ is a substituted phenyl wherein $X_3$ and $X_5$ are H and $X_4$ is CN or wherein $X_3$ and $X_5$ are selected from the group consisting of $CF_3$, $CH_3$, Cl, H and F and wherein $X_4$ is selected from the group consisting of H and CN; and wherein $R_2$ is selected from the group consisting of $CH(CH_3)C_6H_5$, $CH_2C_6H_5$, $CH(CH_3)$-c-$C_6H_{11}$, $CH(C_6H_5)_2$, c-$C_6H_{11}$, c-$C_7H_{13}$, endo-norbornyl, c-$C_8H_{15}$, c-$C_9H_{17}$, c-$C_{10}H_{17}$, c-$C_{10}H_{19}$, $SO_2C_6H_5$ and $SO_2C_7H_{13}$. Specifically preferred compounds include 1'-N-[N-cyclononylamino(4-cyanophenylimino)-methyl]-5-aminomethyltetrazole ($R_1$, $X_3$, $R_4$, $R_5$ and $X_5$ are H; $X_4$ is CN and $R_2$ is c-$C_9H_{17}$); 1'-N-[N-cyclooctylamino(4-cyanophenylimino)methyl]-5-aminomethyltetrazole ($R_1$, $X_3$, $R_4$, $R_5$ and $X_5$ are H, $X_4$ is CN and $R_2$ is c-$C_8H_{15}$); 1'-N-(N-1-naphthylamino-(4-cyanophenylimino)methyl]-5-aminomethyltetrazole ($R_1$, $R_4$, $R_5$, $X_3$ and $X_5$ are H, $X_4$ is CN and $R_2$ is naphthyl); 1'-N-[N-benzenesulfonylamino-(4-cyanophenylimino)methyl]-5-aminomethyltetrazole ($R_1$, $R_4$, $R_5$, $X_3$ and $X_5$ are H, $X_4$ is CN and $R_2$ is $SO_2C_6H_5$); 1'-N-[N-(S)-phenethylamino-(3,5-dimethylphenylimino)methyl]-5-aminomethyltetrazole ($R_1$, $R_4$, $R_5$ and $X_4$ are H, $X_3$ and $X_5$ are $CH_3$ and $R_2$ is (S)($CH(CH_3)C_6H_5$); 1'-N-[N-(S)-phenethylamino-(3,5-dichlorophenylimino)methyl]-5-aminomethyltetrazole ($R_1$, $R_4$, $R_5$ and $X_4$ are H, $X_3$ and $X_5$ are Cl and $R_2$ is (S)($CH(CH_3)C_6H_5$); 1'-N-[N-endo-norbornylamino-(4-cyanophenylimino)methyl]-5-aminomethyltetrazole ($R_1$, $R_4$, $R_5$, $X_3$ and $X_5$ are H, $X_4$ is CN and $R_2$ is endo-norbornyl); 1'-N-[N-cyclooctylamino(3-chloro-4-cyanophenylimino) methyl]-5-aminomethyltetrazole ($R_1$, $R_4$, $R_5$ and $X_5$ are H, $X_3$ is Cl, $X_4$ is CN and $R_2$ is c-$C_8H_{15}$); 1'-N-(N-cyclooctylamino(4-cyano-3-methylphenylimino)methyl]-5-aminomethyltetrazole ($R_1$, $R_4$, $R_5$ and $X_5$ are H, $X_3$ is $CH_3$, $X_4$ is CN and $R_2$ is c-$C_8H_{15}$); 1'-N-[N-benzylamino(3,5-dichlorophenylimino)-methyl]-5-aminomethyltetrazole ($R_1$, $R_4$, $R_5$ and $X_4$ are H, $X_3$ and $X_5$ are Cl and $R_2$ is $CH_2C_6H_5$); 1'-N-(N-methyl-N-benzylamino (3,5-dichlorophenylimino)methyl]-5-aminomethyltetrazole ($R_1$ is $CH_3$, $X_4$ , $R_4$, $R_5$ are H, $X_3$ and $X_5$ are Cl and $R_2$ is $CH_2C_6H_5$); 1'-N-[N-(S)-1-cyclohexylethylamino(3,5-dichlorophenylimino)-methyl]-5-aminomethyltetrazole ($R_1$, $R_4$, $R_5$ and $X_4$ are H, $X_3$ and $X_5$ are Cl and $R_2$ is (S)$CH(CH_3)$-c-$C_6H_{11}$); 1'-N-[N-(S)-$\alpha$-methylbenzylamino(4-cyanophenylimino)methyl]-5-aminomethyltetrazole ($R_1$, $X_3$, $R_4$, $R_5$ and $X_5$ are H, $X_4$ is CN and $R_2$ is (S)$CH(CH_3)C_6H_5$) and 1'-N-[N-cycloheptylamino(3,5-dichlorophenylimino) methyl]-5-aminomethyltetrazole ($R_1$, $R_4$, $R_5$ and $X_4$ are H, $X_3$ and $X_5$ are Cl and $R_2$ is c-$C_7H_{13}$).

Preferred compounds are those wherein $X_4$ is selected from H, CN, C(O)$OCH_3$, F and $NO_2$, as well as those compounds wherein $R_2$ is selected from n-$C_5H_{11}$, n-$C_6H_{13}$, n-$C_7H_{15}$, $CH(CH_3)(CH_2)_2CH_3$, $CH(CH_3)$-$(CH_2)_3CH_3$, $(CH_2)_3CH(CH_3)_2$, $(CH_2)_4CH(CH_3)_2$, $(CH_2)_5CH(CH_3)_2$, $C_6H_5$, $C_6$-$C_{10}$-cycloalkyl, $(CH_3(CH_2)_3)C_6$-$C_{16}$ cycloalkyl,

$CH_2C_6H_5$, $CH_2$-c-$C_6H_{11}$, $CH(CH_3)C_6H_5$, $CH(C_6H_5)C_6H_5$, $CH(CH_3)$-c-$C_6H_{11}$, $(CH_2)_2C_6H_5$, $(CH_2)_2$-c-$C_6H_{11}$, $CH(CH_3)CH_2C_6H_5$, $CH(CH_3)CH_2$-c-$C_6H_{11}$,

$C_6H_4(CH_3)$, $C_6$-$C_{10}$ cycloalkyl($CH_3$),

$CH_2C_6H_4(CH_3)$, $CH_2$-c-$C_6H_{10}(CH_3)$, $CH(CH_3)C_6H_4(CH_3)$, $CH(CH_3)$-c-$C_6H_{10}(CH_3)$,

$(CH_2)_2CH(c\text{-}C_3H_5)_2$, $(CH_2)_3CH(c\text{-}C_3H_5)_2$, $CH(CH_3)CH_2CH(c\text{-}C_3H_5)_2$, $CH(CH_3)(CH_2)_2CH(c\text{-}C_3H_5)_2$,

naphthyl, 5,6,7,8-tetrahydronaphthyl, perhydronaphthyl, indenyl, indanyl,

naphthyl($CH_3$), 5,6,7,8-tetrahydronaphthyl($CH_3$), perhydronaphthyl($CH_3$), indenyl($CH_3$), indanyl($CH_3$), fenchyl,

$CH_2$-naphthyl, $CH_2$-5,6,7,8-tetrahydronaphthyl, $CH_2$-perhydronaphthyl, $CH_2$-indenyl, $CH_2$-indanyl,

$CH_2$-naphthyl($CH_3$), $CH_2$-5,6,7,8-tetrahydronaphthyl($CH_3$), $CH_2$-perhydronaphthyl($CH_3$), $CH_2$-indenyl-($CH_3$), $CH_2$-indanyl($CH_3$),

adamantyl, endo-norbornyl, exo-norbornyl,

$CH_2$-adamantyl, $CH(CH_3)$adamantyl, tetralyl, decalyl, and $CH_2$-adamantyl($CH_3$).

A further preferred compound is that of the formula

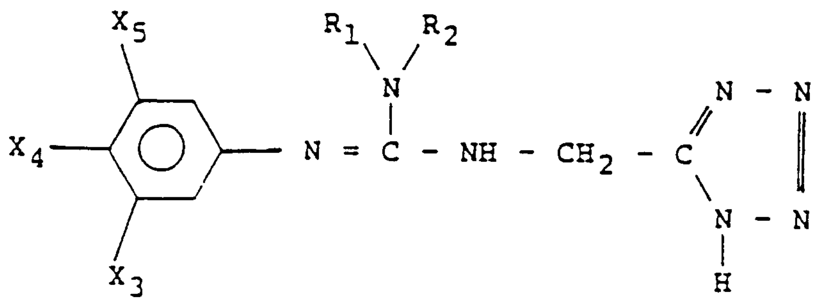

especially that wherein $R_1$ in the above formula is $CH_3$.

A further preferred compound is that wherein $R_3$ is 4-cyanophenyl, 3,5-dichlorophenyl, 3,5-dimethylphenyl or phenyl, $R_4$ is H and which has the formula

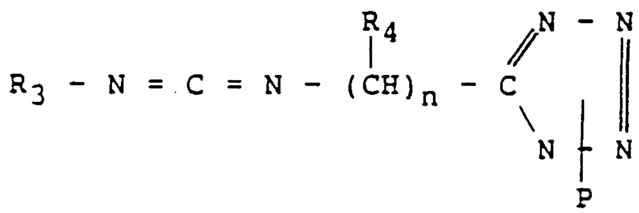

and wherein P is H,

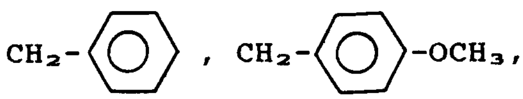

$CH_2OCO$-t-$C_4H_9$ or $CH_2CH_2COOC_2H_5$.

Furthermore, the compounds of the formula

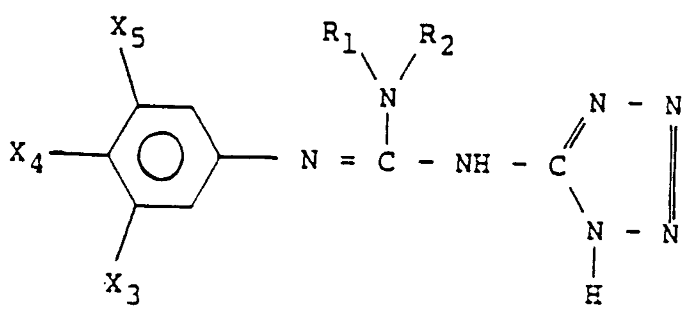

are preferred especially those wherein $R_1$ is H, $X_3$ and $X_5$ being H and $X_4$ being CN, or wherein $R_2$ is phenyl, benzyl, cyclohexyl, cyclooctyl, cyclononyl, 4-pyranyl, 2-decalyl, endo-norbornyl, 1-naphthyl, alpha-phenethyl or benzhydryl or the compounds of the above formula wherein $X_4$ is H and $X_3$ and $X_5$ are Cl or $CH_3$ and wherein $R_2$ may further be of the meaning as just indicated above.

The present invention also includes physiologically acceptable salts of the presented guanidines, i.e., sulfate, phosphate, citrate, hydrochloride, sodium, potassium, ammonium, calcium and magnesium salts.

Additionally, the present invention relates to edible products containing the present guanidine compounds as sweetening agents either alone or in combination with other sweeteners such as carbohydrate sweeteners or high potency sweeteners. Also provided by the present invention is a process for sweetening edible products such as foods, beverages, candies, chewing gums, sweets, pharmaceuticals and veterinary preparations.

The present invention further contemplates compositions of the present guanidines in combination with other sweetening agents and/or a physiologically acceptable carrier which may be a bulking agent. Suitable carriers include polydextrose, starch, maltodextrins, cellulose, methylcellulose, maltitol, carboxymethylcellulose, hydroxymethylcellulose, microcrystalline cellulose, sodium alginate, pectins, gums, lactose, maltose, glucose, leucine, glycerol, mannitol, sorbitol, sodium bicarbonate and phosphoric, citric, tartaric, fumaric, benzoic, sorbic, propionic acids and their sodium, potassium and calcium salts and mixtures of all of the above.

Suitable sweetening agents which may be used in combination with the present guanidines can be sugar or high potency sweeteners such as sucrose, corn syrups, fructose, high fructose corn syrup, aspartame, alitame, neohesperidine dihydrochalcone, hydrogenated isomaltulose, stevioside, L-sugars, glycyrrhizin, xylitol, acesulfam-K, saccharin (sodium, potassium or calcium salt), cyclamic acid (sodium, potassium or calcium salt), trichlorogalactosucrose (TGS or sucralose), monellin and thaumatin and mixtures thereof.

The present invention also relates to a method of preparing the tetrazole containing guanidine compounds. The substituted guanidines of the present invention are prepared employing synthetic methods which are analogous to known methods disclosed in the literature. These known methods have been summarized in a recent paper by Maryanoff (C. Maryanoff, R.C. Stanzione, J.N. Plampin, and J.E. Mills, J. Org. Chem. 1986, 51, 1882-1884). The techniques are further described in J. Med. Chem., 1978, no. 21, pp. 773-781; Chem. Ber. 1966, 99, 1252-157; U.K. patent 1587 258; J. Org. Chem., 1970, 35, pp. 2067-2069; Chem. Ber. 1967, 100, pp. 591-604; J. fur Prakt. Chem. 1977, 319, pp. 149-157; and The Chemistry of Amidines and Imidates, S. Patai, ed. Wiley-Interscience 1975, pp. 283-348. Two general techniques are particularly useful. The first involves formation of an isothiourea intermediate while the second involves transformation of a carbodiimide intermediate. Either of these intermediates are reacted with a 5-substituted tetrazole resulting in formation of the present guanidines. The preparation of 5-substituted tetrazoles is described by Grzonka and Liberek in Roczniki Chemii, Ann. Soc. Chim. Polonorum, 45, 967-986 (1971).

In general, a reactive intermediate, with an easily liberated activating moiety (leaving group), designated by the letter L in the following reactants, can be employed wherein the intermediate contains appropriately substituted amine groups corresponding to the substituents present on the guanidine nitrogens. The leaving groups are preferably selected from the group consisting of S-alkyl (isothiourea), O-alkyl, $OSO_2$-aryl, $SO_3H$ and halogen moieties. A protecting group designated by the letter P will be attached to the tetrazole. Preferred protecting groups have the formulas

H,

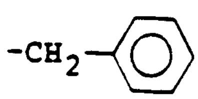 ,

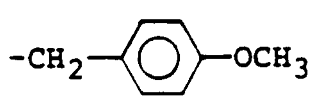 ,

$CH_2OCO\text{-}t\text{-}C_4H_9$,
or $-CH_2CH_2COOC_2H_5$.

As a general principle, a reaction is brought about between the reactant intermediate and the appropriate complementary amine, that is to say, the following compounds are brought into contact with one another:

(1) 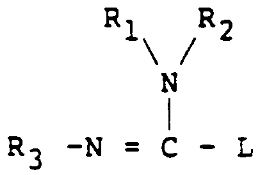 and 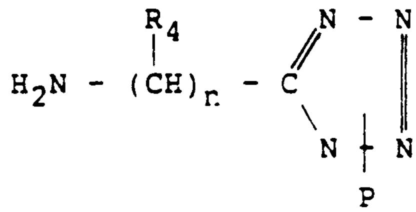

(2) $R_3 - NH_2$ and 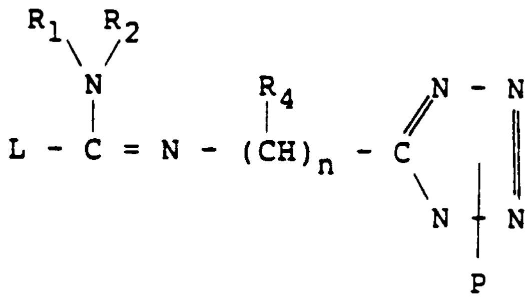

(3) 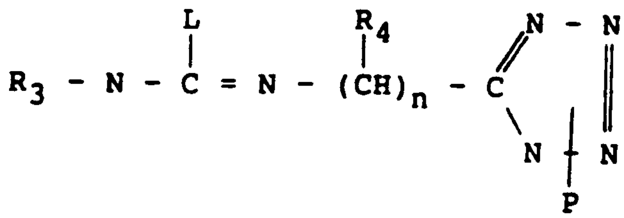 and 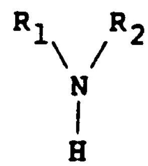

The following compounds are brought into contact with one another in the carbodiimide method:

(4) 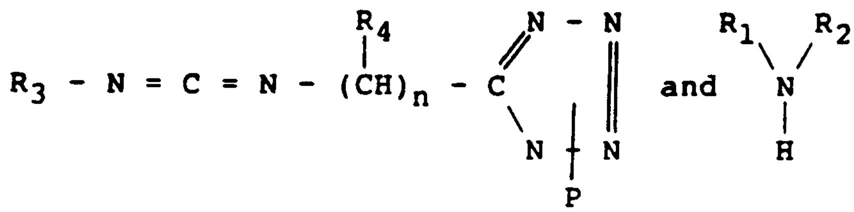
and

(5) 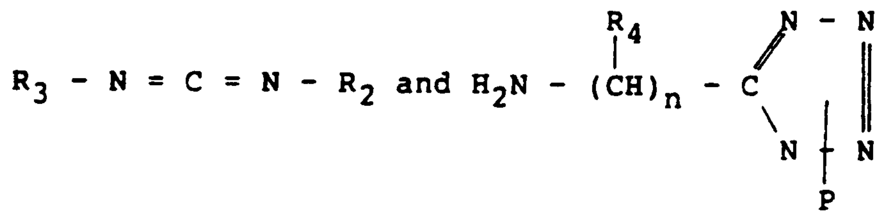
and

(6) 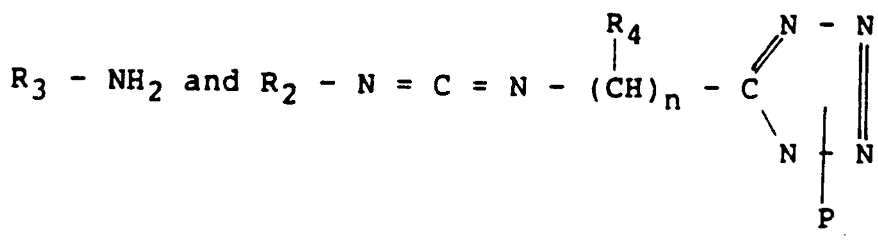
and

These reactions can be carried out in water or in organic solvents such as ethanol, methanol, acetone, chloroform, carbon tetrachloride or pyridine at a temperature which may vary from ambient temperature to boiling. The choice of the solvent and the temperature employed will depend on the L group and the reactivity of the amine employed and can be readily determined by one skilled in the art.

Additionally, the present invention encompasses novel intermediates used in preparing the present guanidine compounds as seen in the tetrazole containing reactants in (3) and (4) above.

Various methods may be employed to prepare the intermediaries but the corresponding thiourea derivative will generally have to be prepared as follows, for example:

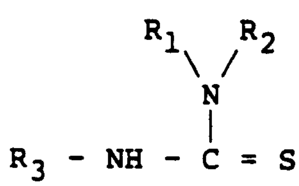

One of the preferred methods of synthesis employed to obtain these thiourea derivatives consists of allowing an alkyl or aryl isothiocyanate to react with a suitable amine. The reaction is conducted from ambient temperature to boiling, depending on the reciprocal reactivity of the two compounds and takes place in an organic solvent such as ethanol, ethyl acetate, acetonitrile, chloroform or acetone. These reactions can be characterized as follows:

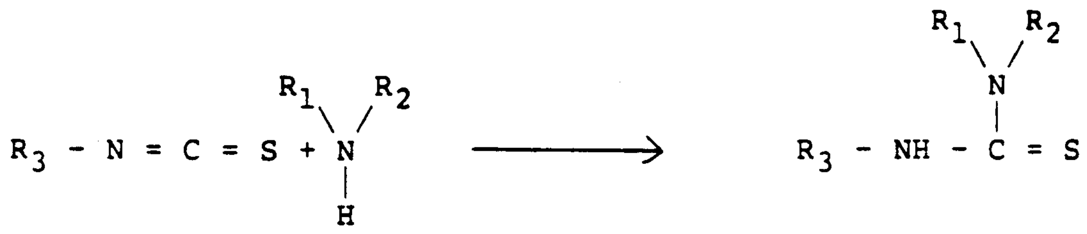

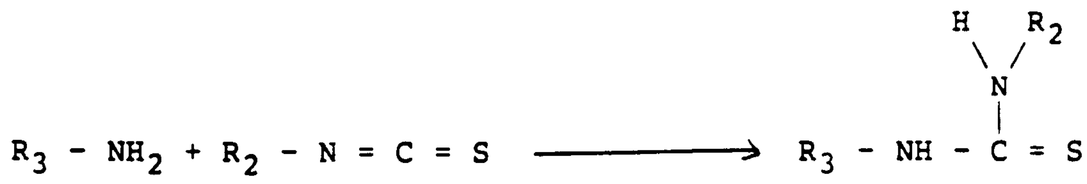

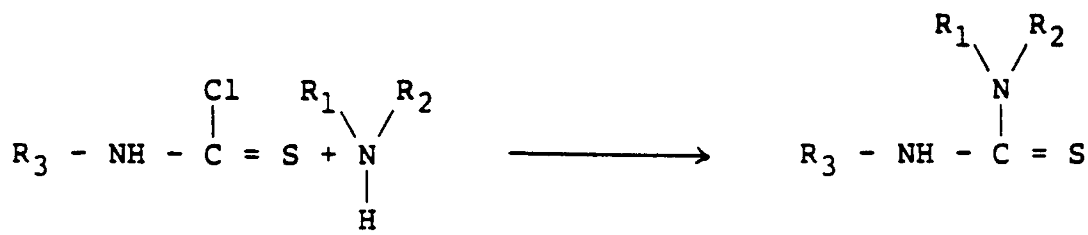

The preferred method consists of transforming thiourea derivatives obtained in this way into S-alkyl derivatives. The preferred L activating group is the $S-CH_3$ group, such as in the following compound, for example:

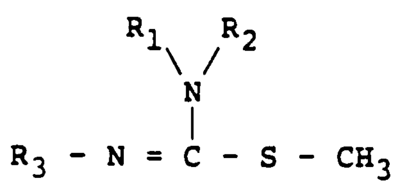

These intermediates are obtained by treating the corresponding thiourea derivative with an alkylating agent (such as methyl iodide or dimethyl sulfate) in solution in an organic solvent such as acetone or 2-butanone at a temperature ranging from ambient temperature to boiling depending upon reactivity of the materials. The S-methylisothiourea derivatives are thus obtained in the form of salts (iodide or sulfate). These salts are next treated in a solution of sodium hydroxide or potassium hydroxide in order to free their base form. They are next condensed with an aminomethyltetrazole in a suitable solvent, at a temperature ranging from ambient to boiling depending on reactivity of the materials.

Method 1: The Isothiourea Route:

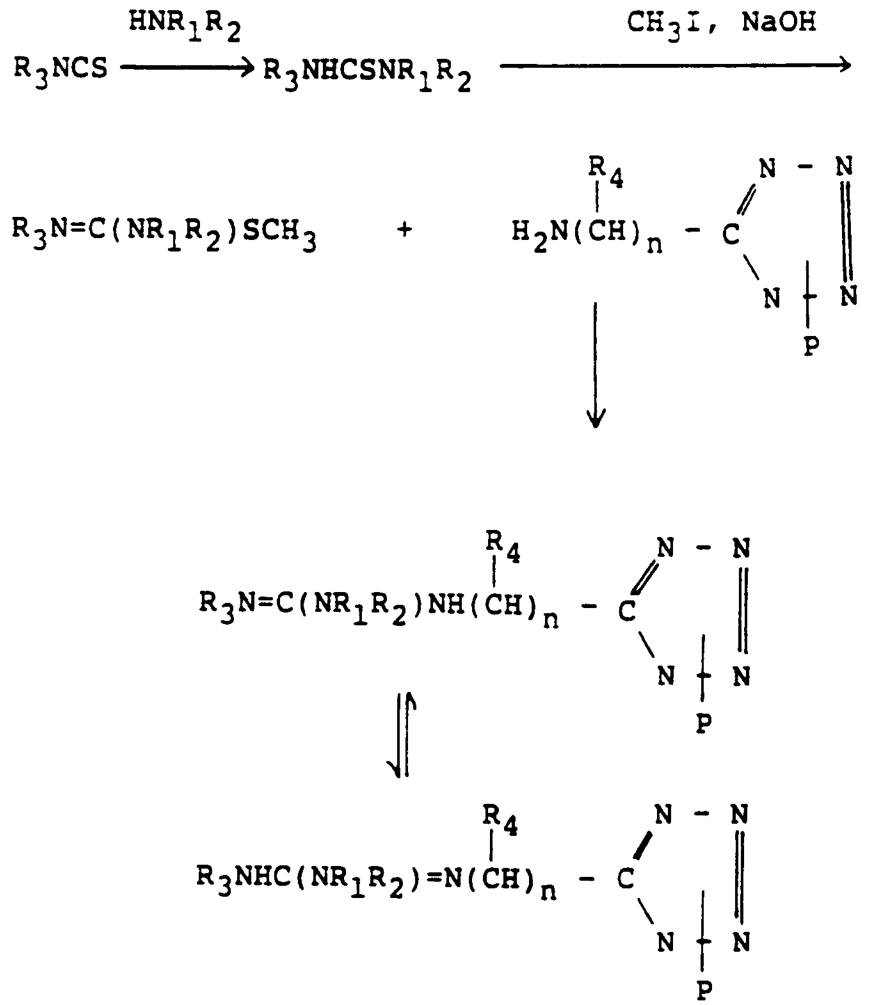


According to this general method, an alkyl or aryl isothiocyanate, $R_3NCS$, is allowed to condense with an amine $HNR_1R_2$ preferably at ambient temperature and in an organic solvent to yield a thiourea $R_3NHCSNR_1R_2$. The thiourea is purified by recrystallization or other standard procedures and is then methylated with methyl iodide or dimethyl sulfate. This reaction is conducted in an organic solvent (e.g., acetone) at temperatures varying from ambient to 100 ° C depending on thiourea reactivity. With methyl iodide, a crystalline isothiouronium salt, e.g., $R_3N=C(NR_1R_2)SCH_3]^+ I^-$, is thus obtained which is dissolved in water and treated with one molar equivalent of NaOH. The isothiourea $R_3N=C(NR_1R_2)SCH_3$ is then obtained by extraction by an organic solvent, i.e., methylene chloride, and recrystallization. The isothiourea is then reacted with the amino methyltetrazole to give the guanidine

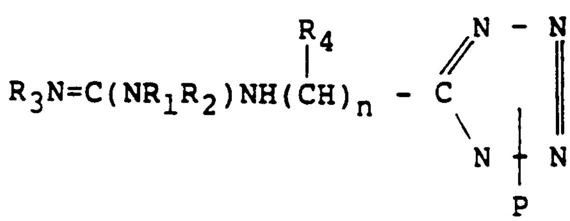

This reaction may be conducted in water or organic solvents and at temperatures varying from ambient to 100 ° C. The guanidine is then purified by recrystallization or other standard methods.

The second general method is based on the use of a carbodiimide intermediate which may be obtained by the reaction of an appropriate thiourea with phosgene or with an equimolecular mixture of triphenylphosphine, tertiary amine and carbon tetrachloride in an organic solvent, such as carbon tetrachloride or dichloromethane, at a temperature ranging from ambient temperature to boiling.

Method 2: The Carbodiimide Route:

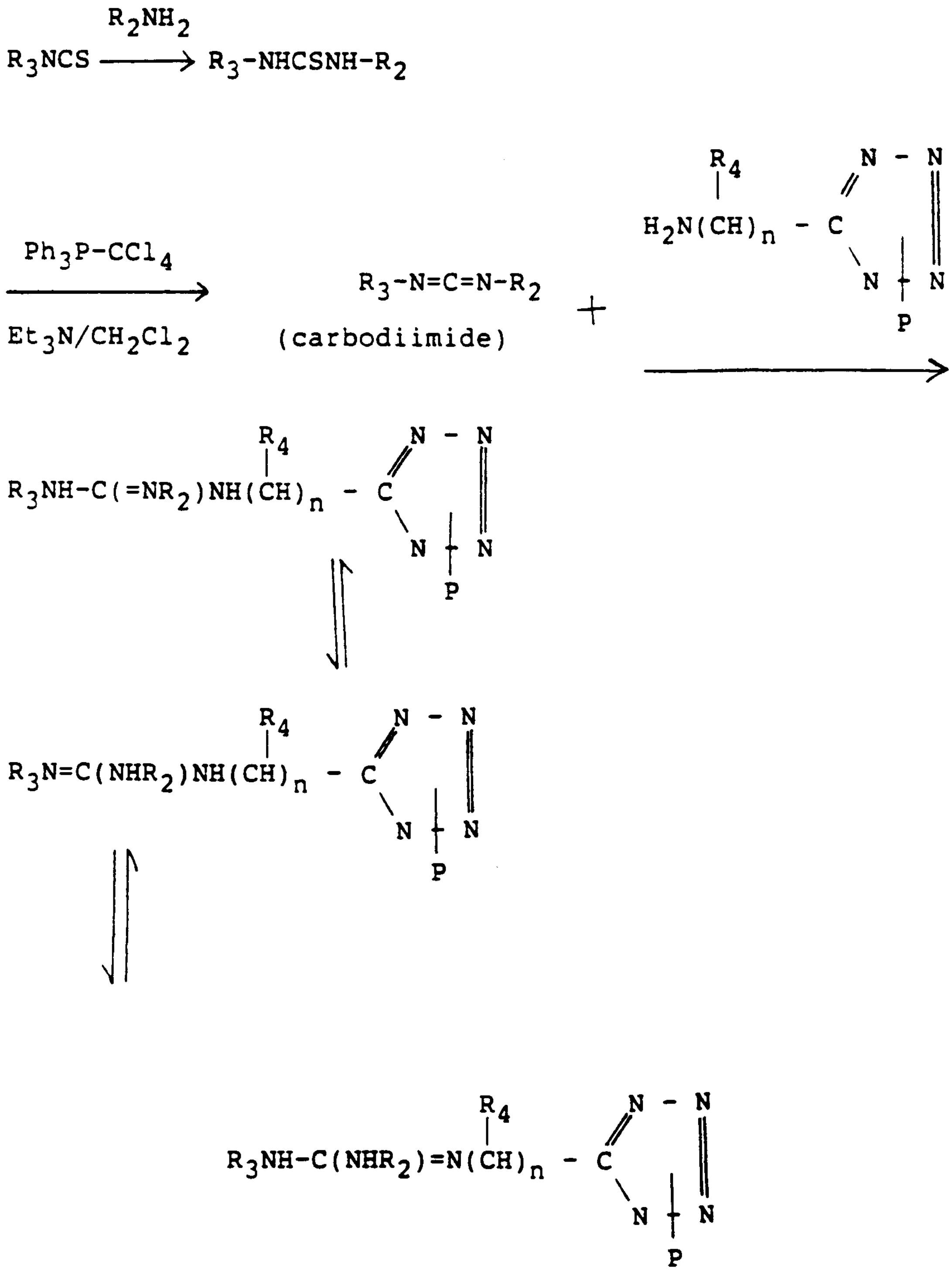


According to this general method, an aryl isothiocyanate $R_3NCS$ is allowed to condense with a primary amine ($R_2$-$NH_2$) to yield an N,N′-disubstituted thiourea $R_3$-NHCS-$NHR_2$. After purification by standard techniques such as recrystallization or chromatography, the thiourea is then converted to a carbodiimide $R_3$-N=C=N-$R_2$ on reaction in refluxing dichloromethane with one equivalent each of triphenylphosphine,

carbon tetrachloride and triethylamine. The carbodiimide is not purified but rather the carbodiimide is reacted directly with an aminoalkyltetrazole or an aminotetrazole to give the trisubstituted guanidine.

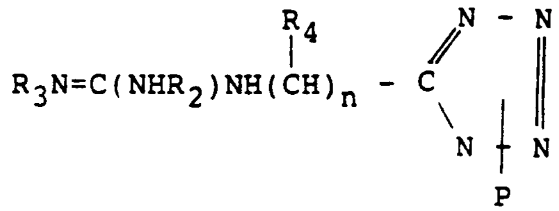

Tetra and penta-substituted guanidines may be obtained on further alkylation of the trisubstituted guanidine with alkyl halides.

The present guanidines may exist as an equilibrium mixture of tautomeric forms. All tautomers of the guanidines are contemplated by the present invention. The guanidines are shown in the general formula as the tautomer with $R_3N=C$- unsaturation; however, this tautomer is invariably in equilibrium with the tautomers, with $-C=NR_2$ and

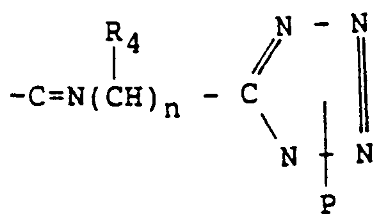

When P is hydrogen, the sweetening agents according to the present invention may exist in zwitterion or in acid form. They can thus be converted into salts by acids or by physiologically acceptable organic or inorganic bases. One of the best methods of preparing such salts consists of concentrating to dryness in vacuo a mixture of a compound according to the present invention in an aqueous solution with an equivalent amount of an acid or of an organic or inorganic base. The preferred salts according to the present invention are hydrochloride, citrate, phosphate, sulfate, or sodium, potassium, ammonium, calcium or magnesium salts.

Especially preferred starting materials are those of the formula

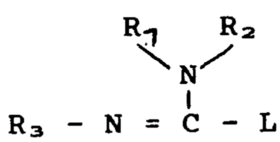

wherein
$R_2$ is cyclohexyl, cyclooctyl, alpha-phenethyl, diphenylmethyl, benzyl, phenyl, endo-norbornyl, 1-naphthyl or 2-decalyl,
or wherein the compounds are prepared from the starting material of the formula

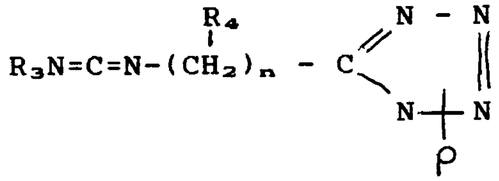

wherein $R_3$ is 4-cyanophenyl, 3,5-dichlorophenyl, 3,5-dimethylphenyl or phenyl,
and from an amine $HNR_1R_2$ wherein $R_2$ is cyclohexyl, cyclooctyl, alpha-phenethyl, diphenylmethyl, benzyl, phenyl, endo-norbornyl, 1-naphthyl or 2-decalyl,
and wherein $R_3$ is 4-cyanophenyl, 3,5-dichlorophenyl, 3,5-dimethylphenyl or phenyl.

The present sweetening agents may take the form of a balanced mixture of tautomeric forms. Thus, the following tautomeric forms may be obtained, when $R_1$ is H:

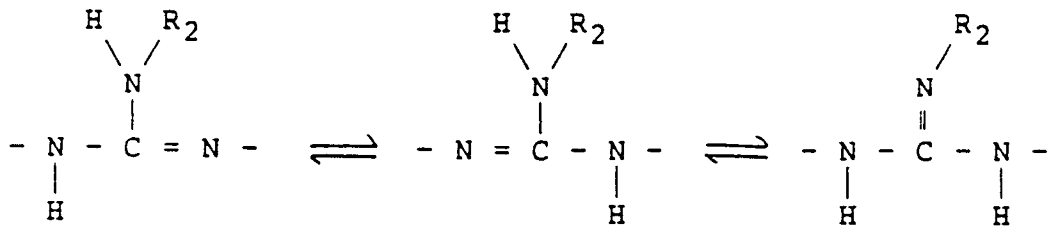

or when $R_1 = CH_3$:

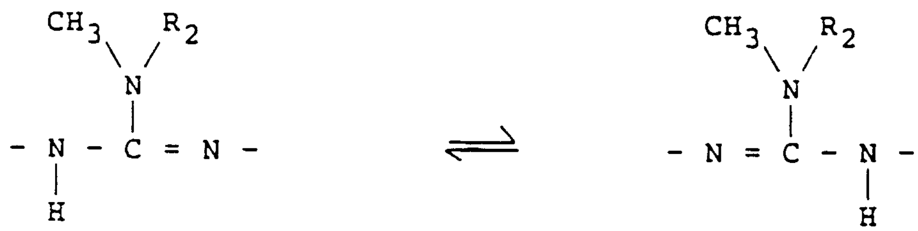

and the following tautomeric forms for tetrazole will apply:

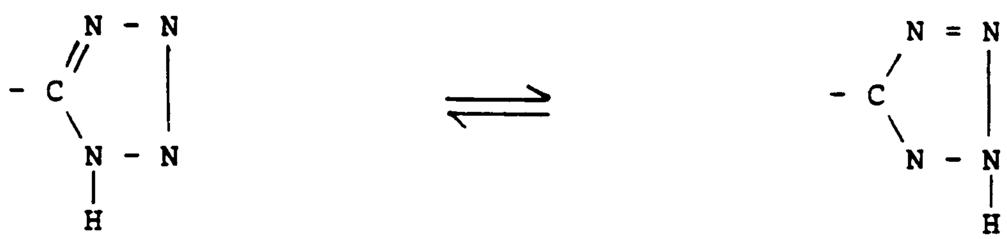

This is why the present guanidines are represented in Formula (I) by one of the tautomeric forms in the descriptive part of the present specification, in the full knowledge that the tautomeric form must necessarily be in balance with the other tautomeric forms, depending on the nature of the substituents $X_3$, $X_4$, $X_5$, $R_1$, and $R_2$, as well as on the pH.

The present N,N',N''-trisubstituted guanidines may have an asymmetrical carbon atom, i.e., optically active site. These compounds exist in (R) and (S) enantiomeric forms. Both the (R) and (S) enantiomers of the N,N',N''-trisubstituted guanidines are contemplated by the present invention.

The present invention also relates to a method of sweetening foods or comestible products. In such uses, the present guanidines are added to any consumable product in which it is desired to have a sweet taste. The present sweetening agents are added to such products in amounts effective to impart the desired level of sweetness. The optimum amount of sweetening agent will vary depending on a variety of factors such as, for example, the sweetness potency of the particular sweetening agent, storage and use conditions of the product, the particular components of the products, the flavor profile of the comestible products and the level of sweetness desired. One skilled in the art can readily determine the optimum amount of sweetening agent to be employed in a particular formulation of a food product by conducting routine sweetness (sensory) experiments. Usually, the present sweetening agents are added to the comestible products in amounts of from about 0.00001 to about 0.1 percent by weight of the comestible product, advantageously from about 0.00005 to about 0.05 weight percent and preferably from about 0.001 to about 0.02 weight percent. Concentrates, of course, will contain higher percentages of sweetening agent(s), and are diluted for end use purposes.

Suitable products which are sweetened by the present sweetening agents include any products for which a sweet flavor component is desired such as food products (for human or animal consumption), beverages (alcoholic, soft drinks, juices, carbonated beverages), confectionary products (candies, chewing gum, baked goods, pastries, breads, etc.), toilet and hygiene products, cosmetics, pharmaceutical products and veterinary products. In sweetening gum, the present guanidines can be added in amounts in excess of a sucrose equivalent normally found in gum. This excess amount of guanidine sweetener provides a longer sweet taste and enhancement of flavor (flavor enhancer).

The present guanidines can be added in pure form to foods to impart a sweet flavor. However, because of the high sweetness potency of the present sweetening agents, they are typically admixed with a carrier or bulking agent. Suitable carriers or bulking agents include polydextrose, starch, malto-dextrins, cellulose, methylcellulose, carboxymethylcellulose, hydroxymethylcellulose, microcrystalline cellulose, cellulose derivatives, sodium alginate, pectins, gums, lactose, maltose, maltitol, glucose, leucine, glycerol, mannitol, sorbitol, sodium bicarbonate and phosphoric, citric, tartaric, fumaric, benzoic, sorbic and propionic acids and their sodium, potassium and calcium salts and mixtures of all of the above.

The present guanidines can be employed alone as the sole sweetening agent in a comestible product. Mixtures of the present guanidines can also be employed. Additionally, the present guanidines can be used in combination with other sweetening agents such as sugars (such as sucrose and fructose), corn syrups, dipeptide sweeteners such as aspartame and alitame and other sweeteners such as glycyrrhizin, aminoacyl sugars, xylitol, sorbitol, mannitol, acesulfam K, thaumatin, monellin, cyclamates, saccharin, neohesperidine dihydrochalcone, hydrogenated isomaltulose, stevioside, L-sugars, trichlorogalactosucrose (TGS), and mixtures thereof.

The following examples illustrate the practice of the present invention.

EXAMPLE 1

Synthesis of 1'-N-[N-Cyclooctylamino (4-Cyanophenylimino)methyl]-5-Aminomethyltetrazole:

Step 1: Preparation of N-Cyclooctyl-N'-(4-Cyanophenyl)Thiourea:

4-Cyanophenylisothiocyanate (20.0 g; 125 mmol) was dissolved in 300 ml ethyl acetate. Cyclooctylamine (17.1 ml, 125 mmol) having a density of 0.928 was added to the reaction mixture with stirring at room temperature. After 10 minutes, a precipitate formed. The reaction mixture was stirred overnight and filtered to give 23.9 g (67%) of white solid (mp = 149-151 ° C) which was dried at room temperature under a vacuum. PMR ($CDCl_3$) $\delta$ 8.71 (br s, 1H), 7.66 (d, J = 8 Hz, 2H), 7.39 (d, J = 8 Hz, 2H), 6.36 (d, J = 8 Hz, 1H), 4.6-4.4 (m, 1H), 2.0-1.9 (m, 2H) and 1.7-1.4 (m, 12H). CMR ($CDCl_3$) $\delta$ 178.9, 142.2, 134.5, 123.6, 119.0, 109.0, 56.0, 32.6, 27.6 26.2 and 24.4.

Step 2: Synthesis of N-Cyclooctyl-N′-(4-Cyanophenyl)-S-Methylisothiourea:

A mixture of 20 g (69.7 mmol) of the compound obtained previously and 10.9 ml (d = 2.28; 175 mmol) of methyl iodide was stirred overnight in 250 ml of acetone. In the morning, a solid was present and it was filtered to give 27.3 g (91%) of a white solid. The filtrate was concentrated under reduced pressure to give an orange solid which was triturated with ether and filtered to give 2.1 g of off-white solid which was added to the 27.3 g filtered before. The combined solid produced was dissolved in 100 ml of a 1N solution of sodium hydroxide. The resulting alkaline mixture was extracted with methylene chloride ($CH_2Cl_2$). The $CH_2Cl_2$ layer was washed with saturated NaCl and dried with anhydrous sodium sulfate. The $CH_2Cl_2$ layer was then concentrated under reduced pressure to give 19.8 g (94%) of a white solid (mp = 84-86 ° C). PMR ($CDCl_3$) $\delta$ 7.54 (d, J = 7 Hz, 2H), 6.96 (d, J = 7 Hz, 2H), 4.58 (br s, 1H), 3.96 (br s, 1H), 2.24 (s, 3H), 2.0-1.8 (m, 2H) and 1.64-1.44 (m, 12H). CMR ($CDCl_3$) $\delta$ 154.5, 153.2, 133.2, 123.0, 119.8, 104.9, 52.9, 32.5, 27.2, 25.6, 23.7 and 14.3.

Step 3: Preparation of 1′-N-[N-Cyclooctylamino (4-Cyanophenylimino)methyl]-5-Aminomethyltetrazole:

A mixture of 5-aminomethyltetrazole (0.50 g, 5.05 mmol), sodium hydroxide (0.20 g, 5.00 mmol) and 1.5 ml of water was added to a solution of N-cyclooctyl-N′-(4-cyanophenyl)-S-methylisothiourea (1.52 g, 5.05 mmol) in 15 ml of absolute ethanol. The mixture was heated to 70 ° for 20 hours. After cooling, the solution was concentrated to dryness and the residue was dissolved in 20 ml of water. The resulting solution was washed with dichloromethane (3 x 20 ml) and then neutralized with a 1 N HCl solution to bring its pH to 7.5.

The desired guanidine compound precipitated and was separated out by filtration to give 1.2 g (70%) of white solid. PMR ($CD_3OD$) δ 7.75 (d, J=8 Hz, 2H), 7.41 (d, J=8 Hz, 2H), 4.70 (s, 2H), 3.82 (m, 1H) and 1.96-1.46 (m, 14H). CMR (DMSO-D6) δ 158.3, 153.3, 144.0, 133.7, 122.0, 119.0, 105.5, 52.8, 37.8, 31.2, 26.8, 24.6 and 22.9. Analysis calculated for $C_{18}H_{24}N_8$ (0.6 $H_2O$): C, 59.52; H, 6.99; N, 30.85. Found: C, 59.38; H, 7.07; N, 31.18. The compound was tasted and was sweeet at a concentration of 10 ug/ml.

The hydrochloride of this compound was prepared by dissolving, with heating, 1.0 g of the above zwitterion in a mixture of 20 ml 1 N HCl and 10 ml ethanol. Upon cooling, 900 mg (82%) of white crystalline compound precipitated and was collected by filtration. Analysis calculated for $C_{18}H_{24}N_8$ HCl • (0.4 $H_2O$): C, 54.58; H, 6.56; N, 28.29. Found: C, 54.65, H, 6.40, N, 28.33. This HCl salt was also sweet when tasted.

The phosphate of this compound can be prepared by dissolving, with heating, 1.0 g (2.84 mmoles) of the above zwitterion in a mixture of 15 ml 1 M phosphoric acid and 7 ml ethanol. Upon cooling, 1.2 g (92%) of white crystalline compound slowly precipitates and is collected by filtration. PMR ($CD_3OD$) δ 7.78-7.40 (apparent AB q, J=9 Hz, 4H), 4.81 (s, 2H), 3.88-3.77 (m, 1H) and 1.97-1.45 (m, 14H). Analysis calculated for $C_{18}H_{27}N_8PO_4$(1.85 $H_2O$): C, 44.69; H, 6.40; N, 23.16. Found: C, 44.68, H, 6.13, N, 23.19.

The hemi-citrate of this compound can be prepared by dissolving, with heating, 1.0 g (2.84 mmoles) of the above zwitterion in a mixture of 15 ml 1 M phosphoric acid and 7 ml ethanol. Upon cooling, 0.94 g (63%) of white crystalline compound slowly precipitates and is collected by filtration. PMR ($CD_3OD$) δ 7.78-7.42 (apparent AB q, J=9 Hz, 4H), 4.73 (s, 2H), 3.88-3.77 (m, 1H), 2.92-2.74 (AB q, J=15 Hz, 2H), 1.98-1.84 (m, 2H), 1.82-1.67 (m, 4H) and 1.64-1.45 (m, 8H). Analysis calculated for $C_{18}H_{24}N_8$ • 0.5 $C_6H_8O_7$: C, 56.24; H, 6.29; N, 24.98. Found: C, 55.84, H, 6.23, N, 24.70.

The sulfate of this compound can be prepared by dissolving, with heating, 1.0 g (2.84 mmoles) of the above zwitterion in a mixture of 15 ml 1 M sulfuric acid and 7 ml ethanol. Upon cooling, 0.56 g (43%) of white crystalline compound slowly precipitates and is collected by filtration. PMR (DMSO-D6) δ 8.79 (br s, 1H), 8.48 (br s, 1H), 7.88 (d, J=8 Hz, 2H), 7.39 (br s, 2H), 4.80 (s, 2H), 3.90-3.75 (m, 1H), 1.88-1.73 (m, 2H) and 1.70-1.30 (m, 12H). Analysis calculated for $C_{18}H_{24}N_8$ • $H_2SO_4$ (0.4 $H_2O$): C, 47.22; H, 5.90; N, 24.47, S, 7.00. Found: C, 47.20, H, 5.83, N, 24.46, S, 6.66.

## EXAMPLE 2

Synthesis of 1′-N-[N-Cyclohexylamino(4-Cyanophenylimino)methyl]-5-Aminomethyltetrazole:

Step 1: Preparation of N-Cyclohexyl-N′-Cyclohexyl-N′-(4-Cyanophenyl Thiourea:

4-Cyanophenylisothiocyanate (100 g; 625 mmol) was dissolved in 1 liter of ethyl acetate. Cyclohexylamine (62 g, 625 mmol) was added to the reaction mixture with stirring at room temperature. After 10 minutes, a precipitate formed. The reaction mixture was stirred overnight and filtered to give 140 g (86%) of white solid (mp=192-193°C) which was dried at room temperature under a vacuum. PMR (DMSO-D6) δ 8.08 (d, 1H), 7.80 (m, 4H), 7.66 (s, 1H), 4.12 (br s, 1H) and 1.95-1.2 (m, 10H). CMR (DMSO-D6) δ 178.6, 144.3, 132.6, 120.9, 119.0, 52.1, 31.5, 25.0 and 24.4. Analysis calculated for $C_{14}H_{17}N_3S$: C, 64.83; H, 6.61; N, 16.70; S, 12.36. Found: C, 64.70; H, 6.56; N, 16.31; S, 12.48.

Step 2: Synthesis of N-Cyclohexyl-N′-(4-Cyanophenyl)-S-Methylisothiourea:

A misture of (30 g, 115.8 mmol) of the compound obtained previously and (26 g, 183.7 mmol) of methyl iodide was stirred overnight in 130 ml of acetone. In the morning, a solid was present and it was filtered. The solid produced was dissolved in 274 ml of a 1 N solution of sodium hydroxide. The resulting alkaline mixture was extracted with methylene chloride ($CH_2Cl_2$). The $CH_2Cl_2$ layer was washed with saturated NaCl and dried with anhydrous sodium sulfate. The $CH_2Cl_2$ layer was then dried with anhydrous sodium sulfate. The $CH_2Cl_2$ layer was then concentrated under reduced pressure to give 31.0 g (98%) of a white solid (mp=84-85°C). PMR ($CDCl_3$) δ 7.53 (d, 4H), 6.93 (d, 2H), 4.52 (br s, 1H), 3.73 (br s, 1H), 2.24 (s, 3H) and 2.05-1.1 (m, 10H). CMR ($CDCl_3$) δ 154.3, 133.1, 123.0, 104.9, 51.7, 31.1, 25.5, 24.8 and 14.2. Analysis calculated for $C_{15}H_{19}N_3S$: C, 65.90; H, 7.01; N, 15.37; S, 11.73. Found: C, 65.80; H, 6.91; N, 15.35; S, 11.71.

Step 3: Preparation of 1′-N-[N-Cyclohexylamino(4-Cyano-phenylimino)methyl]-5-Aminomethyltetrazole:

A mixture of 5-aminomethyltetrazole (0.50 g, 5.05 mmol), sodium hydroxide (0.20 g, 5.00 mmol) and 1.5 ml of water was added to a solution of N-cyclohexyl-N′-(4-cyanophenyl)-S-methylisothiourea (2.76 g, 10.1

mmol) in 15 ml of absolute ethanol. The mixture was heated to 70°C for 20 hours. After cooling, the solution was concentrated to dryness and the residue was dissolved in 20 ml of water. The resulting solution was washed with ether (2 x 20 ml) and then neutralized with a 1 N HCl solution to bring its pH to 7.5. The desired guanidine compound precipitated and was separated out by filtration to give 0.85 g (53%) of the desired compound. PMR ($CD_3OD$) δ 7.75 (d, J=8 Hz, 2H), 7.45 (d, J=8 Hz, 2H), 4.73 (s, 2H), 3.62 (m, 1H), 2.1-1.9 (m, 2H) and 1.85-1.10 (m, 8H). CMR ($CD_3OD$) δ 157.9, 153.4, 141.1, 133.0, 122.5, 117.5, 107.7, 51.8, 36.8, 31.6, 24.1 and 23.9. Analysis calculated for $C_{16}H_{20}N_8$ (1.25 $H_2O$): C, 55.40; H, 6.54; N, 32.30; Found: C, 55.48; H, 6.59, N, 32.17. The compound was tasted and was sweet at a concentration of 100 μg/ml.

EXAMPLE 3

Synthesis of 1′-N-[N-cyclooctylamino(4-cyanophenylimino)methyl]-5-aminotetrazole:

Step 1: Preparation of Carbodiimide:

N-cyclooctyl-N′-(4-cyanophenyl)thiourea (1.0 g, 3.48 mmol) prepared in Example 1 was suspended in methylene chloride (5 ml). Carbon tetrachloride (0.47 ml, 4.86 mmol) was added to the suspension followed by the addition of triphenylphosphine (1.2 g, 4.58 mmol) and triethylamine (0.48 ml, 3.45 mmol). This mixture was heated to reflux resulting in a homogeneous solution. The mixture was stirred at reflux for 1.5 hours resulting in a yellow solution with a solid precipitate. The mixture was cooled and concentrated under reduced pressure resulting in a residue which was triturated with hexane and filtered. The filtrate was concentrated under reduced pressure to give 0.79 g of N-cyclooctyl-N′-(4-cyanophenyl) carbodiimide. IR and NMR spectroscopy verified the structure.

Step 2: Preparation of 1′-N-[N-Cyclooctylamino(4-cyanophenylimino)methyl]-5-aminotetrazole:

A solution containing 0.72 g (6.99 mmol) of 5-aminotetrazole in 10 ml of dimethylformamide (DMF) was added to the carbodiimide (0.79 g, 3.12 mmol) obtained in step 1 above. The reaction was heated to 100°C and stirred overnight. In the morning, the reaction was concentrated under reduced pressure at 40°C. The residue was dissolved in 5 ml of 1 N sodium hydroxide and extracted twice with ether. The basic solution was neutralized to pH 7.5 using 1 N HCl to give 0.42 g (38%) of white solid which was filtered. PMR ($CD_3OD$) δ 7.62 (s, 4H), 4.04 (m, 1H), 2.06-1.92 (m, 2H), and 1.82-1.48 (m, 8H). CMR ($CD_3OD$) δ 158.8, 151.5, 143.2, 132.0, 121.1, 117.9, 105.0, 51.7, 30.8, 26.5, 24.1 and 22.3.

EXAMPLE 4

Synthesis of 1′-N-[N-(S)-Phenylethylamino (4-Cyanophenylimino)methyl]-5-Aminomethyltetrazole:

Step 1: Preparation of N-(S)-Phenylethyl-N′-(4-Cyanophenyl) Thiourea:

4-Cyanophenylisothiocyanate (10.0 g; 62.5 mmol) was dissolved in 150 ml ethyl acetate. Phenylethylamine (8.05 ml, 62.5 mmol) having a density of 0.940 was added to the reaction mixture with stirring at room temperature. The reaction mixture was stirred overnight and concentrated under reduced pressure to give a yellow solid. This solid was triturated with ether to give a yellow solid. This solid was triturated with ether to give 15.5 g (88%) of white solid (mp=128-129°C). PMR ($CDCl_3$) δ 7.9 (br s, 1H), 7.6 (d, J=8 Hz, 2H), 7.5-7.25 (m, 7H), 6.56 (br s, 1H), 5.42 (br s, 1H) and 1.60 (d, J=6 Hz, 3H).

Step 2: Synthesis of N-(S)-Phenylethyl-N′-(4-Cyanophenyl)-S-Methylisothiourea:

A mixture of 11.2 g (40 mmol) of the compound obtained previously and 6.23 ml (d = 2.28; 100 mmol); of methyl iodide was stirred overnight in 135 ml of acetone. In the morning, a solid was present and it was filtered to give 14.3 g of a white solid. The solid produced was dissolved in 60 ml of a 1 N solution of sodium hydroxide. The resulting alkaline mixture was extracted with ether. The ether layer was washed with saturated NaCl and dried with anhydrous magnesium sulfate. The ether layer was then concentrated under reduced pressure to give 9.7 g (82%) of a colorless oil. PMR ($CDCl_3$) δ 7.52 (d, J=9 Hz, 2H), 7.40-7.25 (m, 5H), 6.90 (d, J=9 Hz, 2H), 5.09 (q, J=6 Hz, 1H), 4.84 (br s, 1H), 2.22 (s, 3H) and 1.54 (d, J=6 Hz, 3H).

Step 3: Preparation of 1′-N-[N-(S)-Phenylethylamino(4-Cyanophenylimino)methyl]-5-Aminomethyltetrazole:

A mixture of 5-aminomethyltetrazole (0.50 g, 5.05 mmol), sodium hydroxide (0.20 g, 5.00 mmol) and 1.5 ml of water was added to a solution of N-(S)-phenyl-ethyl-N′-(4-cyanophenyl)-S-methylisothiourea (1.52 g, 5.05 mmol) in 15 ml of absolute ethanol. The mixture was heated to 70°C for 20 hours. After cooling, the solution was concentrated to dryness and the residue was dissolved in 20 ml of water. The resulting solution was dissolved in 20 ml of water. The resulting solution was washed with ether (2 x 20 ml) and then neutralized with a 1 N HCl solution to bring its pH to 7.5. The desired guanidine compound precipitated and was separated out by filtration to give 1.2 g (72%) of the title compound. PMD ($CD_3OD$) δ 7.68 (d, J=9 Hz, 2H), 7.4-7.2 (m, 7H), 5.0-4.8 (m, 1H, partially obscured), 4.73 (s, 2H) and 1.60 (d, J=6 Hz, 3H). CMR ($CD_3OD$) δ 157.4, 154.2, 141.1, 140.9, 133.2, 128.3, 127.3, 125.3, 122.6, 117.6, 108.3, 52.8, 37.1 and 21.3. Analysis calculated for $C_{18}H_{18}N_8$: C, 62.41; H, 5.24; N, 32.35. Found: C, 62.06; H, 5.15; N, 32.12. The compound was tasted and was sweet at a concentration of 100 µg/ml.

EXAMPLE 5

Synthesis of 1′-N-[N-(S)-Phenylethylamino (Phenylimino)methyl]-5-Aminomethyltetrazole:

Step 1: Preparation of N-(S)-Phenylethyl-N′-phenyl Thiourea:

Phenylisothiocyanate (10.0 ml; d=1.130; 62.5 mmol) was dissolved in 150 ml ethyl acetate. Phenylethylamine (10.8 ml, 83.9 mmol) having a density of 0.940 was added to the reaction mixture with stirring at room temperature. The reaction mixture was stirred overnight and concentrated under reduced pressure to give a yellow solid. This solid was triturated with hexanes to give 19.7 g (92%) of white solid (mp=64-66°C). PMR ($CDCl_3$) δ 8.0 (br s, 1H), 7.45-7.15 (m, 10H), 6.28 (br s, 1H), 5.19 (br s, 1H) and 1.55 (d, J=6 Hz, 3H).

Step 2: Synthesis of N-(S)-Phenylethyl-N′-Phenyl-S-Methylisothiourea:

A mixture of 10.2 g (40 mmol) of the compound obtained previously and 6.23 ml (d=2.28; 100 mmol) of methyl iodide was stirred overnight in 135 ml of acetone. In the morning, the reaction was concentrated under reduced pressure. The residue was triturated with ether. The solid produced was dissolved in 60 ml of a 1 N solution of sodium hydroxide. The resulting alkaline mixture was extracted with ether. The ether layer was washed with saturated NaCl and dried with anhydrous magnesium sulfate. The ether layer was then concentrated under reduced pressure to give 10.0 g (92%) of a pale yellow oil. PMR ($CDCl_3$) δ 7.4-7.2 (m, 7H), 7.02 (t, J=7 Hz, 1H), 6.87 (d, J=7 Hz, 2H), 5.08 (br s, 1H), 4.70 (br s, 1H), 2.28 (s, 3H) and 1.50 (d, J=7 Hz, 3H).

Step 3: Preparation of 1′-N-[N-(S)-Phenylethylamino(Phenylimino)methyl]-5-Aminomethyltetrazole:

A mixture of 5-aminomethyltetrazole (0.50 g 5.05 mmol), sodium hydroxide (0.20 g, 5.00 mmol) and 1.5 ml of water was added to a solution of N-(S)-phenylethyl-N′-phenyl-S-methyl-isothiourea (1.36 g, 5.04 mmol) in 15 ml of absolute ethanol. The mixture was heated to 70°C for 20 hours. After cooling, the solution was concentrated to dryness and the residue was dissolved in 20 ml of water. The resulting solution was washed with ether (2 x 20 ml) and then neutralized with a 1 N HCl solution to bring its pH to 7.5. The desired guanidine compound precipitated and was separated out by filtration to give 1.0 g (62%) of the title compound. PMR ($CD_3OD$) δ 7.4-7.1 (m, 10H), 4.9-4.8 (m, 1H, partially obscured), 4.70 (s, 2H) and 1.60 (d, J=6 Hz, 3H). Analysis calculated for $C_{17}H_{19}N_7$ ($H_2O$): C, 60.16; H, 6.24; N, 28.89. Found: C, 59.80; H, 5.71; N, 29.14. The compound was tasted and was sweet at a concentration of 100 µg/ml.

EXAMPLE 6

Synthesis of 1′-N-[N-(S)-Phenylethylamino(3,5-Dichlorophenylimino)methyl]-5-Aminomethyltetrazole:

Step 1: Preparation of N-(S)-Phenylethyl-N′-(3,5-Dichlorophenyl) Thiourea:

3,5-Dichlorophenylisothiocyanate (4.0 g; 19.6 mmol) was dissolved in 40 ml ethyl acetate. Phenylethylamine (2.53 ml, 19.6 mmol) having a density of 0.940 was added to the reaction mixture with

stirring at room temperatue. The reaction mixture was stirred overnight and concentrated under reduced pressure to give a yellow solid. This solid was triturated with ether to give 5.8 g (91%) of white solid (mp-168-169 °C). PMR ($CDCl_3$) δ 7.75 (br s, 1H), 7.45-7.15 (m, 8H), 6.40 (br s, 1H), 5.5 (br s, 1H) and 1.59 (d, J = 7 Hz, 3H).

Step 2: Synthesis of N-(S)-Phenylethyl-N′-3,5-Dichlorophenyl)-S-Methylisothiourea:

A mixture of 5.0 g (15.4 mmol) of the compound obtained previously and 2.4 ml (d = 2.28; 38.5 mmol) of methyl iodide was stirred overnight in 50 ml of acetone. In the morning, the reaction was concentrated under reduced pressure and triturated with ether. The solid produced was dissolved in 20 ml of 1 N solution of sodium hydroxide. The resulting alkaline mixture was extracted with ether. The ether layer was washed with saturated NaCl and dried with anhydrous magnesium sulfate. The ether layer was then concentrated under reduced pressure to give 4.5 g (86%) of an orange oil. PMR ($CDCl_3$) δ 7.42-7.27 (m, 5H), 6.98 (t, J = 2 Hz, 1H), 6.75 (d, J = 2 Hz, 2H), 5.06 (q, J = 6 Hz, 1H) 4.74 (br s, 1H), 2.27 (s, 3H) and 1.52 (d, J = 6 Hz, 3H).

Step 3: Preparation of 1′-N-[N-(S)-Phenylethylamino(3,5-Dichlorophenylimino)methyl]-5-Aminomethyl-tetrazole:

A mixture of 5-aminoethyltetrazole (0.50 g, 5.05 mmol), sodium hydroxide (0.20 g, 5.00 mmol) and 1.5 ml of water was added to a solution of N-(S)-phenylethyl-N′-(3,5-dichlorophenyl)-S-methylisothiourea (1.7 g, 5.01 mmol) in 15 ml of absolute ethanol. The mixture was heated to reflux for 20 hours. After cooling, the solution was concnetrated to dryness and the residue was dissolved in 20 ml of water. The resulting solution was washed with ether (2 x 20 ml) and then neutralized with a 1 N HCl solution to bring its pH to 7.5. The desired guanidine compound precipitated and was separated out by filtration to give 1.7 g (85%) of the title compound. PMR ($CD_3OD$) δ 7.4-7.25 (m, 6H), 7.13 (d, J = 2 Hz, 2H), 4.73 (s, 2H) and 1.60 (d, J = 7 Hz, 3H). CMR ($CD_3OD$) δ 159.7, 155.9, 142.7, 140.0, 136.9, 130.0, 129.0, 127.1, 123.5, 54.3, 38.7 and 22.9. Analysis calculated for $C_{17}H_{17}N_7Cl_2$ (0.3 $H_2O$): C, 51.60; H, 4.48; N, 24.78. Found: C, 51.53; H, 4.28; N, 24.81. The compound was tasted and was sweet at a concentration of 10 µg/ml.

## EXAMPLE 7

Synthesis of 1′-N-[N-Benzylamino (3,5-Dichlorophenylimino)methyl]-5-Aminomethyltetrazole:

Step 1: Preparation of N-Benzyl-N′-(3,5-Dichlorophenyl) Thiourea:

3,5-Dichlorophenylisothiocyanate (4.0 g; 19.6 mmol) was dissolved in 40 ml ethyl acetate. Benzylamine 2.14 ml, 19.6 mmol) having a density of 0.981 was added to the reaction mixture with stirring at room temperature. The reaction mixture was stirred overnight and concentrated under reduced pressure to give a yellow solid. This solid was triturated with ether to give 5.7 g (93%) of white solid (mp = 148-150 °C). PMR ($CDCl_3$) δ 8.12 (br s, 1H), 7.41-7.12 (m, 8H), 6.38 (br s, 1H) and 4.87 (d, J = 5 Hz, 2H).

Step 2: Synthesis of N-Benzyl-N′-(3,5-Dichlorophenyl)-S-Methylisothiourea:

A mixture of 5.0 g (16.1 mmol) of the compound obtained previously and 2.5 ml (d = 2.28; 40.1 mmol) of methyl iodide was stirred overnight in 50 ml of acetone. In the morning, a solid was present and it was filtered to give 6.2 g of a white solid. The solid produced was dissolved in 20 ml of a 1 N solution of sodium hydroxide. The resulting alkaline mixture was extracted with ether. The ether layer was washed with saturated NaCl and dried with anhydrous magnesium sulfate. The ether layer was then concentrated under reduced pressure to give 4.1 g (79%) of a white solid (mp = 110-111 °C). PMR ($CDCl_3$) δ 7.42-7.28 (m, 5H), 6.99 (t, J = 2 Hz, 1H), 6.82 (d, J = 2 Hz, 2H) 4.79 (br s, 1H), 4.52 (s, 2H) and 2.29 (s, 3H).

Step 3: Preparation of 1′-N-[Benzylamino (3,5-Dichlorophenylimino)methyl]-5-Aminomethyltetrazole:

A mixture of 5-aminomethyltetrazole (0.50 g, 5.05 mmol), sodium hydroxide (0.20 g, 5.00 mmol) and 1.5 ml of water was added to a solution of N-benzyl-N′-(3,5-dichlorophenyl)-S-Methylisothiourea (1.64 g, 5.05 mmol) in 15 ml of absolute ethanol. The mixture was heated to reflux for 20 hours. After cooling, the solution was concentrated to dryness and the residue was dissolved in 20 ml of water. The resulting

solution was washed with ether (2 x 20 ml) and then neutralized with a 1 N HCl solution to bring its pH to 7.5. The desired guanidine compound precipitated and was separated out by filtration to give 1.2 g (63%) of the title compound. PMR ($CD_3COOD$) δ 7.4-7.15 (m, 8H), 5.02 (s, 2H) and 4.58 (s, 2H). CMR ($CD_3COOD$) δ 154.1, 153.4, 136.1, 134.6, 134.0, 127.7, 127.0, 126.4, 122.7, 45.0 and 34.4. Analysis calculated for $C_{16}H_{15}N_7Cl_2$ (0.4 $H_2O$): C, 50.12; H, 4.15; N, 25.57. Found: C, 50.01; H, 3.90; N, 25.62. The compound was tasted and was sweet at a concentration of 100 µg/ml.

EXAMPLE 8

Synthsis of 1′-N[N-Benzylamino (4-Cyanophenylimino)methyl]-5-Aminomethyltetrazole:

Step 1: Preparation of N-Benzyl-N′-(4-Cyanophenyl) Thiourea:

4-Cyanophenylisothiocyanate (10.0 g; 62.5 mmol) was dissolved in 150 ml ethyl acetate. Benzylamine (6.82 ml, 62.5 mmol) having a density of 0.981 was added to the reaction mixture with stirring at room temperature. An immediate precipitate formed. The reaction mixture was stirred overnight and filtered to give 13.4 g (80%) of white solid (mp = 174-176 °C) which was dried at room temperature under a vacuum. PMR ($CDCl_3/CD_3OD$) δ 7.70-7.56 (m, 4H), 7.40-7.25 (m, 5H) and 4.82 (s, 2H).

Step 2: Synthesis of N-Benzyl-N′-(4-Cyanophenyl)-S-Methylisothiourea:

A mixture of 10.0 g (37.5 mmol) of the compound obtained previously and 5.8 ml (d - 2.28; 93.1 mmol) of methyl iodide was stirred overnight in 125 ml of acetone. In the morning, the reaction was concentrated under reduced pressure and triturated with ether. The solid produced was dissolved in 50 ml of a 1 N solution of sodium hydroxide. The resulting alkaline mixture was extracted with ether. The ether layer was washed with saturated NaCl and dried with anhydrous magnesium sulfate. The ether layer was then concentrated under reduced pressure to give 9.5 g (90%) of a white solid (mp = 76-78 °C). PMR ($CDCl_3$) δ 7.53 (d, J = 8 Hz, 2H), 7.41-7.28 (m, 5H), 6.97 (d, J = 8 Hz, 2H), 4.88 (br s, 1H), 4.55 (s, 2H) and 2.27 (s, 3H).

Step 3: Preparation of 1′-N-[N-Benzylamino (4-Cyanophenylimino)methyl]-5-Aminomethyltetrazole:

A mixture of 5-aminomethyltetrazole (0.50 g, 5.05 mmol), sodium hydroxide (0.20 g, 5.00 mmol) and 1.5 ml of water was added to a solution of N-benzyl-N′-(4-cyanophenyl)-S-methyl-isothiourea (1.42 g, 5.05 mmol) in 15 ml of absolute ethanol. The mixture was heated to reflux for 20 hours. After cooling, the solution was concentrated to dryness and the residue was dissolved in 20 ml of water. The resulting solution was washed with ether (2 x 20 ml) and then neutralized with a 1 N HCl solution to bring its pH to 7.5. The desired guanidine compound precipitated and was separated out by filtration to give 0.74 g (43%) of the title compound. PMR ($CD_3COOD$) δ 7.71 (d, J = 8 Hz, 2H), 7.5-7.2 (m, 7H), 5.02 (s, 2H) and 4.08 (s, 2H) and 4.08 (S, 2H). CMR ($CD_3COOD$) δ 154.4, 153.6, 138.8, 134.1, 132.9, 127.9, 127.2, 126.5, 123.6, 116.9, 108.9, 45.2 and 35.0. Analysis calculated for $C_{17}H_{16}N_8$: C, 61.43; H,4.85; N, 33.71. Found: C, 61.38; H, 4.82; N, 33.91. The compound was tasted and was sweet at a concentration of 100 µg/ml.

EXAMPLE 9

Synthesis of 1′-N-[N-Cyclooctylamino (3,5-Dichlorophenylimino)methyl]-5-Aminomethyltetrazole:

Step 1: Preparation of N-Cyclooctyl-N′-(3,5-Dichlorophenyl) Thiourea:

3,5-Dichlorophenylisothiocyanate (4.0 g; 19.6 mmol) was dissolved in 40 ml ethyl acetate. Cyclooctylamine (2.7 ml, 19.7 mmol) having a density of 0.928 was added to the reaction mixture with stirring at room temperature. After 10 minutes, a precipitate formed. The reaction mixture was stirred overnight and filtered to give 2.6 g (40%) of white solid (mp = 146-147 °C). The filtrate was concentrated under reduced pressure and triturated with ether to give an additional 2.8 g (43%) of white solid. The combined solid (5.4 g, 83%) was dried at room temperature under a vacuum. PMR ($CDCl_3$) δ 8.0 (br s, 1H), 7.3-7.2 (m, 1H), 7.2-7.1 (m, 2H), 6.1 (br s, 1H), 4.49 (br s, 1H), 2.05-1.87 (m, 2H) and 1.8-1.54 (m, 12H).

Step 2: Synthesis of N-Cyclooctyl-N′-(3,5-Dichlorophenyl)-S-Methylisothiourea:

A mixture of 5.0 g (15.1 mmol) of the compound obtained previously and 2.35 ml (d = 2.28; 37.7 mmol) of methyl iodide was stirred overnight in 50 ml of acetone. In the morning, a solid was present and it was filtered to give 5.1 g (72%) of a white solid. The solid produced was dissolved in 20 ml of a 1 N solution of sodium hydroxide. The resulting alkaline mixture was extracted with ether. The ether layer was washed with saturated NaCl and dried with anhydrous magnesium sulfate. The ether layer was then concentrated under reduced pressure to give 3.5 g (69%) of a pale yellow oil that solidified upon standing (mp = 45-46 ° C). PMR ($CDCl_3$) δ 6.97 (t, J = 2 Hz, 1H), 6.79 (d, J = 2 Hz, 2H), 4.48 (br s, 1H), 3.92 (br s, 1H), 2.28 (s, 3H), 1.96-1.80 (m, 2H) and 1.70-1.45 (m, 12H). CMR ($CDCl_3$) δ 153.1, 151.6, 134.4, 121.6, 52.3, 32.0, 26.6, 25.1, 23.3 and 13.7.

Step 3: Preparation of 1′-N-[N-Cyclooctylamino (3,5-Dichlorophenylimino)methyl]-5-Aminomethyltetrazole:

A mixture of 5-aminomethyltetrazole (0.50 g, 5.05 mmol), sodium hydroxide (0.20 g, 5.00 mmol) and 1.5 ml of water was added to a solution of N-cyclooctyl-N′-(3,5-dichlorophenyl)-S-methylisothiourea (1.74 g, 5.04 mmol) in 15 ml of absolute ethanol. The mixture was heated to reflux for 20 hours. After cooling, the solution was concentrated to dryness and the residue was dissolved in 20 ml of water. The resulting solution was washed with ether (2 x 20 ml) and then neutralized with a 1 N HCl solution to bring its pH to 7.5. The desired guanidine compound precipitated and was separated out by filtration to give 1.6 g (80%) of the title compound. PMR ($CD_3OD$) δ 7.30 (s, 3H), 4.70 (s, 2H), 3.80 (m, 1H) and 1.96-1.40 (m, 14H). CMR ($CD_3OD$) δ 158.7, 154.3, 139.4, 136.0, 125.9, 122.3, 54.7, 37.6, 31.7, 27.3, 25.2 and 23.5. Analysis calculated for $C_{17}H_{23}N_7Cl_2$ (1.1 $H_2O$): C, 49.07; H, 6.10; N, 23.56. Found: C, 48.98; H, 6.10; N, 23.42. The compound was tasted and was sweet at a concentration of 100 µg/ml.

EXAMPLE 10

Synthesis of 1′-N-[N-Cyclohexylamino (3,5-Dichlorophenylimino)methyl]-5-Aminomethyltetrazole:

Step 1: Preparation of N-Cyclohexyl-N′-(3,5-Dichlorophenyl) Thiourea:

3,5-Dichlorophenylisothiocyanate (4.0 g; 19.6 mmol) was dissolved in 40 ml ethyl acetate. Cyclohexylamine (2.3 ml, 20.1 mmol) having a density of 0.867 was added to the reaction mixture with stirring at room temperature. The reaction mixture was stirred overnight and filtered to give 3.7 g (63%) of white solid (mp = 169-171 ° C). The filtrate was concentrated under reduced pressure and triturated with ether to give an additional 1.7 g (29%) of white solid. The combined solid (5.4 g, 91%) was dried at room temperature under a vacuum. PMR ($CDCl_3$) δ 8.60 (br s, 1H), 7.3-7.21 (m, 1H), 7.2-7.1 (m, 2H), 6.0 (br s, 1H), 4.21 (br s, 1H), 2.15-2.00 (m, 2H) 1.9-1.58 (m, 3H), 1.5-1.3 (m, 2H) and 1.3-1.1 (m, 3H).

Step 2: Synthesis of N-Cyclohexyl-N′-(3,5-Dichlorophenyl)-S-Methylisothiourea:

A mixture of 5.0 g (16.5 mmol) of the compound obtained previously and 2.6 ml (d = 2.28; 41.7 mmol) of methyl iodide was stirred at room temperature in 50 ml of acetone. After 40 minutes a precipitate formed. In the morning, the solid was filtered to give 7.1 g (97%) of a white solid. The solid produced was dissolved in 23 ml of a 1 N solution of sodium hydroxide. The resulting alkaline mixture was extracted with ether. The ether layer was washed with saturated NaCl and dried with anhydrous magnesium sulfate. The ether layer was then concentrated under reduced pressure to give 4.9 g (94%) of a white solid (mp = 95-96 ° C). PMR ($CDCl_3$) δ 6.98 (t, J = 2 Hz, 1H), 6.80 (d, J = 2 Hz, 2H), 4.40 (br s, 1H), 3.68 (m, 1H), 2.28 (s, 3H), 2.1-2.0 (m, 2H), 1.80-1.56 (m, 3H), 1.45-1.29 (m, 2H) and 1.25-1.07 (m, 3H).

Step 3: Preparation of 1′N-[N-Cyclohexylamino (3,5-Dichlorophenylimino)methyl]-5-Aminomethyltetrazole:

A mixture of 5-aminomethyltetrazole (0.50 g, 5.05 mmol), sodium hydroxide (0.20 g, 5.00 mmol) and 1.5 ml of water was added to a solution of N-cyclohexyl-N′-(3,5-dichlorophenyl)-S-methylisothiourea (1.6 g, 5.05 mmol) in 15 ml of absolute ethanol. The mixture was heated to reflux for 20 hours. After cooling, the solution was concentrated to dryness and the residue was dissolved in 20 ml of water. The resulting solution was washed with ether (2 x 20 ml) and then neutralized with a 1 N HCl solution to bring its pH to 7.5. The desired guanidine compound precipitated and was separated out by filtration to give 1.3 g (68%) of

the title compound. PMR ($CD_3COOD$) δ 7.34 (s, 1H), 7.25 (s, 2H), 5.0 (s, 2H), 3.6 (m 1H), 2.0-1.85 (m, 2H), 1.8-1.66 (m, 2H), 1.65-1.55 (m, 1H) and 1.42-1.02 (m, 5H). CMR ($CD_3COOD$) δ 155.3, 153.7, 137.8, 136.0, 127.2, 123.7, 52.9, 35.9, 32.5, 25.1 and 24.9. Analysis calculated for $C_{15}H_{19}N_7Cl_2$ (0.3 $H_2O$): C, 48.21; H, 5.29: N, 26.24. Found: C, 48.16; H, 5.13; N, 26.16. The compound was tasted and was sweet at a concentration of 1000 μg/ml.

EXAMPLE 11

Synthesis of 1′-N-[N-(S)-alpha-methylbenzylamino-(3,5-dimethylphenylimino)methyl]-5-aminomethyltetrazole:

N-(S)-alpha-methylbenzyl-N′-(3,5-dimethylphenyl)thiourea:

To a stirred solution of 3,5-dimethylphenylisothiocyanate (7.63 g, 46.7 mmol) in 60 ml of ethyl acetate was added 6.1 ml of (S)-alpha-methylbenzylamine (5.7 g, 47.3 mmol). The reaction mixture was stirred for 3 days. The resulting slurry was filtered and the product was washed with ether to afford 6.56 g (49.4%) of the desired thiourea. The filtrate was concentrated, slurried in ether, and filtered to yield an additional 3.20 g (24.1%) of the thiourea. PMR (DMSO-D6) ppm 9.31 (s, 1H, ArNH), 8.6 (d, 1H, J = 9 Hz, $NHCHCH_3$), 7.5-7.15 (m, 5H), 7.0 (s, 2H), 6.72 (s, 1H), 5.56 (m, 1H, CHMeN), 2.21 (s, 6H, $2CH_3$), 1.45 (d, 3H, J = 6 Hz, $CHCH_3$). [13]CMR (DMSO-D6) ppm 20.9, 21.8, 52.6, 120.7, 125.6, 126.2, 126.7, 128.3, 137.5, 139.2, 143.9 and 179.7.

N-(S)-alpha-methylbenzyl-N′-(3,5-dimethylphenyl)-S-methylisothiourea:

To a stirred suspension of the above thiourea (6.50 g, 22.8 mmol) in 65 ml of acetonitrile was added 2.84 ml of methyl iodide (6.49 g, 45.7 mmol). The resulting solution was stirred overnight. The solution was concentrated, dissolved in 50 ml of methylene chloride, washed with 1 N NaOH (40 ml), and water (25 ml). The organic layer was dried ($Na_2SO_4$), and concentrated to afford 6.61 g (96.8%) of the isothiourea as an oil. PMR (DMSO-D6) ppm 7.5-7.15 (m, 5H, Ph), 6.60 (d, 1H, J = 7.5 Hz, NHCH), 6.51 (s, 1H), 6.22 (s, 2H), 5.08 (dt, 1H, J = 7.7, 7.0 Hz), 2.32 (s, 3H, $SCH_3$), 2.15 (s, 6H, 2 $CH_3$), 1.45 (d, 3H, J = 7 Hz, $CHCH_3$). [13]CMR (DMSO-D6) ppm 150.7, 149.9, 145.0, 137.2, 128.0, 123.2, 119.6, 126.3, 126.0, 51.0, 22.4, 20.9, 13.7.

1′-N-[N-(S)-alpha-methylbenzylamino(3,5-dimethylphenylimino)methyl]-5-aminomethyltetrazole:

To a stirred solution of the above S-methylisothiourea (1.51 g, 5.06 mmol) in 15 ml of ethanol was added a solution of 5-aminomethyltetrazole (0.50 g, 5.1 mmol) and NaOH (.206 g, 5.15 mmol) in 2 ml of water. The resulting solution was refluxed for 20 hours. The reaction mixture was concentrated, dissolved in a water/ether mixture, the organic layer removed, and the aqueous layer washed with ether. The pH of the aqueous layer was adjusted to 6-7 with 1 N HCl, the guanidine precipitated as a gum. The mixture was stirred overnight, filtered and the white solid washed with water and air-dried to afford 1.07 g (60.5%) of the desired product. PMR (DMSO-D6) ppm 8.4 (bs, 2H), 7.5-7.2 (m, 5H, Ph), 6.8 (s, 2H, Ar), 6.7 (s, 2H, Ar), 4.95 (s, 1H, CH), 4.5 (s, 2H, $CH_2$), 2.21 (s, 6H, 2 $CH_3$), 1.5 (d, 3H, J = 7 Hz, $H_3CCH$). Analysis Calculated for $C_{19}H_{23}N_7$-1.25 $H_2O$: C, 61.35; H, 6.91; N, 26.36. Found: C, 61.33; H, 6.91; N, 26.36.

EXAMPLE 12

Synthesis of 1′-N-[N-(S)-alpha-methylbenzylamino (4-carbomethoxyphenylimino)methyl]-5-aminomethyltetrazole:

N-(S)-alpha-methylbenzyl-N′-(4-carbomethoxyphenyl)thiourea:

To a stirred solution of 4-carbomethoxyphenylisothiocyanate (5.52 g, 28.5 mmol) in 50 ml of ethyl acetate was added 3.9 ml of (S)-alpha-methylbenzylamine (3.7 g, 30 mmol). After stirring for 3 days, the reaction mixture was concentrated. The residue was slurried in ether, filtration afforded 6.73 g (75%) of the desired thiourea. The filtrate was concentrated, slurried in a minimal amount of ether and filtered to afford an additional 0.800 g (7.6%) of the thiourea. PMR (DMSO-D6) ppm 9.8 (s, 1H, NHAr), 8.5 (d, 1H, NHCH), 7.9 (d, 2H, J = 8.7 Hz, Ar), 7.75 (d, 2H, J = 8.7 Hz, Ar), 7.5-7.3 (m, 5H), 5.55 (m, 1H, CHNH), 3.8 (s, 3H), 1.5 (d, 3H, J = 7.3 Hz). [13]CMR (DMSO-D6) ppm 179.3, 165.8, 144.4, 143.5, 129.8, 128.3, 126.9, 123.8, 120.7, 120.6, 51.5, 51.8 and 21.8.

N-(S)-alpha-methylbenzyl-N′-(4-carbomethoxyphenyl)-S-methylisothiourea:

To a stirred suspension of the above thiourea (6.00 g, 19.1 mmol) in 60 ml of acetone was added 3.0 ml of methyl iodide (6.8 g, 48 mmol). After 15 hours, the reaction solution was concentrated, dissolved in 50 ml of methylene chloride, and washed with 1 N NaOH (30 ml) and water (25 ml). The solution was dried ($Na_2SO_4$) and concentrated to yield 5.92 g (94.4%) of the isothiourea. PRM ($CDCl_3$) ppm 7.95 (m, 2H), 7.45-7.2 (m, 5H, Ph), 6.9 (m, 2H), 5.17 (q, 1H, J=7.0 Hz, CHN), 4.8 (s, 1H, NH), 3.9 (s, 3H, $CO_2CH_3$), 2.2 (s, 3H, $SCH_3$), 1.5 (d, 3H, J=7.0 Hz, $CHC\underline{H}_3$).

1′-N-[N-(S)-alpha-methylbenzylamino(4-carbomethoxyphenylimino)methyl]-5-aminomethyltetrazole:

To a stirred solution of the above isothiourea (1.64 g, 4.99 mmol) in 15 ml of ethanol was added a solution of 5-aminomethyltetrazole (0.55 g, 5.6 mmol) and NaOH (0.22 g, 5.5 mmol) in 2 ml of water. The resulting solution was heated in a 70-75° C oil bath for 23 hours. The reaction mixture was concentrated, dissolved in a mixture of ether/water (25 ml/25 ml), the organic layer removed and the aqueous layer was washed with ether (25 ml). The pH of the aqueous layer was adjusted to 7 and the resulting suspension stirred overnight. The suspension was filtered and the product washed with water and air-dried to afford 0.72 g (38%) of product as a white powder. PMR (DMSO-D6) ppm 9.0 (bs, 1-2H), 7.9 (d, 2H, J=8.5 Hz), 7.45-7.15 (m, 7H), 5.0 (q, 1H, J=7 Hz, $C\underline{H}CH_3$), 4.60 (s, 2H, $CH_2NH$), 3.8 (s, 3H, $CO_2CH_3$), 1.51 (d, 3H, J=7 Hz, $CHC\underline{H}_3$). $^{13}$CMR (DMSO-D6) ppm 22.6, 37.8, 52.0, 52.1, 121.6, 125.3, 126.0, 127.4, 128.5, 130.6, 142.1, 142.5, 153.9, 158.1, 165.7. HPLC: 6.70 min. 95.7%. Analysis calculated for $C_{19}H_{21}N_7O_2$-0.34 $H_2O$: C, 59.16; H, 5.67; N, 25.42. Found: C, 59.15; H, 5.51; N, 25.39.

EXAMPLE 13

Synthesis of 1′-N-[N-(1-napthylamino)(4-cyanophenylimino)methyl]-5-aminomethyltetrazole:

N-(1-napthyl)-N′-(4-cyanophenylthiourea):

A mixutre of 1-napthylamine (7.53 g, 52.6 mmol) and 4-cyanophenylisothiocyanate (8.42 g, 52.6 mmol) in 75 ml of ethyl acetate was stirred overnight. The reaction mixture was concentrated, slurried in 150 ml of ethanol for 2 hours. The suspension was filtered and the purple solid washed with copious amounts of ether to afford after air-drying 15.5 g (95%) of the thiourea. PMR (DMSO-D6) ppm 10.22 (m, 2H), 8.1-7.4 (m, 11H). $^{13}$CMR (DMSO-D6) ppm 181.1, 144.3, 134.7, 133.9, 132.6, 129.7, 128.2, 128.1, 127.0, 126.9, 126.3, 126.1, 125.6, 125.3, 123.0, 122.6, 119.1, 105.4.

N-(1-napthyl-N′-(4-cyanophenyl)-S-methylisothiourea:

To a stirred suspension of above thiourea (6.00 g, 19.8 mmol) in 120 ml of acetone was added 2.5 ml of methyl iodide (5.7 g, 40 mmol). After 15 hours, an additional 1.2 ml of methyl iodide (2.7 g, 19 mmol) was added. The mixture was stirred an additional 24 hours and concentrated. The residue was dissolved in methylene chloride (50 ml), washed with 1 N NaOH (30 ml) and water (25 ml). The organic layer was dried ($Na_2SO_4$) and concentrated to afford 5.00 g (80%) of the isothiourea. PMR ($CDCl_3$) ppm 8.1-7.4 (m, 11H), 7.20 (d, 1H), 6.85 (bs, >1H), 2.19 (bs, 3H). $^{13}$CMR ($CDCl_3$) ppm 134.1, 133.1, 128.0, 126.1, 125.8, 128.7, 122.8, 120.4, 120.3, 119.2, 105.5, 14.7.

1′-N-[N-(1-napthylamino)(4-cyanophenylimino)-methyl]-5-aminomethyltetrazole:

To a stirred solution of the above isothiourea (1.59 g, 5.01 mmol) in 15 ml of ethanol was added a solution of 5-aminomethyltetrazole (0.532 g, 5.37 mmol) and NaOH (0.215 g, 5.38 mmol) in 2 ml of water. The resulting solution was refluxed for 20 hours. The reaction mixture was concentrated, dissolved in a mixture of 50 ml of water and 25 ml of ether. The ether layer was removed and the aqeous layer washed with 25 ml of ether. The pH of the aqueous was adjusted to 7 with 1 N HCl and the resulting suspension stirred overnight. Filtration, followed by air-drying afforded 1.31 g (70.8%) of the product as a light purple powder. PMR (DMSO-D6) ppm 8.2-7.0 (m, 11H), 4.7 (s, 2H). HPLC: 6.60 min 100%.

EXAMPLE 14

Synthesis of 1′-N-[N-(exo-2-norbornylamino)(4-cyanophenylimino)methyl]-5-aminomethyltetrazole:

N-(exo-2-norbornyl)-N′-(4-cyanophenyl)thiourea:

To a stirred suspension of 4-cyanophenylisothiocyanate (7.53 g, 47.0 mmol) in 80 ml of ethyl acetate was added 5.6 ml of exo-2-aminonorbornane (5.2 g, 47 mmol). After 23 hours, the reaction mixture was filtered and the precipitate washed with ether. The yellow tinged solid was air-dried to afford 11.46 g (89.8%) of the thiourea. Analysis calculated for $C_{15}N_{17}N_3S$: C, 66.39; H, 6.31, N, 15.48. Found: C, 65.98; H, 6.39; N, 15.35.

N-(exo-2-norbornyl)-N′-(4-cyanophenyl)-S-methylisothiourea:

To a stirred suspension of the above thiourea (6.00 g, 22.1 mmol) in 60 ml of acetone was added 2.8 ml of methyl iodide (6.3 g, 44 mmol) and stirred overnight. The resulting dark red solution was concentrated, dissolved in 50 ml of methylene chloride and washed with 1 N NaOH (30 ml) and water (25 ml). The organi phase was dried ($Na_2SO_4$) and concentrated to yield 6.05 g (95.9%) of the isothiourea. PMR (DMSO-D6) ppm 7.6 (d, 2H, J = 8.5 Hz), 6.85 (d, 2H, J = 8.5 Hz), 6.5 (d, 1H, J = 5.5 Hz), 3.6 (m, 1H) 2.4-2.1 (m, 2H), 2.24 (s, 3H, $SCH_3$), 1.7-1.3 (m, 5H), 1.1 (m, 3H). $^{13}$CMR (DMSO-D6) ppm 14.0, 26.1, 28.1, 34.9, 35.1, 38.2, 41.1, 56.1, 103.0, 119.6, 123.0, 132.7, 132.8, 152.5, 155.0.

1′-N-[N-(exo-2-norbornylamino)(4-cyanophenylimino)methyl]-5-aminomethyltetrazole:

To a stirred solution of the N-(exo-2-norbornyl)-N′-(4-cyanophenyl)-S-methylisothiourea (1.51 g, 5.30 mmol) in 15 ml of ethanol was added a solution of 5-aminomethyltetrazole (0.538 g, 5.43 mmol) and NaOH (0.212 g, 5.30 mmol) in 2.5 ml of water and the mixture refluxed for 24 hours. The reaction mixture was concentrated. The residue was dissolved in a mixture of water (50 ml) and ether (25 ml). The organic layer was removed and the aqueous layer washed with ether (25 ml). The pH of the aqueous layer was adjusted to 7 with 1 N NCl and stirred overnight. The suspension was filtered, the precipitate washed with water, and air-dried to afford a 1.69 g (94.4%) of the product as a white powder. PMR (DMSO-D6) ppm 7.84 (d, 2H, J = 7.5 Hz), 7.40 (d, 2H, J = 7.5 Hz), 4.6 (s, 2H), 3.60 (m, 2H), 2.30 (m, 2H), 1.7 (m, 1H), 1.6-1.4 (m, 4H), 1.2-0.9 (m, 3H). HPLC 6.31 min., 100%. Analysis calculated for $C_{17}H_{20}N_8$-2.98 $H_2O$: C, 52.32; H, 6.71; N, 28.71. Found: C, 52.31; H, 6.52; N, 28.82.

EXAMPLE 15

Synthesis of 1′-N-[N-(endo-2-norbornylamino)(4-cyanophenylimino)methyl]-5-aminomethyltetrazole:

N-(endo-2-norbornyl)-N′-(4-cyanophenyl)thiourea:

To a stirred suspension of 4-cyanophenylisothiocyanate (4.10 g, 25.6 mmol) in 60 ml of ethyl acetate was added endo-2-aminonorbornane (3.0 g, 26.9 mmol). After 18 hours, the reaction mixture was diluted with 50 ml of ethanol and stirred for 5 hours. The reaction mixture was filtered and the precipitate washed with either. The yellow tinged solid was air-dried to afford 4.66 g (67.1%) of the thiourea. PMR (DMSO-D6) ppm 9.8 (s, 1H, NHAr), 8.16 (d, 2H, J = 8 Hz), 7.85 (d, 2H, J = 8 Hz), 7.7 (d, 2H, J = 8 Hz), 4.4 (m, 1H), 2.5 (m, 1H), 2.2 (s, 1H), 2.0 (m, 1H) 1.7-1.15 (m, 1H), 0.85 (m, 1H).

N-(endo-2-norbornyl)-N′-(4-cyanophenyl)-S-methylisothiourea:

To a stirred suspension of the above thiourea (2.00 g, 7.37 mmol) in 25 ml of acetone was added 0.9 ml of methyl iodide (2.1 g, 14 mmol) and stirred for 45 hours. The resulting dark red solution was concentrated, dissolved in 50 ml of methylene chloride and washed with 1 N NaOH (10 ml) and water (25 ml). The organic phase was dried ($Na_2SO_4$) and concentrated to yield 2.11 g (quantitative yield) of the isothiourea. PMR ($CDCl_3$) ppm 7.52 (m, 2H), 6.96 (m, 2H), 4.7 (s, 1H, NH), 4.06 (m, 1H, 2.55 (s, 1H), 2.25 (s, 3H, $SCH_3$), 2.4-2.05 (m, 2H), 1.7-1.1 (m, 7H), 0.75 (m, 1H).

1′-N-[N-(endo-2-norbornyamino)(4-cyanophenylimino)methyl]-5-aminomethyltetrazole:

To a stirred solution of the N-(endo-2-norbornyl-N′-(4-cyanophenyl)-S-methylisothiourea (1.61 g, 5.64 mmol) in 15 ml of ethanol was added a solution of 5-aminomethyltetrazole (0.580 g, 5.85 mmol) and NaOH (0.234 g, 5.85 mmol) in 2.5 ml of water and the mixture refluxed for 18 hours. The reaction mixture was concentrated. The residue was dissolved in a mixture of water (50 ml) and ether (25 ml). The organic layer was removed and the aqueous layer washed with ether (25 ml). The pH of the aqueous layer was adjusted to 7 with 1 N HCl and stirred overnight. The suspension was filtered, the precipitate washed with water, and air-dried to afford a 1.27 g (66.8%) of the product as a white powder. PMR ($CD_3CO_2D$) ppm 7.74 (d, 2H, J=9.0 Hz, Ar), 7.4 (d, 2H, J=9.0 Hz, Ar), 5.0 (s, 2H, $CH_2C$), 4.0 (m, 1H, CH), 2.5 (m, 1H), 2.2 (s, 1H), 2.0 (m, 1H), 1.65-0.9 (m, 7H), HPLC 6.13 min. 100%. Analysis calculated for $C_{17}H_{20}N_8$-0.15 $H_2O$: C, 60.21; H, 6.03; N, 33.04. Found: C, 60.19; H, 5.83; N, 32.88.

EXAMPLE 16

Synthesis of 1′-N-[N-(cycloheptylmethylamino)(4-cyanophenylimino)methyl]-5-aminomethyltetrazole:

N-cycloheptylmethyl-N′-(4-cyanophenyl)thiourea:

To a stirred suspension of 4-cyanophenylisothiocyanate (4.28 g, 26.7 mmol) in 35 ml of ethyl acetate was added cycloheptylmethylamine (3.40 g, 26.7 mmol) and the reaction mixture was stirred overnight. The reaction solution was concentrated and the residue slurried in ether. The resulting suspension was filtered and the precipitate washed with ether to afford 6.52 g (88.7%) of the thiourea as a white powder. PMR ($CDCl_3$) ppm 8.8 (s, 1H), 7.7 (d, 2H, J=9.0 Hz), 7.4 (d, 2H, J=9.0 Hz), 6.5 (s, 1H), 3.45 (s, 2H), 2.0-1.0 (m, 13H). Analysis calculated for $C_{16}H_{21}N_3S$: C, 66.86; H, 7.36; N, 14.62. Found: C, 66.99; H, 7.47; N, 14.61.

N-cycloheptylmethyl-N′-(4-cyanophenyl)-S-methylisothiourea:

To a stirred suspension of the above thiourea (3.00 g, 10.9 mmol) in 25 ml of acetone was added 1.7 ml of methyl iodide (3.9 g, 27 mmol) and stirred overnight. The reaction solution was concentrated, dissolved in 50 ml of methylene chloride and washed with 1 N NaOH (15 ml) and water (25 ml). The organic phase was dried ($Na_2SO_4$) and concentrated to yield 3.4 g (quantitative yield) of the isothiourea as a yellow oil which slowly solidified. PMR ($CDCl_3$) ppm 7.55 (d, 2H, J=9.0 Hz), 6.95 (d, 2H, J=9.0 Hz), 4.7 (s, 1H), 3.2 (bs, 2H), 2.25 (s, 3H, 1.9-1.1 (m, 13H).

1′-N-[N-cycloheptylmethylamino(4-cyanophenylimino)methyl]5-aminomethyltetrazole:

To a stirred solution of the above isothiourea (1.36 g, 4.70 mmol) in 15 ml of ethanol was added a solution of 5-aminomethyltetrazole (0.466 g, 4.70 mmol) and NaOH (0.188 g, 4.70 mmol) in 2.5 ml of water and the mixture refluxed for 50 hours. The reaction mixture was concentrated. The residue was dissolved in a mixture of water (50 ml) and ether (25 ml). The organic layer was removed and the aqueous layer washed with ether (25 ml). The pH of the aqueous layer was adjusted to 7 with 1 N HCl and stirred overnight. The suspension was filtered, the precipitate washed with water, and air-dried to afford a 0.847 g (53%) of the product as a white powder. This was recrystallized from acetonitrile/water to afford 400 mg of the desired material. PMR ($CD_3CO_2D$) ppm 7.75 (d, 2H, J=9.0 Hz), 7.56 (d, 2H, J=9.0 Hz), 5.0 (s, 2H), 3.15 (d, 1H, J=7 Hz), 1.9-0.9 (m, 12H). Analysis calculated for $C_{18}H_{24}N_8$-0.88 $H_2O$: C, 58.70; H, 7.24; N, 30.43. Found: C, 58.03; H, 7.24; N, 30.81. HPLC 8.10 min., 100%.

EXAMPLE 17

Synthesis of 1′-N-[N-nonylamino(4-cyanophenylimino)methyl]-5-aminomethyltetrazole:

N-nonyl-N′-(4-cyanophenyl)thiourea:

To a stirred solution of 7.0 ml nonylamine (5.5 g, 38 mmol) in 50 ml of ethyl acetate was added 4-cyanophenylisothiocyanate (6.00 g, 37.5 mmol) and the mixture stirred overnight. The resulting suspension was filtered and the precipitate washed with ether to afford 1.81 g (15.9%) of the thiourea as a white powder. The filtrate was concentrated, slurried in ether, and filtered to afford an additional 7.10 g (62.3%) of

the thiourea. PMR ($CDCl_3$) ppm 8.9 (s, 1H), 7.6 (d, 2H, Ar), 7.44 (d, 2H, Ar), 6.53 (s, 1H), 3.60 (m, 2H, $NCH_2$), 1.6 (m, 2H), 1.45-1.1 (m, 12H), 1.26 (t, 3H, $CH_3$). Analysis calculated for $C_{17}H_{25}N_3S$: C, 67.28; H, 8.30; H, 13.85. Found: C, 67.03, H, 8.28; N, 13.76.

N-nonyl-N′-(4-cyanophenyl)-S-methylisothiourea:

To a stirred solution of the above thiourea (5.00 g, 16.5 mmol) in 50 ml of acetone was added 2.6 ml of methyl iodide (5.9 g, 41 mmol) and the mixture stirred overnight. The reaction solution was concentrated, dissolved in 50 ml of methylene chloride and washed with 1 N NaOH (15 ml) and water (25 ml). The organic phase was dried ($Na_2SO_4$) and concentrated to yield 5.35 g (crude quantitative yield) of the isothiourea as an oil which solidified. PMR ($CDCl_3$) ppm 7.53 (d, 2H, J = 8.5 Hz, Ar), 6.95 (d, 2H, J = 8.5 Hz, Ar), 4.6 (s, 1H, NH), 3.3 (t, 2H, J = 6.5 Hz, $NCH_2$), 2.25 (s, 3H, $SCH_3$), 1.58 (m, 2H), 1.4-1.1 (m, 12H), 0.88 (t, 3H, J = 8.5 Hz, $CH_2\underline{CH_3}$).

1′-N-[N-nonylamino)(4-cyanophenylimino)methyl]-5-aminomethyltetrazole:

To a stirred suspension of the above isothiourea (1.92 g, 6.05 mmol) in 20 ml of ethanol was added a solution of 5-aminomethyltetrazole (0.600 g, 6.05 mmol) and NaOH (0.242 g, 6.05 mmol) in 4 ml of water and the mixture refluxed for 20 hours. The reaction mixture was concentrated. The residue was dissolved in a mixture of water (50 ml) and ether (25 ml). A bad emulsion resulted, the aqueous phase was washed with an additional 25 ml of ether. The pH of the aqueous layer was adjusted to 7 with 1 N HCl and stirred overnight. The suspension was filtered, the precipitate washed with water, and air-dried to afford a 1.32 g (59.5%) of the product as a white powder. PMR (DMSO-D6) ppm 7.85 (d, 2H, J = 7.5 Hz), 7.4 (d, 2H, J = 7.5 Hz), 4.6 (s, 2H), 3.25 (t, 2H, J = 7 Hz), 1.55 (m, 2H), 1.25 (m, 12H), 0.85 (t, J = 7 Hz). Analysis calculated for $C_{19}H_{28}N_8$-0.61 $H_2O$: C, 60.14; H, 7.76; N, 29.53. Found: C, 60.12; H, 7.66; N, 29.63. HPLC: 10.81 min. 98.2%.

EXAMPLE 18

Synthesis of 1′-N-[N-dodecylamino(4-cyano phenylimino)methyl]-5-aminomethyltetrazole:

N-dodecyl-N′-(4-cyanophenyl)thiourea:

To a stirred solution of dodecylamine (4.86 g, 26.2 mmol) in 80 ml of ethyl acetate was added 4-cyanophenylisothiocyanate (4.20 g, 26.2 mmol) and the mixture stirred for 16 hours. The resulting suspension was filtered and the precipitate washed with ether to afford 4.53 g (50%) of the thiourea as a white powder. the filtrate was concentrated, slurried in ether, and filtered to afford an additional 3.71 g (41.0%) of the thiourea. PMR ($CDCl_3$) ppm 8.40 (s, 1H, NH), 7.7 (d, 2H, J = 8.5 Hz, Ar), 7.36 (d, 2H, Ar), 6.28 (s, 1H, NH), 3.62 (m, 2H, $CH_2N$), 1.6 (m, 2H), 1.4-1.15 (m, 18H), 0.88 (t, 3H, J = 7.0 Hz, $CH_3$). Analysis calculated for $C_{20}H_{31}N_3S$: C, 69.52; H, 9.04; N, 12.16. Found: C, 69.73; H, 9.02; N, 12.14.

N-dodecyl-N′-(4-cyanophenyl)-S-methylisothiourea:

To a stirred solution of the above thiourea (4.00 g, 11.6 mmol) in 50 ml of acetone was added 1.8 ml of methyl iodide (4.1 g, 29 mmol) and the mixture stirred overnight. The reaction solution was concentrated, dissolved in 50 ml of methylene chloride and washed with 1 N NaOH (20 ml) and water (25 ml). The organic phase was dried ($Na_2SO_4$) and concentrated to yield 4.10 g (98.3%) of the isothiourea as a wax. PMR ($CDCl_3$) ppm 7.55 (d, 2H, J = 9H, Ar), 6.95 (d, 2H, J = 9 Hz, Ar), 4.65 (s, NH), 3.42 (t, 2H, J = 7.5 Hz, $NCH_2$), 2.25 (s, 3H, $SCH_3$), 1.68 (m, 2H), 1.4-1.15 (m, 19H), 0.88 (t, 2H, J = 7.5 Hz). $^{13}$CMR ($CDCl_3$) ppm 14.1, 22.7, 26.9, 29.3, 29.3, 29.4, 29.5, 29.5, 29.6, 31.9, 43.3, 105.0, 119.7, 123.0, 133.0, 133.1, 154.3.

1′-N-[N-dodecylamino)(4-cyanophenylimino)-methyl]-5-aminomethyltetrazole:

To a stirred suspension of the above isothiourea (2.92 g, 8.13 mmol) in 20 ml of ethanol was added a solution of 5-aminomethyltetrazole (0.805 g, 8.13 mmol) and NaOH (0.325 g, 8.13 mmol) in 4 ml of water and the mixture refluxed for 20 hours. The reaction mixture was concentrated. The residue was dissolved in a mixture of water (50 ml) and ether (25 ml). A bad emulsion resulted, the aqueous phase was washed with an additional 25 ml of ether. The pH of the aqueous layer was adjusted to 7 with 1 N HCl and stirred

overnight. The suspension was filtered, the precipitate washed with water, and air-dried to afford a 2.59 g (77.6%) of the product as a powder. PMR (DMSO-D6) ppm 7.85 (d, 2H, J = 9 Hz), 7.4 (d, 2H, J = 9 Hz), 4.58 (s, 2H), 3.25 (t, 2H, J = 7.5 Hz), 1.55 (m, 2H), 1.4-1.1 (m, 18H), 0.85 (t, 3H, J = 7.5 Hz). HPLC: 14.3 minutes 98.7%. Analysis calculated for $C_{22}H_{34}N_8$: C, 64.36; H, 8.35; N, 27.29. Found: C, 64.05; H, 8.58; N, 27.31.

EXAMPLE 19

Synthesis of 1′-N-[N-phenylsulfonylamino(3,5-dichlorophenylimino)methyl]-5-aminomethyltetrazole:

N-Benzenesulfonyl-N′-(3,5-dichlorophenyl)thiourea Sodium Salt:

In 50 ml of acetone were dissolved 6.33 g (31 mmol) of 3,5-dichlorophenylisothiocyanate and 6.3 g (40 mmol) of benzenesulfonamide. With stirring was added 1.6 g (40 mmol) of NaOH dissolved in 3 ml of water. After stirring overnight, 1.84 g (9 mmol) of additional isothiocyanate was added and allowed to react for an additional 2 hours. The solvent was removed under reduced pressure and the yellow solid triturated with ether to yield 10.74 g (71%) of a white solid after drying. $^{1}H$ NMR ($Me_2SO$-D6, 300 MHz) $\delta$ 7.4 (m, 8H), 9.45 (s, 1H); $^{13}C$ NMR ($Me_2SO$-D6, 75.5 MHz) $\delta$ 182.3, 144.8, 143.5, 133.4, 130.2, 127.8, 127.1, 119.9, 117.5.

N-Benzenesulfonyl-N′-(3,5-dichlorophenyl)-S-methylisothiourea:

To 50 ml of absolute ethanol were added 10 g (26 mmol) of the sodium salt of N-benzenesulfonyl-N′-(3,5-dichlorophenyl)thiourea and 2.5 ml (39 mmol) of iodomethane and the slurry stirred for 30 hours. The solvent was removed under reduced pressure and the residue redissolved in methylene chloride. The solution was washed with water (2 x 50 ml) and the organic layer dried with anhydrous $MgSO_4$. The solvent was removed under reduced pressure to yield 12.23 g of an off white solid. The crude product was slurried in 50 ml of ether and the product isolated by filtration to yield 8.07 g (83%) of the desired compound. TLC analysis (1:9 $EtOAcCH_2Cl_2$) swuv visualization) showed a single spot at $R_f$ 0.57. $^{1}H$ NMR ($CDCl_3$, 300 MHz) $\delta$ 2.33 (s, 3H), 7.6 (m, 8H) 9.8 (s, 1H); $^{13}C$ NMR ($CDCl_3$, 75.5 MHz) $\delta$ 168.9, 141.8, 137.7, 135.5, 132.6, 128.9, 128.5, 126.3, 125.6, 14.7.

1′-N-[N-phenylsulfonylamino)3,5-dichlorophenylimino)methyl]-5-aminomethyltetrazole:

To 3.75 g (10 mmol) of N-benzenesulfonyl-N′-(3,5-dichlorophenyl)-S-methylisothiourea dissolved in 70 ml absolute ethanol was added a solution containing 0.99 g (10 mmol) of 5-aminomethyltetrazole and 0.40 g (10 mmol) of NaOH in 7 ml of water. The clear solution was refluxed for 2 days and the solvent removed under reduced pressure. The residue was dissolved in 300 ml water and washed with ether (2 x 100 ml). The aqueous layer as adjusted to pH 3.5 with 1 N HCl and the product isolated by filtration and dried (50 °C and 10 mmHg to yield 3.24 g (76%) of the desired product. HPLC indicated the compound to be 97.7% pure. $^{1}H$ NMR ($Me_2SO$-D6, 300 MHz) $\delta$ 4.72 (s, 2H), 7.5 (m, 8H), 8.15 (t, 1H), 9.25 (s, 1H); $^{13}C$ NMR ($Me_2SO$-D6, 75.5 MHz) $\delta$ 154.8, 153.2, 143.3, 140.0, 133.9, 131.6, 128.7, 125.5, 36.0. Analysis calculated for $C_{15}H_{13}N_7Cl_2SO_2$ (0.5 $H_2O$): C, 41.38; H, 3.01; N, 22.53; S, 7.36. Found: C, 41.53; H, 3.1; N, 23.07; S, 7.27.

EXAMPLE 20

Synthesis of 1′-N-[N-phenylsulfonylamino(4-cyanophenylimino)methyl]-5-aminomethyltetrazole:

N-Benzenesulfonyl-N′-(4-cyanophenyl)thiourea Sodium Salt:

In 50 ml of acetone were dissolved 5.0 g (31 mmol) of 4-cyanophenylisothiocyanate and 6.3 g (40 mmol) of benzenesulfonamide. With stirring was added 1.6 g (40 mmol) of NaOH dissolved in 3 ml of water. After stirring overnight, solids were recovered by filtration and after an ether wash yielded 7.85 g (75%) of a white solid. $^{1}H$ NMR ($Me_2SO$-D6, 300 MHz) $\delta$ 7.7 (m, 9H), 9.55 (s, 1H); $^{13}C$ NMR ($Me_2SO$-D6, 75.5 MHz) $\delta$ 182.7, 145.3, 144.5, 132.5, 130.1, 127.6, 127.3, 119.8, 119.3, 40.0.

N-Benzenesulfonyl-N′-(4-cyanophenyl)-S-methylisothiourea:

To 50 ml of absolute ethanol were added 7.0 g (20.6 mmol) of the sodium salt of N-benzenesulfonyl-N′-(4-cyanophenyl)thiourea and 1.9 ml (30 mmol) of iodomethane and the slurry stirred for 30 hours. The

solvent was removed under reduced pressure and the residue redissolved in methylene chloride. The solution was washed with water (2 x 50 ml) and the organic layer dried with anhydrous $MgSO_4$. The solvent was removed under reduced pressure to yield 6.29 g (92%) of an off-white solid. TLC analysis (1:9 EtOAc-$CH_2Cl_2$, swuv visualization showed a single spot at $R_f$ 0.49. $^1H$ NMR ($Me_2CO$-D6, 300 MHz) δ 2.47 (s, 3H), 7.75 (m, 9H), 9.65 (s, 1H); $^{13}C$ NMR ($Me_2CO$-D6, 75.5 MHz) δ 205.8, 141.5, 133.6, 133.5, 133.4, 132.8, 129.4, 126.9, 126.84, 126.8, 118.4, 14.6.

1′-N-[N-phenylsulfonylamino(4-cyanophenylimino)methyl]-5-aminomethyltetrazole:

To 3.3 g (10 mmol) of N-benzenesufonyl-N′-(4-cyanophenyl)-S-methylisothiourea dissolved in 70 ml absolute ethanol was added a solution containing 0.99 g (10 mmol) of 5-aminomethyltetrazole and 0.40 g (10 mmol) of NaOH in 7 ml of water. The clear solution was refluxed for 2 days, cooled, and the solvent removed under reduced pressure. The residue was dissolved in 300 ml water and washed with ether (2 x 100 ml). The aqueous layer was adjusted to pH 3.5 with 1 N HCl and the product isolated by filtration and dried at 50°C and 10 mmHg to yield 3.11 g (81%) of the desired product. HPLC indicated the compound to be 98.0% pure. $^1H$ NMR ($Me_2SO$-D6, 300 MHz) δ 4.8 (s, 2H), 7.65 (m, 9H), 8.3 (t, 1H), 9.25 (s, 1H); $^{13}C$ NMR ($Me_2SO$-D6) δ 154.8, 153.2, 143.3, 141.9, 133.1, 131.7, 128.8, 125.5, 123.7, 118.7, 106.0, 36.0. Analysis calculated for $C_{16}H_{14}N_8SO_2$: C, 50.25; H, 3.69; N 29.30; S, 8.38. Found: C, 48.98; H, 3.65; N, 29.08; S, 8.33.

## EXAMPLE 21

Synthesis of 1′-N-[N-(2,4,6-triisopropylbenzenesulfonyl)amino(4-cyanophenylimino)methyl]-5-aminomethyltetrazole:

N-(2,4,6-triisopropylbenzenesulfonyl-N′-(4-cyanophenyl)thiourea Sodium Salt:

In 50 ml of acetone were dissolved 3.95 g (24.7 mmol) of 4-cyanophenylisothiocyanate and 7.0 g (24.7) mmol) of triisopropylbenzenesulfonamide. With stirring was added 1.0 g (25 mmol) of NaOH dissolved in 3 ml of water. After stirring for 4 hours, the solvent was removed under reduced pressure and the residual bright yellow solids slurried in diethyl ether. The product was recovered by filtration and after thoroughly drying yielded 9.56 g (83%) of the desired compound. $^1H$ NMR ($Me_2SO$-D6, 300 MHz) δ 0.8 (d, 12H), 0.9 (d, 6H), 2.6 (m 1H), 4.4 (m, 2H), 6.75 (s, 2H), 7.3-7.8 (m, 4H), 9.0 (s, 1H).

N-(2,4,6-triisopropylbenzenesulfonyl-N′-(4-cyanophenyl)-S-methylisothiourea:

To 50 ml of absolute ethanol were added 9.33 g (20 mmol) of the sodium salt of N-(2,4,6-triisopropylbenzenesulfonyl-N′-(4-cyanophenyl)thiourea and 1.9 ml (30 mmol of iodomethane and the slurry stirred for 24 hours. The solvent was removed under reduced pressure and the residue slurried in diethyl ether. The product was recovered by filtration and dried to yield 10 g of the desired compound. $^1H$ NMR ($Me_2SO$-D6, 300 MHz), δ 1.1 (d, 12H), 1.18 (d, 6H), 2.55 (s, 3H), 2.9 (m, 1H), 4.25 (m, 2H), 7.15 (s, 2H), 7.7-7.85 (dd, 4H), 9.65 (s, 1H). Analysis calculated for $C_{24}H_{31}N_3O_2S_2$.NaI: C, 47.4; H, 5.3; N, 6.9. Found: C, 47.8; H, 5.23; N, 6.98.

1′-N-[N-(2,4,6-triisopropylbenzenesulfonyl)-amino(4-cyanophenylimino)methyl]-5-aminomethyltetrazole:

To 4.58 g (7.5 mmol) of N-(2,4,6-triisopropylbenzenesulfonyl)-N′-(4-cyanophenyl)-S-methylisothiourea dissolved in 70 ml absolute ethanol was added a solution containing 0.99 g (10 mmol) of 5-aminomethyltetrazole and 0.40 g (10 mmol) of NaOH in 7 ml of water. The clear solution was refluxed for 2 days and the solvent removed under reduced pressure. The residue was dissolved in 300 ml water and washed with ether (2 x 200 ml). The aqueous layer was adjusted to pH 3.5 with 1 N HCl and the product isolated by filtration and dried (50°C and 10 mmHg) to yield 3.14 g (83%) of the desired compound. HPLC indicated the compound to be 99.0% pure. $^1H$ NMR ($ME_2SO$-D6, 300 MHz) δ 1.08 (d, 12H), 1.15 (d, 6H), 2.85 (m, 1H), 4.3 (m, 2H), 4.9 (s, 2H), 7.1 (s, 2H), 7.5-7.75 (dd, 4H), 8.25 (t, 1H), 9.2 (s, 1H); $^{13}C$ NMR ($Me_2SO$-D6, 75.5 MHz ) δ 152.2, 151.1, 148.4, 142.4, 136.9, 133.0, 122.95, 122.8, 118.85, 106.1, 36.05, 33.3, 28.75, 24.55, 23.5. Analysis calculated for $C_{25}H_{32}N_8SO_2$ (0.5 $H_2O$): C, 58.0; H, 6.23; N, 21.65; S, 6.2. Found: C, 57.6; H, 6.35; N, 21.68; S, 6.08.

EXAMPLE 22

Synthesis of 1′-N-[N-(2,4,6-trimethylbenzene sulfonyl)amino(4-cyanophenylimino)methyl-5-aminomethyltetrazole:

2,4,6-Trimethylbenzenesulfonamide:

To 30 ml of concentrated ammonium hydroxide was added 3.00 g (13.7 mmol) of mesitylenesulfonyl chloride. The solution was heated under reflux for 10 minutes and then poured into 200 ml of cold water. The crude product was filtered and redissolved in 10 ml of hot ethanol. 200 ml of hot water was added and after cooling, filtering, and drying 2.0 g (74%) of white needles were isolated, mp 144-145° C.

N-(2,4,6-trimethylbenzenesulfonyl)-N′-(4-cyanophenyl)thiourea Sodium Salt:

In 25 ml of acetone were dissolved 1.6 g (10 mmol) of 4-cyanophenyl isothiocyanate and 2.0 g (10 mmol) of trimethylbenzenesulfonamide. With stirring was added 0.40 g (10 mmol) of NaOH dissolved in 1 ml of water. After stirring for 4 hours solids were recovered by filtration and washed with cold acetone and diethyl ether to give 1.75 g (49%) of a product with mp greater than 250° C. $^1$H NMR ($Me_2SO$-D6, 300 MHz) δ 1.95 (s, 3H), 2.3 (s, 6H), 6.5 (s, 2H), 7.3-7.8 (m, 4H), 9.1 (s, 1H); Analysis calculated for $C_{17}H_{16}N_3O_2S_2Na$: C, 53.52; H, 4.19; N 11.02. Found: C, 53.26; H, 4.10; N 10.92.

N-(2,4,6-trimethylbenzenesulfonyl)-N′-(4-cyanophenyl)-S-methylisothiourea:

To 25 ml of absolute ethanol were added 1.7 g (4.5 mmol) of the sodium salt of N-(2,4,6-trimethylbenzenesulfonyl-N′-(4-cyanophenyl)thiourea and 0.42 ml (6.7 mmol) of iodomethane and the slurry stirred for 30 hours. The solvent was removed under reduced pressure and the residue slurried in diethyl ether. The product was isolated by filtration and dried (50° C, 10 mmHg) to yield 1.88 g of the desired product (mp 183-186° C). $^1$H NMR ($Me_2SO$-D6, 300 MHz) δ 2.5 (s, 3H), 2.68 (s, 6H), 2.72 (s, 3H), 7.15 (s, 2H), 7.8-8.1 (m, 4H); $^{13}$C NMR ($Me_2SO$-D6, 75.5 MHz) δ 164.2, 142.1, 141.2, 137.7, 136.4, 133.0, 132.9, 131.3, 123.9, 118.6, 107.6, 22.3, 20.4, 15.4.

1′-N-[N-(2,4,6-trimethylbenzenesulfonyl)amino-(4-cyanophenylimino)methyl-5-aminomethyltetrazole:

To 1.5 g (4.0 mmol) of N-(2,4,6-trimethylbenzenesulfonyl)-N′-(4-cyanophenyl)-S-methylisothiourea dissolved in 70 ml absolute ethanol was added a solution containing 0.40 g (4.0 mmol) of 5-aminomethyltetrazole and 0.16 g (4.0 mmol) of NaOH in 7 ml of water. The clear solution was refluxed for 2 days, cooled, and the solvent removed under reduced pressure. The residue was dissolved in 300 ml water and washed with ether (2 x 100 ml). The aqueous layer was adjusted to pH 3.5 with 1 N HCl and the product isolated by filtration and dried at 50° C and 10 mmHg to yield 0.83 g (49.%) of the desired product. HPLC indicated the compound to be 99.2% pure. $^1$H NMR ($Me_2SO$-D6, 300 MHz) δ 2.0 (s, 3H), 2.3 (s, 6H), 4.6 (s, 2H), 6.7 (s, 2H), 7.2-7.6 (dd, 4H), 8.1 (t, 1H), 9.0 (s, 1H), $^{13}$C NMR ($Me_2SO$-D6, 75.5 MHz δ 156, 153.3, 142.8, 141.1, 138.4, 137.8, 133.7, 131.8, 123.6, 119.5, 106.9, 36.6, 22.9, 21.0.

EXAMPLE 23

Synthesis of 1′-N-[N-Methylamino (3,5-dichlorophenylimino)methyl]-5-aminomethyltetrazole:

N-(3,5-Dichlorophenyl)-N′-methylthiourea:

A suspension of 3,5-dichlorophenylisothiocyanate (5.0 g, 24.5 mmol) in 35 ml of absolute ethanol was treated dropwise with 2.5 ml of aqueous methylamine (40 wt. % soln, 29.4 mmol). The reaction was exothermic and precipitate formation was noted after ca. 5 min. The reaction mixture was further diluted with 10 ml of ethanol, stirred at 25° C for an additional 1.5 h, and then concentrated under reduced pressure. The residue was triturated with hexane to give 5.05 g (88%) of a white solid: $^1$H NMR (DMSO-D6) δ 2.92 (d, 3H, J = 4.4 Hz), 7.24 (s, 1H), 7.54 (s, 2H), 8.03 (br s, 1H) and 9.81 (br s, 1H).

N-(3,5-Dichlorophenyl)-N′-methyl-S-methylisothiourea:

To a solution of the thiourea prepared above (5.05 g, 21.4 mmol) in 70 ml of acetone was added 3.3 ml of methyl iodide (7.62 g, 53.7 mmol). The resulting yellow solution was then stirred at room temperature overnight. After ca. 16 h, the reaction mixture was filtered and the precipitate washed with ether. The solid was dissolved in 1 N aqueous sodium hydroxide and the alkaline solution was then extracted with ether. The ether layer was dried over anhydrous sodium sulfate, filtered, and concentrated under reduced pressure to give 5.16 g (97%) of a colorless oil: $^1H$ NMR ($CDCl_3$) δ 2.28 (s, 3H), 2.92 (s, 3H), 4.58 (br s, 1H), 6.80 (d, 2H, J = 2 Hz) and 6.99 (d, 1H, J = 2 Hz).

$1^1$-N-[N-Methylamino(3,5-dichlorophenylimino)methyl]-5-aminomethyltetrazole:

A mixture of 5-aminomethyltetrazole (0.50 g, 5.05 mmol), sodium hydroxide (0.20 g, 5.05 mmol), and 1.5 ml of water was added to a solution of N-(3,5-dichlorophenyl-N′-methyl-S-methylisothiourea (1.26 g, 5.05 mmol) in 15 ml of absolute ethanol. The reaction mixture was heated at reflux for 24 h, then cooled to room temperature and concentrated at reduced pressure. Addition of water and ether to the solid residue yielded an unbreakable emulsion. After removal of the organic solvent, the aqueous suspension was adjusted to pH 7.5 with 10% aqueous HCl and then filtered. The precipitate was washed with water and hexane, and then dried in vacuo to give 1.02 g (67%) of a white solid (d > 260 ° C): IR (Nujol) 3300, 2923, 2853, 1652, 1573, 1445, 1390, 1335, 1252, 1176, 1102, 1004, 842 and 799 $cm^{-1}$; $^1H$ NMR (DMSO-D6) δ 2.84 (s, 3H), 4.55 (s, 2H), 7.31 (s, 2H) and 7.41 (s, 1H); $^{13}C$ NMR (DMSO-D6) δ 28.9, 37.5, 121.9, 124.4, 134.6, 140.6, 155.1 and 157.9; An analytical sample was recrystallized from ethanol-water. Analysis calculated for $C_{10}H_{11}Cl_2N_7$ (1.06 $H_2O$): C, 37.60; H, 4.15; N, 30.70. Found: C, 37.60; H, 4.04; N, 30.60.

EXAMPLE 24

Synthesis of 1′-N-[N-1-Naphthylamino (3,5-dichlorophenylimino)methyl]-5-aminomethyltetrazole:

N-(3,5-Dichlorophenyl)-N′-1-naphthylthiourea:

To a suspension of 3,5-dichlorophenylisothiocyanate (5.0 g, 24.5 mmol) in 35 ml of absolute ethanol was added 1-naphthylamine (3.51 g, 24.5 mmol) in one portion. The resulting dark red suspension was then stirred at room temperature overnight. After 16 h, the precipitate was isolated by filtration and washed with hexane to afford 8.14 g (96%) of an off-white solid: $^1H$ NMR (DMSO-D6) δ 7.30 (s, 1H), 7.48-7.60 (m, 4H), 7.64 (s, 2H), 7.87 (d, 1H, J = 7.6 Hz), 7.94 (t, 2H, J = 7.6 Hz), 9.95 (br s, 1H and 10.18 (br s, 1H).

N-(3,5-Dichlorophenyl)-N′-1-naphthyl-S-methylisothiourea:

A suspension of the thiourea prepared above (8.14 g, 23.4 mmol) in 80 ml of acetone was treated with 3.65 ml of methyl iodide (8.32 g, 58.6 mmol). The reaction mixture was then stirred at room temperature overnight. After 18 h, the yellow suspension was filtered and the precipitate was washed with ether. The solid was dissolved in 100 ml of 1 N aqueous sodium hydroxide and then the alkaline solution was extracted with two 100 ml portions of ether. The combined organic phases were dried over anhydrous sodium sulfate, filtered, and concentrated under reduced pressure to give 8.30 g (98%) of an off-white solid (mp 97-99 ° C): $^1H$ NMR ($CDCl_3$) δ 2.27 (s, 3H), 6.45 (br s, 1H), 7.04 (t, 1H, J = 1.8 Hz), 7.18-7.28 (m, 3H), 7.43 (t, 1H, J = 7.8 Hz), 7.48-7.52 (m, 2H), 7.66-7.69 (m, 1H), 7.83-7.87 (m, 1H) and 7.94-7.97 (m, 1H).

1′-N-[N′-1-Naphthylamino(3,5-dichlorophenylimino)methyl]-5-amino-methyltetrazole:

A mixture of 5-aminomethyltetrazole (0.50 g, 5.05 mmol), sodium hydroxide (0.20 g, 5.05 mmol), and 1.5 ml of water was added to a suspension of N-(3,5-dichlorophenyl)-N′-1-naphthyl-S-methylisothiourea (1.82 g, 5.05 mmol) in 25 ml of absolute ethanol. The reaction mixture was heated at reflux for 64 h, then cooled to room temperature and concentrated at reduced pressure. The residue was dissolved in 50 ml of water and then adjusted to pH 7.5 with 10% aqueous HCl. The solid that precipitated was isolated by filtration and washed with water and ether. This material was recrystallized from acetonitrile-water to give 0.94 g (45%) of an off-white solid (mp 175-178 ° C): IR (Nujol) 3400, 3200, 2925, 2856, 1654, 1587, 1448, 1328, 1240, 1108, 853 and 781 $cm^{-1}$; $^1H$ NMR (DMSO-D6) δ 4.73 (s, 2H), 7.04 (s, 1H), 7.16 (s, 2H), 7.20 (d, 1H, J = 8.1 Hz), 7.38 (t, 1H, J = 8.1 Hz), 7.47-7.50 (m, 2H), 7.63 (d, 1H, J = 8.1 Hz), 7.85-7.88 (m, 1H) and

7.96-8.00 (m, 1H); $^{13}C$ NMR ($MeOH-d_4/CDCl_3$) δ 38.2, 122.3, 123.1, 124.2, 125.9, 126.8, 127.4, 128.0, 129.0, 129.3, 131.4, 135.0, 135.9, 138.4, 155.9 and 158.8; Analysis calculated for $C_{19}H_{15}Cl_2N_7$ (0.73 $H_2O$): C, 53.64; H, 3.90; N, 23.05. Found: C, 53.62; H, 3.62; N, 23.05.

EXAMPLE 25

Synthesis of 1′-N-[N′-Methylamino (4-cyanophenylimino)methyl]-5-aminomethyltetrazole:

N-(4-Cyanophenyl)-N′-methylthiourea:

A suspension of 4-cyanophenylisothiocyanate (10.0 g, 62.4 mmol) in 70 ml of absolute ethanol was treated dropwise with 6.5 ml of aqueous methylamine (40 wt. % soln, 74.9 mmol). The reaction was exothermic with immediate formation of a precipitate. The reaction mixture was stirred at room temperature for an additional 1.5 h, and then concentrated under reduced pressure. The residue was triturated with ether to provide 10.6 g (89%) of an off-white solid: $^1H$ NMR (DMSO-D6) δ 2.93 (s, 3H), 7.71 (s, 4H), 8.10 (br s, 1H) and 9.98 (br s, 1H).

N-(4-Cyanophenyl)-N′-methyl-S-methylisothiourea:

To a suspension of the thiourea prepared above (10.6 g, 55.4 mmol) in 180 ml of acetone was added 8.6 ml of methyl iodide (19.7 g, 139 mmol). The resulting light brown solution was then stirred at room temperature overnight. After ca. 16 h, the reaction mixture was concentrated under reduced pressure and the residue triturated with ether. The solid was filtered, dissolved in 200 ml of 1 N aqueous sodium hydroxide and then the alkaline solution was extracted with ether. The organic phase was dried over anhydrous sodium sulfate, filtered, and concentrated to afford 11.2 g (98%) of a pale yellow solid (mp 113-115 °C): $^1H$ NMR ($CDCl_3$) δ 2.23 (s, 3H), 2.93 (s, 3H), 4.65 (br s, 1H), 6.94 (d, 2H, J = 8.3 Hz), and 7.51 (d, 2H, J = 8.3 Hz).

1′-N-[N-Methylamino(4-cyanophenylimino) methyl]-5-aminomethyltetrazole:

A mixture of 5-aminomethyltetrazole (0.50 g, 5.05 mmol), sodium hydroxide (0.20 g, 5.05 mmol), and 1.5 ml of water was added to a suspension of N-(4-cyanophenyl)-N′-methyl-S-methylisothiourea (1.04 g, 5.05 mmol) in 25 ml of absolute ethanol. The reaction mixture was heated at reflux for 64 h, then cooled to room temperature and concentrated at reduced pressure. The residue was suspended in 50 ml of water and then adjusted to pH 7.5 with 10% aqueous HCl. The precipitate was filtered, washed with water and ether, and then dried in vacuo to give 1.24 g (96%) of a white solid (d > 245 °C): IR (Nujol) 3586, 3400, 3200, 2925, 2854, 2223, 1670, 1606, 1568, 1504, 1338, 1261, 1180, 1099 and 813 $cm^{-1}$; $^1H$ NMR (DMSO-D6) δ 2.87 (s, 3H), 4.58 (s, 2H), 7.41 (d, 2H, J = 8.5 Hz) and 7.86 (d, 2H, J = 8.5 Hz); $^{13}C$ NMR (DMSO-D6) δ 29.0, 37.6, 106.3, 118.9, 122.5, 133.7, 142.7, 154.9 and 158.0.

EXAMPLE 26

Synthesis of 1′-N-[N-Hexadecylamino (4-cyanophenylimino)methyl]-5-aminomethyltetrazole:

N-(4-Cyanophenyl)-N′-hexadecylthiourea:

To a suspension of 4-cyanophenylisothiocyanate (10.0 g, 62.4 mmol) in 120 ml of absolute ethanol was added hexadecylamine (15.1 g, 62.4 mmol) in one portion. The resulting thick white suspension could not be easily stirred, and was subsequently diluted with 200 ml each of ethyl acetate and acetonitrile. A mechanical stirred was inserted and then the reaction mixture was stirred thoroughly at room temperature overnight. After ca. 16 h, the reaction mixture was concentrated under reduced pressure and the residue then triturated with hexane to give 23.7 g (94%) of a white solid: $^1H$ NMR (Acetone-D6) δ 0.87 (t, 3H, H = 7.2 Hz), 1.28 (br s, 26H), 1.60-1.68 (m, 2H), 3.58-3.64 (m, 2H), 7.62 (br s, 1H), 7.67 (d, 2H, J = 8.1 Hz), 7.83 (d, 2H, H = 8.1 Hz) and 9.20 (br s, 1H).

N-(4-Cyanophenyl)-N′-hexadecyl-S-methylisothiourea:

A suspension of the thiourea prepared above (23.7 g, 59.0 mmol) in 250 ml of acetone was treated with 9.2 ml of methyl iodide (20.9 g, 147 mmol). The reaction mixture was stirred at room temperature for 18 h and then concentrated under reduced pressure. The residue was dissolved in 1 N aqueous sodium hydroxide and then the alkaline solution was extracted with several portions of ethyl acetate. The combined organic phases were washed with saturated aqueous NaCl, dried over anhydrous magnesium sulfate, filtered, and concentrated to give 24.0 g (98%) of an off-white solid (mp 68-69° C): $^1H$ NMR ($CDCl_3$) δ 0.88 (t, 3H, J=7.2 Hz), 1.25 (br s, 26H), 1.52-1.65 (m, 2H), 2.25 (s, 3H), 3.32 (t, 2H, J=7.2 Hz), 4.55 (br s, 1H), 6.95 (d, 2H, J=8.1 Hz) and 7.54 (d, 2H, J=8.1 Hz).

1′-N-[N-Hexadecylamino(4-cyanophenylimino) methyl]-5-aminomethyltetrazole:

A mixture of 5-aminomethyltetrazole (0.50 g, 5.05 mmol), sodium hydroxide (0.20 g, 5.05 mml), and 2 ml of water was added to a suspension of N-(4-cyanophenyl)-N′-hexadecyl-S-methylisothiourea (2.10 g, 5.05 mmol) in 20 ml of absolute ethanol. The reaction mixture was heated at reflux for 68 h, then cooled to room temperature and concentrated at reduced pressure. Trituration of the residue with ether yielded a white solid. This material was suspended in 50 ml of water and then adjusted to pH 7.5 with 10% aqueous HCl. When the desired guanidine failed to precipitate, the aqueous solution was lyophilized. The residue was subsequently dissolved in absolute ethanol, filtered, and then concentrated to provide 1.73 g (73%) of a white solid (mp 156-160° C): IR (Nujol) 3414, 2919, 2853, 2227, 1675, 1604, 1571, 1504, 1305, 1274, 1085, 830 and 723 $cm^{-1}$; $^1H$ NMR ($MeOH-d_4/CDCl_3$) δ 0.88 (t, 3H, J=6.9 Hz), 1.25 (br s, 26H), 1.58-1.68 (m, 2H), 3.27 (t, 2H, J=7.2), 4.65 (s, 2H), 7.36 (d, 2H, J=8.6 Hz) and 7.70 (d, 2H, J=8.6 Hz); $^{13}C$ NMR ($MeOH-d_4/CDCl_3$) δ 14.2, 22.9, 27.0, 29.0, 29.4, 29.6, 29.7, 29.8, 29.90, 29.93, 32.2, 37.5, 43.5, 108.8, 118.5, 122.9, 134.1, 141.6, 155.1 and 158.6. An analytical sample was recrystallized from ethanol-water. Analysis calculated for $C_{26}H_{42}N_8$ (0.10 $H_2O$): C, 66.66; H, 9.08, N, 23.92. Found: C, 66.65; H, 9.27; N, 23.44.

EXAMPLE 27

Synthesis of 1′-N-[N-(Cyclohexane-n-Butylamino) (4-Cyanophenylimino)methyl]-5-Aminomethyltetrazole:

Step 1: Preparation of N-n-Butylcyclohexyl-N′-4-Cyanophenyl Thiourea:

4-Cyanophenylisothiocyanate (2.22 g; 13.9 mmol) was dissolved in 30 ml ethyl acetate. Cyclohexane n-butylamine (2.15 g, 13.9 mmol) was added to the reaction mixture with stirring at room temperature. After stirring overnight, the reaction mixture was concentrated under reduced pressure. The residue was triturated with ether and filtered to give 3.5 g (80%) of white solid (mp=117-118° C). PMR ($CDCl_3$) δ 8.91 (br s, 1H), 7.64-7.44 (AB q, J=8, Hz, 4H), 6.58 (br s, 1H), 3.60 (m, 2H), 1.71-1.52 (m, 7H), 1.40-1.08 (m, 8H) and 0.90-0.77 (m, 2H). CMR ($CDCl_3$) δ 178.8, 142.1, 133.4, 123.0, 118.4, 108.1, 45.4, 33.2, 28.9, 26.5 and 24.1.

Step 2: Synthesis of N-n-Butylcyclohexyl-N′-4-Cyanophenyl-S-Methylisothiourea:

A mixture of 3.0 g (9.52 mmol) of the compound obtained previously and 1.5 ml (d = 2.28; 24.1 mmol) of methyl iodide was stirred overnight in 30 ml of acetone. The reaction mixture was concentrated under reduced pressure and the residue was triturated with ether and filtered to give 2.8 g of yellow solid. The solid was dissolved in 50 ml of a 1 N solution of sodium hydroxide. The resulting alkaline mixture was extracted with ether. The ether layer was washed with saturated NaCl and dried with anhydrous magnesium sulfate. The ether layer was then concentrated under reduced pressure to give 2.8 g (90%) of an orange oil. PMR ($CDCl_3$) δ 7.56-6.93 (AB q, J=7 Hz, 4H), 4.62 (br s, 1H), 3.42 (t, J=6 Hz, 2H), 2.26 (s, 3H), 1.7-1.5 (m, 7H), 1.4-1.1 (m, 8H) and 0.92-0.77 (m, 2H). CMR ($CDCl_3$) δ 154.3, 133.1, 123.1, 119.8, 105.1, 43.4, 37.4, 37.0, 33.4, 29.7, 26.7, 26.4, 24.1 and 14.2.

Step 3: Preparation of 1′-N-[N-(Cyclohexane-n-Butyl-amino) (4-cyanophenylimino)methyl]-5-Aminomethyltetrazole:

A mixture of 5-aminomethyltetrazole (0.50 g, 5.05 mmol), sodium hydroxide (0.20 g, 5.00 mmol) and 1.5 ml of water was added to a solution of N-n-Butylcyclohexyl-N′-4-cyanophenyl-S-methylisothiourea (1.66 g, 5.04 mmol) in 15 ml of absolute ethanol. The mixture was heated to reflux for 20 hours. After cooling, the

solution was concentrated to dryness and the residue was dissolved in 20 ml of water. The resulting aqueous solution was washed with ether (2 x 20 ml) and then neutralized with a 1 N HCl solution to bring the pH to 7.5. the desired guanidine compound precipitated and was separated out by filtration to give 1.1 g (58%) of the title compound. PMR ($CD_3COOD$) δ 7.78-7.38 (AB q, J = 7 Hz, 4H), 5.02 (s, 2H), 3.34 (t, J = 6 Hz, 2H), 1.72-1.50 (m, 7H), 1.35-1.05 (m, 8H) and 0.92-0.76 (m, 2H). CMR ($CD_3COOD$) δ 156.2, 155.4, 141.0, 134.9, 125.3, 118.8, 110.7, 47.1, 38.5, 37.8, 34.2, 29.7, 27.5, 27.2 and 24.7. Analysis calculated for $C_{20}H_{28}N_8$ (0.1 $H_2O$): C, 62.84; H, 7.44; N, 29.31. Found: C, 62.71; H, 7.53; N, 29.17. The compound was tasted and was sweet at a concentration of 150 µg/ml.

EXAMPLE 28

Synthesis of 1′-N-[N′-1-Aminoindanyl (4-Cyanophenylimino)methyl]-5-Aminomethyltetrazole:

Step 1: Preparation of N-1-Indanyl-N′-4-Cyanophenyl Thiourea:

4-Cyanophenylisothiocyanate (6.0 g; 37.5 mmol) was dissolved in 100 ml ethyl acetate. 1-Aminoindane (5.0 g, 37.5 mmol) was added to the reaction mixture with stirring at room temperature. After 30 minutes, a precipitate formed. The reaction mixture was stirred overnight and filtered to give 7.6 g (69%) of white solid. The filtrate was concentrated under reduced pressure and the residue triturated with ether to give an additional 2.9 g (26%). The combined solid (mp = 175-176 ° C) was dried at room temperature under a vacuum. PMR (DMSO-D6) δ was dried at room temperature under a vacuum. PMR (DMSO-D6) δ 9.90 (br s, 1H), 8.52 (br s, 1H), 7.86-7.73 (AB q, J = 8 Hz, 4H), 7.46-7.38 (m, 1H), 7.32-7.18 (m, 3H), 5.89 (br s, 1H), 3.05-2.78 (m, 2H), 2.61-2.49 (m, 1H), and 2.00-1.84 (m, 1H). CMR (DMSO-D6) δ 179.9, 144.2, 143.1, 142.8, 132.6, 127.7, 126.3, 124.6, 124.0, 121.2, 119.0, 104.6, 58.6, 32.6 and 29.6.

Step 2: Synthesis of N-1-Indanyl-N′-4-Cyanophenyl-S-Methylisothiourea:

A mixture of 6.2 g (21.2 mmol) of the compound obtained previously and 3.3 ml (d = 2.28; 53.0 mmol) of methyl iodide was stirred overnight in 65 ml of acetone. In the morning, a solid was present and it was filtered to give a white solid. The solid produced was dissolved in 50 ml of a 1 N solution of sodium hydroxide. The resulting alkaline mixture was extracted with methylene chloride ($CH_2Cl_2$). The $CH_2Cl_2$ layer was washed with saturated NaCl and dried with anhydrous sodium sulfate. The $CH_2Cl_2$ layer was then concentrated under reduced pressure to give 5.7 g (88%) of a white solid (mp = 125-126 ° C). PMR ($CDCl_3$) δ 7.54-6.98 (AB q, J = 8 Hz, 4H), 7.40-7.38 (m, 1H), 7.28-7.20 (m, 3H), 5.48 (t, J = 7 Hz, 1H), 4.83 (br s, 1H), 3.06-2.80 (m, 2H), 2.72-2.60 (m, 1H), 2.27 (s, 3H) and 1.95-1.82 (m, 1H). CMR ($CDCl_3$) δ 154.0, 143.5, 143.0, 133.1, 128.2, 126.8, 125.0, 124.0, 123.0, 119.7, 105.2, 58.2, 34.2, 30.1 and 14.2.

Step 3: Preparation of 1′-N-[N′-1-Aminoindanyl-(4-cyanophenylimino)methyl]-5-Aminomethyltetrazole:

A mixture of 5-aminomethyltetrazole (0.50 g, 5.05 mmol), sodium hydroxide (0.20 g, 5.00 mmol) and 1.5 ml of water was added to a solution of N-1-indanyl-N′-4-cyanophenyl-S-methylisothiourea (1.55 g, 5.05 mmol) in 15 ml of absolute ethanol. The mixture was heated to reflux for 20 hours. After cooling, the solution was concentrated to dryness and the residue was suspended in 20 ml of water. The resulting aqueous suspension was washed with ether (2 x 20 ml) and then neutralized with a 1 N HCl solution to bring the pH to 7.5. The desired guanidine compound was collected by filtration to give 1.2 g (67%) of the title compound. PMR ($CD_3COOD$) δ 7.76-7.48 (AB q, J = 8 Hz, 4H), 7.24 (s, 4H), 5.39 (t, J = 5, Hz, 1H), 5.04 (m, 2H), 3.05-2.91 (m, 1H), 2.89-2.74 (m, 1H), 2.67-2.52 (m, 1H) and 2.11-1.96 (m, 1H). CMR ($CD_3COOD$) δ 156.4, 155.2, 144.4, 141.0, 134.9, 129.8, 127.9, 125.9, 125.4, 118.9, 110.6, 59.6, 33.8 and 30.8. Analysis calculated for $C_{19}H_{18}N_8$ (0.6 $H_2O$): C, 61.81; H, 5.24; N, 30.55. Found: C, 61.84; H, 4.85; N, 30.28. The compound was tasted and was sweet at a concentration of 10 µg/ml.

EXAMPLE 29

Synthesis of 1′-N-[N′-2-Aminoindanyl (4-Cyanophenylimino)methyl]-5-Aminomethyltetrazole:

Step 1: Preparation of N-[2-Indanyl]-N′-4-Cyanophenyl Thiourea:

4-Cyanophenylisothiocyanate (4.7 g; 29.4 mmol) was dissolved in 40 ml ethyl acetate. An ethyl acetate solution (60 ml) containing 2-aminoindane (neutralized from 5.0 g, 29.5 mmoles, of the hydrochloride) was added with stirring at room temperature. The reaction mixture was stirred overnight and concentrated under reduced pressure. The residue was triturated with ether and filtered to give 3.5 g (80%) of white solid (mp = 138-140 ° C). PMR (DMSO-D6) δ 9.82 (br s, 1H), 8.44 (d, J = 4 Hz, 1H), 7.84-7.72 (AB q, J = 7 Hz, 4H), 7.32-7.23 (m, 2H), 7.20-7.14 (m, 2H), 4.97 (m, 1H), 3.32 (dd, J = 16.7, 7.3 Hz, 2H) and 2.92 (dd, J = 16.7, 5.0 Hz, 2H). CMR (DMSO-D6) δ 179.6, 144.2, 140.8, 132.6, 126.4, 124.5, 120.9, 119.0, 104.8, 54.7 and 38.7.

Step 2: Synthesis of N-2-Indanyl-N′-4-Cyanophenyl-S-Methylisothiourea:

A mixture of 5.6 g (19.1 mmol) of the compound obtained previously and 3.0 ml (d = 2.28; 48.2 mmol) of methyl iodide was stirred overnight in 60 ml of acetone. In the morning, a solid was present and it was filtered to give a white solid. The solid produced was dissolved in 50 ml of a 1 N solution of sodium hydroxide. The resulting alkaline mixture was extracted with methylene chloride ($CH_2Cl_2$). The $CH_2Cl_2$ layer was washed with saturated NaCl and dried with anhydrous sodium sulfate. The $CH_2Cl_2$ layer was then concentrated under reduced pressure to give 5.3 g (90%) of a white solid (mp = 120-122 ° C). PMR ($CDCl_3$) δ 7.53-6.96 (AB q, J = 8, Hz, 4H), 7.28-7.15 (m, 4H), 4.85 (br s, 1H), 4.75-4.66 (m, 1H), 3.38 (dd, J = 15.7, 6.9 Hz, 2H), 2.90 (dd, J = 15.7, 5.0 Hz, 2H) and 2.19 (s, 3H). CMR ($CDCl_3$) δ 154.0, 153.3, 140.6, 133.0, 126.8, 124.8, 122.9, 119.7, 105.1, 54.0, 40.1 and 14.2.

Step 3: Preparation of 1′-N-[N′-2-Aminoindanyl-(4-cyanophenylimino)methyl]-5-Aminomethyltetrazole:

A mixture of 5-aminomethyltetrazole (0.50 g, 5.05 mmol), sodium hydroxide (0.20 g, 5.00 mmol) and 1.5 ml of water was added to a solution of N-2-indanyl-N′-4-cyanophenyl-S-methylisothiourea (1.55 g, 5.05 mmol) in 15 ml of absolute ethanol. The mixture was heated to reflux for 20 hours. After cooling, the solution was concentrated to dryness and the residue was suspended in 20 ml of water. The resulting aqueous suspension was washed with ether (2 x 20 ml) and then neutralized with a 1 N HCl solution to bring the pH to 7.5. The desired guanidine compound was collected by filtration to give 1.2 g (67%) of the title compound. PMR ($CD_3OD$) δ 7.49-6.96 (AB q, J = 8 Hz, 4H), 7.20-7.07 (m, 4H), 4.53 (s, 2H), 4.46 (quint, J = 6 Hz, 1H), 3.23 (dd, J = 16, 7.3 Hz, 2H) and 2.87 (dd, J = 16, 5.9 Hz, 2H). CMR ($CD_3OD$) δ 161.2, 157.0, 155.6, 142.2, 134.3, 127.6, 125.5, 124.9, 121.0, 103,5, 54.3, 40.8 and 38.5. Analysis calculated for $C_{19}H_{18}N_8$ (0.75 $H_2O$): C, 61.36; H, 5.28; N, 30.13. Found: C, 61.24; H, 4.89; N, 29.69. The compound was tasted and was sweet at a concentration of 10 µg/ml.

EXAMPLE 30

Synthesis of 1′-N-[N′-1-Aminotetralyl (4-Cyanophenylimino)methyl]-5-Aminomethyltetrazole:

Step 1: Preparation of N-1-Tetralyl-N′-4-Cyanophenyl Thiourea:

4-Cyanophenylisothiocyanate (5.44 g; 34.0 mmol) was dissolved in 100 ml ethyl acetate. 1-Aminotetraline (5.0 g, 34.0 mmol) was added to the reaction mixture with stirring at room temperature. After 45 minutes, a precipitate formed. The reaction mixture was stirred overnight and filtered to give 6.4 g (62%) of white solid. The filtrate was concentrated under reduced pressure and the residue triturated with ether to give an additional 3.1 g (30%). The combined solid (mp = 187-189 ° C) was dried at room temperature under a vacuum. PMR ($CDCl_3$/$CD_3OD$) δ 7.76-7.55 (AB q, J = 8.7 Hz, 4H), 7.88-7.82 (m, 1H), 7.21-7.07 (m, 3H), 5.75 (m, 1H), 2.90-2.70 (m, 2H), 2.22-2.08 (m, 1H) and 1.95-1.80 (m, 3H). CMR ($CDCl_3$/$CD_3OD$) δ 179.9, 143.7, 137.6, 136.1, 133.0, 129.3, 128.6, 127.5, 126.3, 122.1, 119.1, 106.6, 52.3, 29.6, 29.3 and 20.4.

Step 2: Synthesis of N-1-Tetralyl-N′-4-Cyanophenyl-S-Methylisothiourea:

A mixture of 4.6 g (15.0 mmol) of the compound obtained previously and 2.33 ml (d = 2.28; 37.4 mmol) of methyl iodide was stirred overnight in 65 ml of acetone. In the morning, a solid was present and it was filtered to give 6.35 g (96%) of a white solid. The solid produced was dissolved in 50 ml of a 1 N solution of sodium hydroxide. The resulting alkaline mixture was extracted with 65 ml of methylene chloride ($CH_2Cl_2$). The $CH_2Cl_2$ layer was washed with saturated NaCl and dried with anhydrous sodium sulfate. The $CH_2Cl_2$ layer was then concentrated under reduced pressure to give 4.6 g (96%) of a yellow oil which solidified on standing (mp = 122-123 °C). PMR ($CDCl_3$) δ 7.53-6.98 (AB q, J = 8 Hz, 4H), 7.42-7.35 (m, 1H), 7.23-7.16 (m, 2H), 7.14-7.06 (m, 1H), 5.20 (m, 1H), 4.85 (br s, 1H), 2.90-2.68 (m, 2H), 2.24 (s, 3H) and 2.17-1.78 (m, 4H). CMR ($CDCl_3$) δ 154.0, 137.7, 136.5, 133.1, 129.3, 128.6, 127.5, 126.3, 123.0, 119.7, 105.1, 50.9, 29.8, 29.2, 19.9 and 14.2.

Step 3: Preparation of 1′-N-[1-Aminotetralyl-(4-cyanophenylimino)methyl]-5-Aminomethyltetrazole:

A mixture of 5-aminomethyltetrazole (0.50 g, 5.05 mmol), sodium hydroxide (0.20 g, 5.00 mmol) and 1.5 ml of water was added to a solution of N-1-tetralyl-N′-4-cyanophenyl-S-methylisothiourea (1.62 g, 5.05 mmol) in 15 ml of absolute ethanol. The mixture was heated to reflux for 20 hours. After cooling, the solution was concentrated to dryness and the residue was dissolved in 20 ml of water. The resulting aqueous solution was washed with ether (2 x 20 ml) and then neutralized with a 1 N HCl solution to bring the pH to 7.5. The desired guanidine compound was collected by filtration to give 1.7 g (89%) of the title compound. PMR ($CD_3COOD$) δ 7.74-7.08 (AB q, J = 8 Hz, 4H), 7.50-7.48 (m, 2H), 7.18 (s, 4H), 5.08 (m, 3H), 2.86-2.62 (m, 2H) and 2.20-1.66 (m, 4H). CMR ($CD_3COOD$) δ 156.2, 154.8, 140.8, 138.6, 134.8, 134.2, 130.2, 129.4, 129.0, 127.3, 125.6, 118.8, 110.6, 105.9, 52.9, 30.6 and 29.4. Analysis calculated for $C_{20}H_{20}N_8$ (1.6 $H_2O$): C, 59.87; H, 5.83; N, 27.92. Found: C, 59.65; H, 5.50; N, 27.88. The compound was tasted and was sweet at a concentration of 10 μg/ml.

EXAMPLE 31

Synthesis of 1′-N-[N′-2-Aminodecalyl (4-Cyanophenylimino)methyl]-5-Aminomethyltetrazole:

Step 1: Preparation of 2-Decalone Oxime:

An ethanol solution (25 ml) containing 5.1 g (33.5 mmoles) of 2-decalone was treated with 2.33 g (33.5 mmoles) of hydroxylamine hydrochloride in 10 ml of water followed by 2.32 g (16.8 mmoles) of potassium carbonate in 15 ml water. The resulting solution was heated to reflux for 1 hour, cooled and concentrated under reduced pressure. The aqueous layer remaining was extracted with ether. The ether layer was dried with magnesium sulfate and concentrated under reduced pressure to give 5.5 g (98%) of a pale yellow liquid as a mixture of isomers. PMR ($CDCl_3$) δ 2.8-2.6 (m, 1H), 2.4-2.05 (m, 3H) and 1.95-1.2 (m, 10H). CMR ($CDCl_3$) δ 160.1, 37.4, 36.7, 35.6, 35.5, 29.9, 29.0, 28.7, 28.5, 28.4, 28.3, 28.1, 27.1, 24.3, 24.2, 24.1, 22.9 and 22.8.

Step 2: Preparation of 2-Aminodecalin:

A solution of the oxime obtained above (3.82 g, 100.5 mmoles) in 50 ml THF was added to a suspension containing 5.6 g (33.5 mmoles) of lithium aluminum hydride in 100 ml of THF. The reaction was heated to reflux overnight. After cooling, 6 N KOH was added cautiously with stirring until a white solid formed. This solid was filtered and washed well with ether. The combined filtrates were dried with magnesium sulfate and concentrated under reduced pressure to give 3.9 g (76%) of 2-aminodecalin as a yellow liquid. PMR ($CD_3OD$) δ 2.65-2.54 (m, 1H) and 1.9-1.1 (m, 16H). CMR ($CD_3OD$) δ 52.2, 36.8, 36.4, 36.3, 33.1, 32.1, 31.2, 28.0, 26.7 and 21.8.

Step 3: Preparation of N-2-Decalyl-N′-4-Cyanophenyl Thiourea:

4-Cyanophenylisothiocyanate (4.1 g; 25.6 mmol) was dissolved in 50 ml ethyl acetate. 2-Aminodecalin (3.9 g, 25.5 mmol) was added to the reaction mixture with stirring at room temperature. The reaction mixture was stirred overnight and concentrated under reduced pressure. The residue was triturated with ether and filtered to give 4.7 g (59%) of white solid. PMR ($CDCl_3$) δ 8.94 (br 2, 1H), 7.64-7.44 (AB q, J = 8

Hz, 4H), 6.48 (br s, 1H), 4.22 (m, 1H) and 1.90-1.10 (m, 16H). CMR ($CDCl_3$) δ 178.4, 141.9, 133.6, 123.0, 118.6, 107.9, 55.1, 34.8, 34.6, 31.8, 31.6, 30.6, 27.1, 26.7, 25.5 and 20.8.

Step 4: Synthesis of N-2-Decalyl-N′-4-Cyanophenyl-S-Methylisothiourea:

A mixture of 4.7 g (15.0 mmol) of the compound obtained previously and 2.33 ml (d = 2.28; 37.4 mmol) of methyl iodide was stirred overnight in 65 ml of acetone. In the morning, a solid was present and it was filtered to give 3.1 g (45%) of a white solid. The filtrate was concentrated under reduced pressure to a brown oil which was triturated with ether to give an additional 3.2 g (47%) of off-white solid. The combined solid was dissolved in 50 ml of a 1 N solution of sodium hydroxide. The resulting alkaline mixture was extracted with 65 ml of methylene chloride ($CH_2Cl_2$). The $CH_2Cl_2$ layer was washed with saturated NaCl and dried with anhydrous sodium sulfate. The $CH_2Cl_2$ layer was then concentrated under reduced pressure to give 4.5 g (92%) of an off-white solid foam (mp = 117-119 ° C). PMR ($CDCl_3$), δ 7.54-6.94 (AB q, J = 8 Hz, 4H), 4.51 (br s, 1H), 3.73 (m, 1H), 2.25 (s, 3H) and 1.90-1.15 (m, 16H). CMR ($CDCl_3$) δ 154.3, 133.1, 123.0, 119.8, 104.9, 52.9, 35.0, 34.8, 32.7, 31.7, 30.7, 27.9, 26.7, 25.6, 20.8 and 14.2.

Step 5: Preparation of 1′-N-[2-Aminodecalyl-(4-cyanophenylimino)methyl]-5-Aminoethyltetrazole:

A mixture of 5-aminomethyltetrazole (0.70 g, 7.07 mmol), sodium hydroxide (0.28 g, 7.00 mmol) and 2.0 ml of water was added to a solution of N-2-decalin-N′-4-cyanophenyl-S-methylisothiourea (2.0 g, 6.12 mmol) in 20 ml of absolute ethanol. The mixture was heated to reflux for 20 hours. After cooling, the solution was concentrated to dryness and the residue was dissolved in 20 ml of water. The resulting aqueous solution was washed with ether (2 x 20 ml) and then neutralized with a 1 N HCl solution to bring the pH to 7.5. The desired guanidine compound was collected by filtration to give 1.8 g (78%) of the title compound. PMR ($CD_3OD$) δ 7.76-7.44 (apparent AB q, J = 8 Hz, 4H), 4.74 (s, 2H), 3.63 (m, 1H) and 1.88-1.16 (m, 16H). CMR ($CD_3OD$) δ 155.2, 142.8, 124.2, 119.4, 109.6, 54.6, 38.7, 36.2, 36.0, 32.8, 31.6, 28.0, 27.8, 26.5 and 21.7. Analysis calculated for $C_{20}H_{26}N_8$: C, 63.47; H, 6.92; N, 29.61. Found: C, 63.05, H, 6.76; N, 29.80. The compound was tasted and was sweet at a concentration of 1 µg/ml.

EXAMPLE 32

Synthesis of 1′-N-[N′-3,4-(Methylenedioxy) aniline (4-Cyanophenylimino)methyl]-5-Aminomethyltetrazole:

Step 1: Preparation of N-3,4-Benzodioxolane-N′-4-Cyanophenyl Thiourea:

4-Cyanophenylisothiocyanate (5.44 g; 34.0 mmol) was dissolved in 100 ml ethyl acetate. 3,4-(Methylenedioxy) aniline (4.7 g, 34.3 mmol) was added to the reaction mixture with stirring at room temperature. After 5 minutes, a precipitate formed. The reaction mixture was stirred 5 hours and filtered to give 9.4 g (93%) of white solid (mp = 187-188 ° C). PMR (DMSO-D6) δ 10.08 (s, 1H), 10.02 (s, 1H), 7.77 (s, 4H), 7.11 (d, J = 1.8 Hz, 1H), 6.90 (d, J = 8.2 Hz, 1H), 6.82 (dd, J = 8.2, 1.8 Hz, 1H) and 6.04 (s, 2H). CMR (DMSO-D6) δ 179.5, 146.9, 144.7, 144.1, 132.7, 132.5, 122.3, 119.0, 117.6, 107.8, 106.3, 105.1 and 101.2.

Step 2: Synthesis of N-3,4-Benzodioxolane-N′-4-Cyanophenyl-S-Methylisothiourea:

A mixture of 9.0 g (30.3 mmol) of the compound obtained previously and 4.7 ml (d = 2.28; 75.5 mmol) of methyl iodide was stirred overnight in 130 ml of acetone. The reaction mixture was concentrated under reduced pressure to give a brown oil. The oil was dissolved in 50 ml of a 1 N solution of sodium hydroxide. The resulting alkaline mixture was extracted with methylene chloride. the methylene chloride layer was washed with saturated NaCl and dried with anhydrous magnesium sulfate. The organic layer was then concentrated under reduced pressure to give 10.4 g of brown oil. This compound was crystallized from 35% ethyl acetate/hexane to give 5.0 g (53%) of a yellow solid (mp = 79-84 ° C (dec)). PMR ($CDCl_3$) δ 7.56-7.18 (apparent AB q, J = 8 Hz, 4H), 6.87 (br s, 1H), 6.73 (d, J = 8 Hz, 1H), 6.65 (br d, J = 8 Hz, 1H), 5.94 (s, 2H) and 2.29 (s, 3H). CMR ($CDCl_3$) δ 147.8, 144.9, 133.2, 121.7, 119.5, 115.7, 108.1, 105.4, 104.7, 101.3 and 14.8.

Step 3: Preparation of 1′-N-[3,4-(Methylenedioxy) aniline (4-Cyanophenylimino)methyl]-5-Aminomethyltetrazole:

A mixture of 5-aminomethyltetrazole (0.50 g, 5.05 mmol), sodium hydroxide (0.20 g, 5.00 mmol) and 1.5 ml of water was added to a solution of N-3,4-Benzodioxolane-N′-4-cyano-phenyl-S-methylisothiourea (1.57 g, 5.05 mmol) in 15 ml of absolute ethanol. The mixture was heated to reflux for 6 hours. After cooling, the solution was concentrated to dryness and the residue was dissolved in 20 ml of water. The resulting aqueous solution was washed with ether (2 x 20 ml) and then neutralized with a 1 N HCl solution to bring the pH to 7.5. The desired guanidine compound precipitated and was separated out by filtration to give 1.0 g (55%) of the title compound. PMR (DMSO-D6) δ 7.75-7.32 (AB q, J = 8.5 Hz, 4H), 6.93 (d, J = 1.8 Hz, 1H), 6.88 (d, J = 8.3 Hz, 1H), 6.68 (dd, J = 8.3, 1.8 Hz, 1H), 6.00 (s, 2H) and 4.67 (s, 2H). CMR (DMSO-D6) δ 157.0, 152.2, 147.5, 145.2, 144.6, 133.2, 131.9. 122.0, 119.0, 116.3, 108.3, 104.83, 104.78, 101.2 and 37.4. Analysis calculated for $C_{17}H_{14}N_8O_2$ (0.25 $H_2O$): C, 55.66; H, 3.98; N, 30.54. Found: C, 55.74; H, 4.09; N, 30.27. The compound was tasted and was sweet at a concentration of 100 µg/ml.

EXAMPLE 33

Synthesis of 1′-N-[N′-Tetrahydro-3-Thiophenamine-1,1-dioxide (4-Cyanophenylimino)methyl]-5-Aminomethyltetrazole:

Step 1: Preparation of N-3-Tetrahydrothiophen-1,1-dioxide-N′-4-Cyanophenyl Thiourea:

4-Cyanophenylisothiocyanate (5.92 g; 37.0 mmol) was dissolved in 100 ml ethyl acetate. Tetrahydro-3-thiophenamine 1,1-dioxide (5.0 g, 37.0 mmol) was added to the reaction mixture with stirring at room temperature. An immediate precipitate formed. The reaction mixture was stirred overnight and filtered to give 10.5 g (96%) of white solid. The solid (mp = 209-210 °C (dec.)) was dried at room temperature under a vacuum. PMR (DMSO-D6) δ 10.12 (br s, 1H), 8.49 (br d, J = 7 Hz, 1H), 7.76 (s, 4H), 5.04 (m, 1H), 3.60 (dd, J = 13.4, 7.4 Hz, 1H), 3.38-3.15 (m, 2H), 3.08 (dd, J = 13.4, 7.4 Hz, 1H), 2.60-2.48 (m, 1H) and 2.28-2.11 (m, 1H). CMR (DMSO-D6) δ 179.9, 143.6, 132.7, 121.6, 118.9, 105.1, 54.6, 50.3, 50.2 and 27.9.

Step 2: Synthesis of N-3-Tetrahydrothiophen-1,1-dioxide-N′-4-Cyanophenyl-S-Methylisothiourea:

A suspension of 7.0 g (23.7 mmol) of the compound obtained previously and 3.7 ml (d = 2.28; 59.4 mmol) of methyl iodide was stirred overnight in 100 ml of acetone. In the morning, the reaction was filtered to give 7.5 g (72%) of white solid. The solid produced was dissolved in 50 ml of a 1 N solution of sodium hydroxide. The resulting alkaline mixture was extracted with methylene chloride ($CH_2Cl_2$). The sample did not totally dissolve and was filtered to remove the solid. The $CH_2Cl_2$ layer from the filtrate was washed with saturated NaCl and dried with anhydrous sodium sulfate. The $CH_2Cl_2$ layer was then concentrated under reduced pressure to give 2.5 g (34%) of a white solid (mp = 134-136 °C). PMR ($CDCl_3$) δ 7.57-6.95 (apparent AB q, J = 8, Hz, 4H), 5.43 (br s, 1H), 4.83 (m, 1H), 3.53 (dd, J = 13.6, 7.3 Hz, 1H), 3.38-3.27 (m, 1H), 3.21-3.10 (m, 2H), 2.68-2.54 (m, 1H), 2.49-2.36 (m, 1H) and 2.30 (s, 3H). CMR ($CDCl_3$) δ 153.1, 152.6, 133.1, 122.8, 119.6, 105.5, 55.7, 50.3, 49.0, 28.8 and 14.2.

Step 3: Preparation of 1′-N-[N-Tetrahydro-3-Thiophenamine-1,1-dioxide (4-cyanophenylimino)methyl]-5-Aminomethyl-tetrazole:

A mixture of 5-aminomethyltetrazole (0.50 g, 5.05 mmol), sodium hydroxide (0.20 g, 5.00 mmol) and 1.5 ml of water was added to a solution of N-3-Tetrahydrothiophen-1,1-dioxide-N′-4-cyanophenyl-S-methylisothiourea (1.56 g, 5.05 mmol) in 15 ml of absolute ethanol. The mixture was heated to reflux for 20 hours. After cooling, the solution was concentrated to dryness and the residue was suspended in 20 ml of water. The resulting aqueous suspension was washed with ether (2 x 20 ml) and then neutralized with a 1 N HCl solution to bring the pH to 7.5. The desired guanidine compound was collected by filtration to give 1.5 g (83%) of the title compound. PMR (DMSO-D6) δ 7.82-7.33 (AB q, J = 8 Hz, 4H), 4.64 (s, 2H), 4.55 (m, 1H), 3.59 (dd, J = 13.3, 7.6 Hz, 1H), 3.40-3.28 (m, 1H), 3.18-3.04 (m, 2H), 2.58-2.44 (m, 1H) and 2.23-2.07 (m, 1H). CMR (DMSO-D6) δ 157.3, 153.0, 145.0, 133.5, 122.6, 118.9, 105.3, 54.6, 50.6, 48.8, 37.4 and 28.6. Analysis calculated for $C_{14}H_{16}N_8O_2S$ (1.2 $H_2O$): C, 44.02; H, 4.86; N, 29.33. Found: C, 44.00; H, 4.82; N, 29.26. The compound was tasted and was sweet at a concentration of 100 µg/ml.

EXAMPLE 34

Synthesis of 1′-N-[N′-Cyclooctylamino (6-Indazolylimino)methyl]-5-Aminomethyltetrazole:

Step 1: Preparation of N-Cyclooctyl-N′-6-Indazolyl Thiourea:

Cyclooctylisothiocyanate (1.69 g; 10.0 mmol) was dissolved in 20 ml acetonitrile. 6-Aminoindazole (1.33 g, 10.0 mmol) was added to the reaction mixture with stirring at room temperature. The reaction was heated to reflux and stirred overnight whereupon a precipitate formed. The reaction was cooled and filtered to give 1.9 g (65%) of brown solid (mp = 183-185 °C). PMR ($CDCl_3/CD_3OD$) δ 8.03 (br s, 1H), 7.78 (d, J = 8.5 Hz, 1H), 7.54 (br s, 1H), 7.41 (s, 1H), 7.02 (d, J = 8.5 Hz, 1H), 4.51 (br s, 1H), 2.0-1.88 (m, 2H) and 1.70-1.45 (m, 12H).

Step 2: Synthesis of N-cyclooctyl-N′-6-Indazolyl-S-Methylisothiourea:

A mixture of 1.8 g (5.96 mmol) of the compound obtained previously and 1.0 ml (d = 2.28; 16.1 mmol) of methyl iodide was stirred overnight in 60 ml of acetone. In the morning, a solid was present and it was filtered to give 2.5 g (95%) of a grey solid. The solid produced was dissolved in 15 ml of a 1 N solution of sodium hydroxide. The resulting alkaline mixture was extracted with methylene chloride ($CH_2Cl_2$). The $CH_2Cl_2$ layer was washed with saturated NaCl and dried with anhydrous sodium sulfate. The $CH_2Cl_2$ layer was then concentrated under reduced pressure to give 1.7 g (89%) of an off-white solid (mp = 131-134 °C). PMR ($CDCl_3$) δ 8.00 (s, 1H), 7.63 (d, J = 9 Hz, 1H), 6.92 (s, 1H), 6.78 (dd, J = 9 Hz, 2 Hz, 1H), 3.92 (m, 1H), 2.78 (s, 3H), 1.96-1.80 (m, 2H) and 1.70-1.40 (m, 12H). CMR ($CDCl_3$) δ 149.0, 141.5, 134.4, 121.1, 119.5, 118.2, 101.4, 52.8, 32.6, 27.1, 25.5, 23.6 and 14.1.

Step 3: Preparation of 1′-N-[N′-Cyclooctyl (6-indazolylimino)methyl]-5-Aminomethyltetrazole:

A mixture of 5-aminomethyltetrazole (0.50 g, 5.05 mmol), sodium hydroxide (0.20 g, 5.00 mmol) and 1.5 ml of water was added to a solution of N-cyclooctyl-N′-6-indazolyl-S-methylisothiourea (1.55 g, 5.05 mmol) in 15 ml of absolute ethanol. The mixture was heated to reflux for 20 hours. After cooling, the solution was concentrated to dryness and the residue was suspended in 20 ml of water. The resulting aqueous suspension was washed with ether (2 x 20 ml) and then neutralized with a 1 N HCl solution to bring the pH to 7.5. The desired guanidine compound was collected by filtration to give 1.2 g (67%) of the title compound. PMR (DMSO-D6) δ 8.10 (s, 1H), 7.81 (d, J = 9 Hz, 1H), 7.43 (s, 1H), 7.02 (d, J = 9 Hz, 1H), 4.56 (s, 2H), 3.87 (m, 1H) and 1.94-1.25 (m, 14H). CMR (DMSO-D6) δ 158.3, 153.9, 140.3, 134.7, 133.4, 121.5, 120.8, 117.3, 52.5, 37.4, 31.2, 26.6, 24.5 and 22.8. Analysis calculated for $C_{18}H_{25}N_9$ (0.5 $H_2O$): C, 57.43; H, 6.96; N, 33.48. Found: C, 57.46; H, 6.93; N, 32.53. The compound was tasted and was sweet at a concentration of 10 μg/ml.

EXAMPLE 35

Synthesis of 1′-N-[N′-4-Aminotetrahydropyran (4-Cyanophenylimino)methyl]-5-Aminomethyltetrazole:

Step 1: Preparation of Tetrahydro-4H-pyran-4-oxime:

An ethanol solution (40 ml) containing 5.0 g (50.0 mmoles) of tetrahydro-4H-pyran-4-one was treated with 3.48 g (50.1 mmoles) of hydroxylamine hydrochloride in 17 ml of water followed by 3.45 g (25.0 mmoles) of potassium carbonate in 23 ml water. The resulting solution was heated to reflux for 1 hour, cooled and concentrated under reduced pressure. The aqueous layer remaining was extracted with ether. The ether layer was dried with magnesium sulfate and concentrated under reduced pressure to give 4.2 g (73%) of a white solid (mp = 87-88 °C). PMR ($CDCl_3$) δ 9.68 (br s, 1H), 3.82 (t, J = 5.6 Hz, 2H), 3.77 (t, J = 5.9 Hz, 2H), 2.68 (t, J = 5.9 Hz, 2H) and 2.39 (t, J = 5.6 Hz, 2H). CMR ($CDCl_3$) δ 155.9, 68.2, 66.7, 32.2 and 26.0.

Step 2: Preparation of 4-Aminotetrahydropyran:

A solution of the oxime obtained above (4.0 g, 34.8 mmoles) in 50 ml THF was added to a suspension containing 4.0 g (105 mmoles) of lithium aluminum hydride in 100 ml of THF. The reaction was heated to

reflux overnight. After cooling, 6 N KOH was added cautiously with stirring until a white solid formed. This solid was filtered and washed well with ether. The combined filtrates were dried with magnesium sulfate and concentrated under reduced pressure to give 2.6 g (74%) of 4-aminotetrahydropyran as a colorless liquid. PMR ($CDCl_3$) δ 4.00-3.90 (m, 2H), 3.38 (dt, J = 11.7, 2.1 Hz, 2H), 2.85 (m, 1H), 1.84-1.72 (m, 2H), 1.58 (br s, 2H) and 1.45-1.30 (m, 2H). CMR ($CDCl_3$) δ 66.8, 47.7 and 36.7.

Step 3: Preparation of N-4-Tetrahydropyran-N′-4-Cyanophenyl Thiourea:

4-Cyanophenylisothiocyanate (1.9 g; 11.9 mmol) was dissolved in 35 ml ethyl acetate. 4-Aminotetrahydropyran (1.2 g, 11.9 mmol) was added to the reaction mixture with stirring at room temperature. After 30 minutes, a precipitate formed. The reaction mixture was stirred overnight and filtered to give 1.1 g (35%) of white solid. The filtrate was concentrated under reduced pressure and the residue triturated with ether to give an additional 1.0 g (32%). The combined solid was dried at room temperature under a vacuum. PMR (DMSO-D6) δ 9.82 (br s, 1H), 8.17 (br d, J = 7 Hz, 1H), 7.79-7.72 (AB q, J = 9 Hz, 4H). 4.32 (m, 1H), 3.91-3.81 (m, 2H), 3.45-3.34 (m, 2H), 1.96-1.87 (m, 2H) and 1.50 (dq, J = 11.4, 4.3 Hz, 2H). CMR (DMSO-D6) δ 178.9, 144.1, 132.6, 121.0, 119.0, 104.5, 65.8, 49.7 and 31.7.

Step 4: Synthesis of N-4-Tetrahydropyran-N′-4-Cyanophenyl-S-Methylisothiourea:

A mixture of 2.0 g (7.66 mmol) of the compound obtained previously and 1.19 ml (d = 2.28; 19.1 mmol) of methyl iodide was stirred overnight in 35 ml of acetone. The reaction mixture was concentrated under reduced pressure and the residue was dissolved in 15 ml of a 1 N solution of sodium hydroxide. The resulting alkaline mixture was extracted with methylene chloride. The methylene chloride layer was washed with saturated NaCl and dried with anhydrous magnesium sulfate and then concentrated under reduced pressure to give 1.9 g (90%) of an off-white solid (mp = 131-133 °C). PMR ($CDCl_3$) δ 7.55-6.94 (AB q, J = 8 Hz, 4H), 4.62 (br s, 1H), 4.08-3.92 (m, 3H), 3.48 (dt, J = 11.6, 1.6 Hz, 2H), 2.24 (s, 3H), 2.10-2.00 (m, 2H) and 1.52 (dq, J = 11.5, 4.3 Hz, 2H). CMR ($CDCl_3$) δ 154.0, 152.6, 133.1, 122.9, 119.7, 105.1, 66.7, 49.1, 33.1 and 14.3.

Step 5: Preparation of 1′-N-[N-4-Aminotetrahydropyran) (4-cyanophenylimino)methyl]-5-Aminomethyltetrazole:

A mixture of 5-aminomethyltetrazole (0.50 g, 5.05 mmol), sodium hydroxide (0.20 g, 5.00 mmol) and 1.5 ml of water was added to a solution of N-4-tetrahydropyran-N′-4-cyanophenyl-S-methylisothiourea (1.39 g, 5.05 mmol) in 15 ml of absolute ethanol. The mixture was heated to reflux for 20 hours. After cooling, the solution was concentrated to dryness and the residue was dissolved in 20 ml of water. The resulting aqueous solution was washed with ether (2 x 20 ml) and then neutralized with a 1 N HCl solution to bring the pH to 7.5. The desired guanidine compound precipitated and was separated out by filtration to give 1.0 g (61%) of the title compound. PMR (DMSO-D6) 7.86-7.43 (AB Q, J = 7.8 Hz, 4H), 4.63 (s, 2H), 3.95-3.75 (m, 3H), 3.29 (t, J = 11 Hz, 2H), 1.94-1.72 (m, 2H) and 1.65-1.40 (m, 2H). CMR (DMSO-D6) δ 163.1, 158.4, 148.0, 138.7, 127.2, 123.8, 111.0, 70.6, 53.8, 42.7 and 37.0. Analysis calculated for $C_{15}H_{18}N_8O \cdot 1.5\ H_2O$: C, 51.24; H, 5.96; N, 31.87. Found: C, 51.33; H, 5.83; N, 31.90. The compound was tasted and was sweet at a concentration of 100 µg/ml.

## EXAMPLE 36

Synthesis of 1′-N-[N′-(S-α-phenylpropyl) (4-Cyanophenylimino)methyl]5-Aminomethyltetrazole:

Step 1: Preparation of N-(S-α-phenylpropyl)-N′-4-Cyanophenyl Thiourea:

A mixture of 4-cyanophenyl isothiocyanate (3.60 g, 22.39 mmol) and S-α-phenylpropylamine (3.0 g, 22.39 mmol) in acetonitrile (50 ml) was heated at reflux for 16 hours. The reaction mixture was concentrated to afford a white solid. The solid was recrystallized from hexane-ethyl acetate to afford 6.6 g (quantitative) of the desired product as a white powder. $^1H$ NMR ($CDCl_3$) δ 0.93 (t, 3H, J = 7.4 Hz), 1.84-2.0 (m, 2H), 7.26-7.42 (m, 7H), 7.61 (d, 2H, J = 8.3 Hz). $^{13}C$ NMR ($CDCl_3$) δ 10.7, 29.4, 60.6, 108.5, 118.5, 123.0, 126.7, 128.2, 129.1, 133.4, 133.5, 179.4. Analysis calculated for $C_{17}H_{17}N_3S$: C, 69.12; H, 5.80; N, 14.22. Found: C, 68.92; H, 5.64; N, 14.18.

Step 2: Preparation of N-(S-α-phenylpropyl)-N′-(4-Cyanophenyl)-S-Methylisothiourea:

Iodomethane (9.10 g, 66.33 mmol) was added to a solution of the thiourea (6.50 g, 22.11 mmol) in acetone (100 ml) and the reaction mixture was stirred at ambient temperature for 18 hours. The reaction mixture was concentrated and the residue was partitioned between dichloromethane (500 ml) and sodium hydroxide (1 N, 200 ml). The organic layer was separated, dried ($MgSO_4$) and concentrated to afford 6.5 g (94%) of the desired product as an oil. $^1H$ NMR ($CDCl_3$) δ 0.90 (t, 3H, J=9 Hz), 1.84 (m, 2H), 2.23 (s, 3H), 4.81 (t, 1H, J=7.5 Hz), 6.88 (d, 2H, J=9 Hz), 7.24-7.38 (m, 5H), 7.52 (d, 2H, J=9 Hz). $^{13}C$ NMR ($CDCl_3$) δ 10.8, 14.4, 29.8, 58.5, 105.1, 119.8, 122.9, 126.6, 127.4, 128.6, 133.2, 142.2, 154.1. Analysis calculated for $C_{18}H_{19}N_3S$: C, 69.87; H, 6.19; N, 13.58. Found: C, 69.40; H, 6.20; N, 13.44.

Step 3: Preparation of 1′-N-[N′-S-α-phenylpropyl) (4-cyanophenylimino)methyl] 5-Aminomethyltetrazole:

A solution of Aminomethyl tetrazole (3.04 g, 30,74 mmol) and sodium hydroxide (1.23 g, 30.74 mmol) in water (25 ml) was added to a solution of the isothiourea (3.80 g, 12.30 mmol) in ethanol (250 ml) and the mixture was heated at reflux for 4 hours. The reaction mixture was concentrated in vacuo. The residue was partitioned between dichloromethane (200 ml) and sodium hydroxide (1 N, 300 ml). The aqueous layer was neutralized with conc. hydrochloric acid. Slow evaporation of the aqueous layer afforded a white precipitate which was filtered and dried to give 3.0 g (68%) of the desired material. $^1H$ NMR (DMSO-D6) δ 0.74 (t, 3H), 1.66-1.83 (m, 2H), 4.80 (m, 3H), 7.15-7.19 (m, 7H), 7.62 (d, 2H, J=8.5 Hz). $^{13}C$ NMR (DMSO-D6) δ 11.0, 29.4, 58.8, 107.3, 119.1, 122.9, 126.8, 128.0, 128.9, 133.9, 141.9, 154.5, 156.0.

EXAMPLE 37

Synthesis of 1′-N-[N′-(diphenylmethyl) (4-cyanophenylimino)methyl] 5-Aminomethyl tetrazole:

Step 1: Preparation of N-(diphenylmethyl)-N′-4-Cyanophenyl Thiourea:

A mixture of 4-cyanophenyl isothiocyanate (8.74 g, 54.62 mmol) and aminodiphenylmethane (10.0 g, 54.57 mmol) in acetonitrile (150 ml) was heated at reflux for 4 hours and the reaction mixture was concentrated. Upon addition of hexane-ethyl acetate (9:1) to the residue, a precipitate formed. The precipitate was filtered and recrystallized from hexane-ethyl acetate to afford 18.2 g (97%) of the desired product as a white powder. $^1H$ NMR ($CDCl_3$) δ 6.81 (d, 1H, J=6 Hz), 7.25-7.36 (m, 10H), 7.54 (d, 2H, J=8 Hz), 7.87 (d, 2H, J=8 Hz). $^{13}C$ NMR ($CDCl_3$) δ 60.8, 105.9, 119.1, 121.3, 127.3, 127.6, 128.4, 132.4, 141.3, 143.9, 179.9. Analysis calculated for $C_{21}H_{17}N_3S$: C, 73.44; H, 4.99; N, 12.24. Found: C, 73.13; H, 4.93; N, 12.24.

Step 2: Preparation of N-(diphenylmethyl)-N′-(4-Cyanophenyl)-S-methylisothiourea:

Iodomethane (20.0 g, 146.0 mmol) was added to a solution of the thiourea (17.0 g, 49.56 mmol) in acetone (150 ml) and the reaction mixture was stirred at ambient temperature for 18 hours. The reaction mixture was concentrated and the residue was partitioned between dichloromethane (500 ml) and sodium hydroxide (1 N, 200 ml). The organic layer was separated, dried ($MgSO_4$) and concentrated to afford 16.0 g (90%) of the desired product as a cyrstalline solid. $^1H$ NMR ($CDCl_3$) δ 2.27 (s, 3H), 6.21 (m, 1H), 6.86 (d, 3H, J=8 Hz), 7.25-7.37 (m, 10H), 7.49 (d, 2H, J=7.3 Hz). $^{13}C$ NMR ($CDCl_3$) δ 14.3, 60.5, 105.3, 119.7, 122.7, 127.3, 127.6, 128.7, 133.0, 141.4. Analysis calculated for $C_{22}H_{19}N_3S$: C, 73.92; H, 5.36; N, 11.76. Found: C, 73.51; H, 5.21; N, 11.64.

Step 3: Preparation of 1′-N-[N′-diphenylmethyl) (4-Cyanophenylimino)methyl] 5-Aminomethyl tetrazole:

A solution of Aminomethyl tetrazole (2.77 g, 28.02 mmol) and sodium hydroxide (1.12 g, 28.02 mmol) in water (30 ml) was added to a solutionof the isothiourea (5.0 g, 14.01 mmol) in ethanol (180 ml) and the mixture was heated at reflux for 6 hours. The reaction mixture was concentrated in vacuo. The residue was dissolved in water (200 ml) and sodium hydroxide (1 N, 25 ml). The aqueous solution was neutralized with concentrated hydrochloric acid to afford a precipitate. The precipitate was filtered and dried to give 5.0 g (88%) of the desired guanidine. Recrystallization of this product (methanol-water) afforded pure product. $^1H$ NMR (DMSO-D6) δ 4.75 (s, 2H), 6.27 (s, 1H), 7.24 (d, 2H, J=8.5 Hz), 7.29-7.36 (m, 10H), 7.72 (d, 2H, J=8.4 Hz). $^{13}C$ NMR (DMSO-$D_6$) δ 38.1, 60.4, 106.5, 119.3, 122.7, 127.4, 127.7, 128.1, 128.8, 129.0, 133.9,

140.9, 144.0, 154.4, 157.5.

EXAMPLE 38

Synthesis of 1′-N-[N′-(R,S-α-cyclohexylbenzyl) (4-Cyanophenylimino)methyl]-5-aminomethyltetrazole:

Step 1: Preparation of N′-(R,S-α-cyclohexylbenzyl)-N′-4-Cyanophenyl thiourea:

A mixture of 4-cyanophenyl isothiocyanate (3.80 g, 23.80 mmol) and α-cyclohexylbenzylamine (4.50 g, 23.80 mmol) in acetonitrile (100 ml) was heated at reflux for 4 h and the reaction mixture was concentrated. The residue was recrystallized from hexane-ethyl acetate to afford 7.50 g (90%) of the desired product as a gray-white powder. $^1$H NMR ($CDCl_3$) δ 0.91-1.88 (m, 10H), 7.23-7.36 (m, 7H), 7.57 (d, 2H, J = 7.5 Hz). $^{13}$C NMR ($CDCl_3$) δ 25.8, 25.9, 26.1, 29.8, 43.1, 64.4, 108.4, 118.5, 123.0, 123.1, 127.2, 128.9, 129.0, 133.4, 133.5, 133.47, 133.6, 179.7. Analysis calculated for $C_{21}H_{23}N_3S$: C, 72.17; HJ, 6.63; N, 12.02. Found: C, 72.06; H, 6.72; N, 11.99.

Step 2: Preparation of N-(R,S-α-cyclohexylbenzyl)-N′-(4-Cyanophenyl)-S-Methylisothiourea:

Iodomethane (6.48 g, 47.28 mmol) was added to a solution of the thiourea (5.50 g, 15.76 mmol) in acetone (100 ml) and the reaction mixture was stirred at ambient temperature for 18 hours. The reaction mixture was concentrated and the residue was partitioned between dichloromethane (250 ml) and sodium hydroxide (1 N, 150 ml). The organic layer was separated, dried ($MgSO_4$) and concentrated to afford 5.20 g (91%) of the desired product as a brown oil. $^1$H NMR ($CDCl_3$) δ 0.84-1.82 (m, 10H), 2.15 (s, 3H), 4.61 (d, 1H, J = 7.5 Hz), 6.77 (d, 2H, J = 8.2 Hz), 7.10-7.23 (m, 5H), 7.43 (d, 2H, J = 8.3 Hz). $^{13}$C NMR ($CDCl_3$) δ 14.3, 26.0, 26.1, 26.2, 29.5, 30.1, 43.5, 62.0, 105.0, 119.8, 122.8, 127.0, 127.2, 128.4, 133.1, 141.4. Analysis calculated for $C_{22}H_{25}N_3S$: C, 72.69; H, 6.93; N, 11.56. Found: C, 71.62; H, 6.72; N, 11.99.

Step 3: Preparation of 1′-N-[N′-(R,S-α-cyclohexylbenzyl)(4-Cyanophenylimino)methyl]5-Aminomethyl tetrazole:

A solution of Aminomethyl tetrazole (4.36 g, 44.04 mmol) and sodium hydroxide (1.76 g, 44.04 mmol) in water (50 ml) was added to a solution of the isothiourea (8.0 g, 22.04 mmol) in ethanol (400 ml) and the mixture was heated at reflux for 6 hours. The reaction mixture was concentrated in vacuo. The residue was partitioned between dichloromethane (300 ml) and sodium hydroxide (1 N, 300 ml). The aqueous layer was neutralized with conc. hydrochloric acid. The precipitate was filtered, dried and recrystallized from to afford 9.0 g (98%) of the desired product as a colorless crystalline solid. $^1$H NMR (DMSO-D6) δ 1.04-1.86 (m, 11H), 4.56 (m, 1H), 4.64 (s, 2H), 7.14 δ 7.74 (AB, 4H, J = 8.5 Hz), 7.23-7.35 (m, 5H). $^{13}$C NMR (DMSO-D6) δ 25.7, 26.0, 29.3, 29.6, 37.7, 42.6, 62.6, 107.2, 119.1, 122.7, 122.8, 127.4, 128.0, 128.1, 134.0, 139.9, 142.1, 154.8, 157.6.

EXAMPLE 39

Synthesis of 1′-N-[N′-(Furfurylamino) (4-Cyanophenylimino)methyl]-5-Aminomethyltetrazole:

Step 1: Preparation of N-Furfuryl-N′-4-Cyanophenyl Thiourea:

4-Cyanophenylisothiocyanate (5.0 g; 31.2 mmol) was dissolved in 100 ml ethyl acetate. Furfuryl amine (2.76 ml, 31.3 mmol) was added to the reaction mixture with stirring at room temperature. After stirring overnight, the reaction mixture was concentrated under reduced pressure. The residue was triturated with ether and filtered to give 5.9 g (74%) of white solid (mp = 159-160 °C). PMR (DMSO-D6) δ 10.01 (br s, 1H), 8.50 (br s, 1H), 7.82-7.73 (AB q, J = 8 Hz, 4H), 7.63 (s, 1H), 6.46-6.42 (m, 1H), 6.41-6.37 (m, 1H) and 4.75 (d, J = 4.4 Hz, 2H). CMR (DMSO-D6) δ 180.1, 150.9, 144.0, 142.3, 132.6, 121.4, 119.0, 110.5, 107.7, 104.8 and 40.4.

Step 2: Synthesis of N-Furfuryl-N′-4-Cyanophenyl-S-Methylisothiourea:

A mixture of 5.0 g (19.5 mmol) of the compound obtained previously and 3.04 ml (d = 2.28; 48.8 mmol) of methyl iodide was stirred overnight in 90 ml of acetone. The residue was dissolved in 40 ml of a 1

N solution of sodium hydroxide. The resulting alkaline mixture was extracted with ether. The ether layer was washed with saturated NaCl and dreid with anhydrous magnesium sulfate. The ether layer was then concentrated under reduced pressure to give 4.9 g (92%) of a yellow solid (mp = 106-108 °C). PMR ($CDCl_3$) δ 7.54-6.94 (AB q, J = 8 Hz, 4H), 7.38 (s, 1H), 6.38-6.32 (m, 1H), 6.31-6.27 (m, 1H), 5.00 (br s, 1H), 4.54 (s, 2H) and 2.26 (s, 3H). CMR ($CDCl_3$) δ 153.9, 153.3, 150.9, 142.3, 133.0, 123.0, 119.7, 110.5, 107.9, 105.3, 40.1 and 14.1.

Step 3: Preparation of 1′-N-[N-Furfurylamino) (4-cyanophenylimino)methyl]-5-Aminomethyltetrazole:

A mixture of 5-aminomethyltetrazole (0.50 g, 5.05 mmol), sodium hydroxide (0.20 g, 5.00 mmol) and 1.5 ml of water was added to a solution of N-furfuryl-N′-4-cyanophenyl-S-methylisothiourea (1.37 g, 5.05 mmol) in 15 ml of absolute ethanol. The mixture was heated to reflux for 20 hours. After cooling, the solution was concentrated to dryness and the residue was dissolved in 15 ml of water. The resulting aqueous solution was washed with ether (2 x 20 ml) and then neutralized with a 1 N HCl solution to bring the pH to 7.5. The desired guanidine compound precipitated and was separated out by filtration to give 1.0 g (62%) of the title compound. PMR (DMSO-D6) δ 7.94-7.35 (AB q, J = 8.4 Hz, 4H), 7.64 (s, 1H), 6.44 (s, 2H), 4.62 (s, 2H) and 4.55 (s, 2H). CMR (DMSO-D6) δ 157.6, 154.0, 149.7, 143.6, 142.8, 133.5, 122.4, 118.8, 110.6, 108.0, 105.9, 39.0 and 37.6. Analysis calculated for $C_{15}H_{14}N_8O$ ($H_2O$): C, 52.94; H, 4.74; N, 32.92. Found: C, 52.76; H, 4.59, N, 33.05. The compound was tasted and was sweet at a concentration of 100 μg/ml.

EXAMPLE 40

Synthesis of 1′-N-[N′-2-Thienyl Methylamino (4-Cyanophenylimino)methyl]-5-Aminomethyltetrazole:

Step 1: Preparation of N-2-Thienylmethyl-N′-4-Cyanophenyl Thiourea:

4-Cyanophenylisothiocyanate (5.0 g; 31.2 mmol) was dissolved in 100 ml ethyl acetate. 2-Thienyl methyl amine (3.2 ml, 31.2 mmol) was added to the reaction mixture with stirring at room temperature. After 2 minutes, a precipitate formed. The reaction mixture was stirred overnight and filtered to give 5.0 g (59%) of white solid. The filtrate was concentrated under reduced pressure and the residue triturated with ether to give an additional 2.2 g (26%). The combined solid (mp = 175-176 °C) was dried at rom temperature under a vacuum. PMR (DMSO-D6) δ 10.02 (br s, 1H), 8.62 (br s, 1H), 7.76 (s, 4H), 7.42 (dd, J = 5.1, 1.1 Hz, 1H), 7.12-7.08 (m, 1H), 6.99 (dd, J = 5.1, 3.5 Hz, 1H), and 4.93 (s, 2H). CMR (DMSO-D6) δ 179.9, 143.9, 140.7, 132.7, 126.5, 126.3, 125.4, 121.4, 119.0, 104.8 and 42.0.

Step 2: Synthesis of N-2-Thienylmethyl-N′-4-Cyanophenyl-S-Methylisothiourea:

A mixture of 6.0 g (22.0 mmol) of the compound obtained previously and 3.42 ml (d = 2.28; 54.9 mmol) of methyl iodide was stirred overnight in 100 ml of acetone. The reaction mixture was concentrated under reduced pressure to give a yellow oil. The oil was dissolved in 50 ml of a 1 N solution of sodium hydroxide. The resulting alkaline mixture was extracted with methylene chloride. The methylene chloride layer was washed with saturated NaCl and dried with anhydrous magnesium sulfate. The organic layer was then concentrated under reduced pressure to give 5.9 g (94%) of pale yellow solid (mp = 120-121 °C). PMR ($CDCl_3$) δ 7.54-6.97 (partially obscured AB q, J = 8 Hz, 4H), 7.25 (dd, J = 5.0, 1.0 Hz, 1H), 7.05-6.94 (m, 2H) overlapping AB q, 5.02 (br s, 1H), 4.72 (s, 2H) and 2.25 (s, 3H). CMR ($CDCl_3$) δ 153.8, 153.1, 140.6, 133.0, 126.8, 126.2, 125.5, 123.0, 119.7, 105.3, 41.9 and 14.1.

Step 3: Preparation of 1′-N-[2-Thienyl Methylamino (4-Cyanophenylimino)methyl]-5-Aminomethyltetrazole:

A mixture of 5-aminomethyltetrazole (0.50 g, 5.05 mmol), sodium hydroxide (0.20 g, 5.00 mmol) and 1.5 ml of water was added to a solution of N-2-thienylmethyl-N′-4-cyanophenyl-S-methylisothiourea (1.45 g, 5.05 mmol) in 15 ml of absolute ethanol. The mixture was heated to reflux overnight. After cooling, the solution was concentrated to dryness and the residue was dissolved in 20 ml of water. The resulting aqueous solution was washed with ether (2 x 20 ml) and then neutralized with a 1 N HCl solution to bring the pH to 7.5. The desired guanidine compound precipitated and was separated out by filtration to give 0.70 g (41%) of the title compound. PMR (DMSO-D6) δ 7.83-7.33 (AB q, J = 8.5 Hz, 4H), 7.47 (d, J = 4.5 Hz, 1H), 7.00 (d, J = 3 Hz, 1H), 6.99 (dd, J = 4.5, 3 Hz, 1H), 4.72 (s, 2H) and 4.63 (s, 2H). CMR (DMSO-D6) δ 157.5, 153.6, 144.0, 139.4, 133.5, 126.8, 126.4, 125.9, 122.5, 118.8, 105.7, 40.5, and 37.5. Analysis calculated for

$C_{15}H_{14}N_8S$: C, 53.24; H, 4.17; N, 33.11; S, 9.48. Found: C, 53.03; H, 4.05; N, 33.40; S, 9.60. The compound was tasted and was sweet at a concentration of 100 µg/ml.

EXAMPLE 41

Synthesis of 1′-N-[(4-Cyanophenylimino) methyl]5-Aminomethyl tetrazole:

Preparation of N-(4-Cyanophenyl)thiourea:

To a stirred suspension of 4-cyanophenylisothiocyanate (51.0 g, 318 mmol) in 400 ml of acetone was added 42.5 ml of 15 N $NH_4OH$(aq) in 1 portion. After 16 hours, the resulting suspension was filtered and the solid washed with copious amounts of ether. The solid was air dried to afford 42.3 g (75%) of the desired thiourea. PMR (DMSO-D6) δ 10.07 (s, 1H), 7.74 (m, 6H). IR (KBr) $cm^{-1}$ 3380, 3290, 3180, 3040, 3000, 2040, 1640, 1600, 1580, 1520, 1500, 1480, 1410. Analysis calculated for $C_8H_7N_3S$: C, 54.22; H, 3.98; N, 23.71. Found: C, 54.12; H, 4.11; N, 23.80.

Preparation of N-(4-Cyanophenyl)aminoiminomethanesulfonic acid:

To a stirred ice bath cooled suspension of N-(4-cyanophenyl)thiourea (5.32 g, 30.0 mmol), NaCl (0.66 g, 11.4 mmol), and $Na_2MoO_4 \cdot 2H_2O$ (0.108 g, 0.45 mmol) in 15 ml of water was added 9.7 ml of 30% $H_2O_2$ - (94.5 mmol) at a rate such that the reaction temperature did not exceed 10° C. The reaction mixture was allowed to warm to 20° C for 30 minutes, then to 30° C until the exotherm subsided. The reaction mixture was colled to 10° C and filtered. The solid was washed with water and air-dried to afford 6.31 g (93%) of the desired product. PMR (DMSO-D6) δ 10.65 (bs, 2H), 9.85 (bs, 1H), 7.94 (d, 2H, J=8.5 Hz), 7.54 (d, 2H, J=8.5 Hz). CMR (DMSO-D6) δ 165.5, 138.9, 133.9, 126.1, 118.3, 110.3.

Preparation of 1′-N-[4-Cyanophenylimino)methyl]5-Aminomethyl tetrazole:

To a stirred solution of Aminomethyl tetrazole (0.220 g, 2.22 mmol) and potassium carbonate (0.220 g, 2.22 mmol) in 5 ml of water was added N-(4-cyanophenyl)aminoiminomethanesulfonic acid (0.500 g, 2.22 mmol) over 5 minutes as a solid. The resulting suspension was stirred overnight and filtered to yield 0.465 g (86%) of crude product. The crude product (200 mg) was sonicated in ethanol, filtered, and air-dried to afford 150 mg of the desired product. PMR (DMSO-D6) δ 4.60 (s, 2H), 7.42 (d, 2H, J=8.1 Hz), 7.84 (d, 2H, J=8.1 Hz). CMR (DMSO-D6) δ 157.2, 154.6, 142.0, 133.8, 122.6, 118.8, 106.6, 37.6.

EXAMPLE 42

Synthesis of 1′-N-[N′-2-Amino-6-oxo-cis-hydrindane (4-Cyanophenylimino)methyl]-5-Aminomethyltetrazole:

Step 1: Preparation of N-2-(6-oxo-cis-hydrindanyl)-N′-4-Cyanophenyl Thiourea:

4-Cyanophenylisothiocyanate (2.2 g; 13.7 mmol) was dissolved in 50 ml ethyl acetate. 2-Amino-6-oxo-cis-hydrindane (1.9 g, 13.5 mmol) was added to the reaction mixture with stirring at room temperature. After 5 minutes, a precipitate formed. The reaction mixture was stirred overnight and filtered to give 3.2 g (80%) of white solid. (mp=183-189° C). PMR (DMSO-D6) δ 9.81 (br s, 1H), 8.08 (m, 1H), 7.80-7.71 (AB q, J=8.8 Hz, 4H), 4.36-4.01 (m, 1H), 3.8-3.65 (m, 2H), 3.6-3.48 (m, 2H) and 2.5-1.2 (m, 8H). CMR (DMSO-D6) δ - (major isomer) 178.9, 144.2, 132.6, 120.9, 119.1, 104.3, 72.2, 69.0, 48.3, 36.8, 28.8 and 24.2. (minor isomer) 178.9, 144.2, 132.6, 120.9, 119.1, 104.3, 73.5, 67.7, 51.6, 37.8, 35.8, 32.3, 26.0 and 21.9.

Step 2: Synthesis of N-2-(6-Oxo-cis-hydrindanyl)-N′-4-Cyanophenyl-S-Methylisothiourea:

A mixture of 3.0 g (9.97 mmol) of the compound obtained previously and 1.55 ml (d = 2.28; 24.9 mmol) of methyl iodide was stirred overnight in 50 ml of acetone. In the morning, a solid was present and it was filtered to give a white solid. The solid produced was dissolved in 20 ml of a 1 N solution of sodium hydroxide. The resulting alkaline mixture was extracted with methylene chloride ($CH_2Cl_2$). The $CH_2Cl_2$ layer was washed with saturated NaCl and dried with anhydrous sodium sulfate. The $CH_2Cl_2$ layer was then concentrated under reduced pressure to give 2.7 g (87%) of a white solid (mp=130-138° C). PMR ($CDCl_3$) δ 7.54-6.93 (AB q, J=8.4 Hz, 4H), 4.59 (br s, 1H), 4.0-3.76 (m, 3H), 3.71-3.58 (m, 2H), 2.55-2.31 (m, 1H),

2.24 (s, 3H), 2.18-1.90 (m, 3H) and 1.85-1.17 (m, 4H). CMR ($CDCl_3$) δ (major isomer) 154.2, 133.1, 122.9, 119.8, 104.9, 73.2, 69.8, 47.7, 37.6, 30.5, 30.3, 24.7 and 14.2. (minor isomer) 153.1, 133.1, 123.0, 119.8, 104.9, 74.4, 68.6, 50.8, 38.5, 36.3, 33.3, 27.4, 22.4 and 14.2.

Step 3: Preparation of 1′-N-[N-2-Amino-6-oxo-cis-hydrindane(4-cyanophenylimino)methyl]-5-Aminomethyl-tetrazole:

A mixture of 5-aminomethyltetrazole (0.50 g, 5.05 mmol), sodium hydroxide (0.20 g, 5.00 mmol) and 1.5 ml of water was added to a solution of N-2-(6-Oxo-cis-hydrindanyl)-N′-4-cyanophenyl-S-methyl-isothiourea (1.59 g, 5.05 mmol) in 15 ml of absolute ethanol. The mixture was heated to reflux for 20 hours. After cooling, the solution was concentrated to dryness and the residue was dissolved in 15 ml of water. The resulting aqueous solution was washed with ether (2 x 20 ml) and then neutralized with a 1 N HCl solution to bring the pH to 7.5. The desired guanidine compound precipitated and was separated out by filtration to give 1.1 g (61%) of the title compound. PMR ($CD_3OD$) δ 7.76-7.43 (AB q, J = 8.6 Hz, 4H), 4.73 (s, 2H), 3.9-3.7 (m, 3H), 3.68-3.5 (m, 2H), 2.6-1.67 (m, 6H) and 1.54-1.32 (m, 2H). CMR ($CD_3OD$) δ 159.8, 155.6, 142.9, 134.9, 124.3, 119.4, 109.7, 74.3, 70.2, 38.7, 38.5, 31.3, 30.3 and 25.5. The compound was tasted and was sweet at a concentration of 100 µg/ml. The other isomer remained dissolved in the mother liquor.

All the compounds presented in Examples 1 through 42 were tasted and were sweet.

## EXAMPLE 43

Stability of 1′-N-[N-cyclooctylamino(4-cyanophenylimino)methyl]-5-aminomethyltetrazole (Cpd 1):

Compound 1 above was dissolved in an aqueous sodium phosphate buffer at a pH of 3 at a concentration of 30 µg/ml. The buffer was prepared by adding concentrated phosphoric acid to sodium phosphate diboric until the pH was 3.0. This solution was then maintained at 90° C for 6 days. The percent of compound 1 remaining after 6 days was 98%. Figure 1 shows the "percent remaining" data calculated by HPLC of compound 1 at 90° C and of the glycine derivative at 70° C. The half-life was estimated to be 177 days in this buffer at 90° C. The stability of the glycine derivative of this compound, i.e., 1′-N-[N-cyclooctylamino(4-cyanophenylimino)methyl]-glycine was determined in the same way as Cpd. 1 except at 70° C and 50° C instead of 90° C. The half-life of the glycine derivative was found to be 5 days (70° C) and 13 days (50° C). The calculated half-life at 90° C is about 2 days.

Table I below lists various compounds according to the invention.

TABLE I

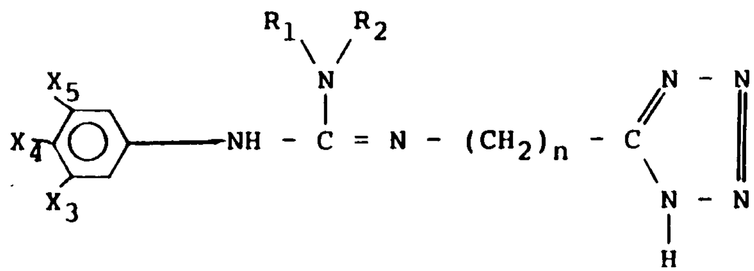

| Cpd. | $X_3$ | $X_4$ | $X_5$ | n | $R_1$ | $R_2$ |
|---|---|---|---|---|---|---|
| 1 | H | CN | H | 1 | H | c-$C_8H_{15}$ |
| 2 | H | CN | H | 1 | H | c-$C_6H_{11}$ |
| 3 | H | CN | H | 0 | H | c-$C_8H_{15}$ |
| 4 | H | CN | H | 1 | H | (S)$CH(CH_3)C_6H_5$ |
| 5 | H | H | H | 1 | H | (S)$CH(CH_3)C_6H_5$ |
| 6 | Cl | H | Cl | 1 | H | (S)$CH(CH_3)C_6H_5$ |
| 7 | Cl | H | Cl | 1 | H | $CH_2C_6H_5$ |
| 8 | H | CN | H | 1 | H | $CH_2C_6H_5$ |
| 9 | Cl | H | Cl | 1 | H | c-$C_8H_{15}$ |
| 10 | Cl | H | Cl | 1 | H | c-$C_6H_{11}$ |
| 11 | $CH_3$ | H | $CH_3$ | 1 | H | (S)$CH(CH_3)C_6H_5$ |
| 12 | H | $C(O)OCH_3$ | H | 1 | H | (S)$CH(CH_3)C_6H_5$ |
| 13 | H | CN | H | 1 | H | 1-napthyl |

| Cpd. | $X_3$ | $X_4$ | $X_5$ | n | $R_1$ | $R_2$ |
|---|---|---|---|---|---|---|
| 14 | H | CN | H | 1 | H | exo-2-norbornyl |
| 15 | H | CN | H | 1 | H | endo-2-norbornyl |
| 16 | H | CN | H | 1 | H | $CH_2$-$\underline{c}$-$C_7H_{13}$ |
| 17 | H | CN | H | 1 | H | n-$C_9H_{19}$ |
| 18 | H | CN | H | 1 | H | n-$C_{12}H_{25}$ |
| 19 | Cl | H | Cl | 1 | H | $SO_2C_6H_5$ |
| 20 | H | CN | H | 1 | H | $SO_2C_6H_5$ |
| 21 | H | CN | H | 1 | H | $SO_2(2,4,6)(C_3H_7)_3C_6H_2$ |
| 22 | H | CN | H | 1 | H | $SO_2(2,4,6)(CH_3)_3C_6H_2$ |
| 23 | Cl | H | Cl | 1 | H | $CH_3$ |
| 24 | Cl | H | Cl | 1 | H | napthyl |
| 25 | H | CN | H | 1 | H | $CH_3$ |
| 26 | H | CN | H | 1 | H | $C_{16}H_{33}$ |
| 27 | H | CN | H | 1 | H | $C_4H_9$-$\underline{c}$-$C_6H_{13}$ |
| 28 | H | CN | H | 1 | H | 1-indanyl ($\underline{c}$-$C_9H_{10}$) |
| 29 | H | CN | H | 1 | H | 2-indanyl ($\underline{c}$-$C_9H_{10}$) |
| 30 | H | CN | H | 1 | H | 1-tetralin ($\underline{c}$-$C_{10}H_{12}$) |
| 31 | H | CN | H | 1 | H | 2-decalin ($\underline{c}$-$C_{10}H_{18}$) |
| 32 | H | CN | H | 1 | H | 1-$C_6H_3$-3,4-($OCH_2O$) |
| 33 | H | CN | H | 1 | H | $\underline{c}$-$C_4H_7SO_2$ |
| 34 | H | C=N-NH | | 1 | H | $\underline{c}$-$C_8H_{17}$ |

| Cpd. | $X_3$ | $X_4$ | $X_5$ | n | $R_1$ | $R_2$ |
|---|---|---|---|---|---|---|
| 35 | H | CN | H | 1 | H | $\underline{c}-C_5H_{10}O$ |
| 36 | H | CN | H | 1 | H | $CH(C_2H_5)(C_6H_5)$ |
| 37 | H | CN | H | 1 | H | $CH(C_6H_5)(C_6H_5)$ |
| 38 | H | CN | H | 1 | H | $CH(C_6H_{11})C_6H_5$ |
| 39 | H | CN | H | 1 | H | $CH_2-\underline{c}-C_4H_3O$ |
| 40 | H | CN | H | 1 | H | $CH_2-\underline{c}-C_4H_3S$ |
| 41 | H | CN | H | 1 | H | H |
| 42 | H | CN | H | 1 | H | $2-C_8H_{13}O$ |

In similar operations, the various guanidine compounds described in formula I are prepared and are used to sweeten food products.

## Claims

### Claims for the following Contracting States : AT, BE, CH, DE, FR, GB, GR, IT, LI, LU, NL, SE

**1.** A compound corresponding to the formula,

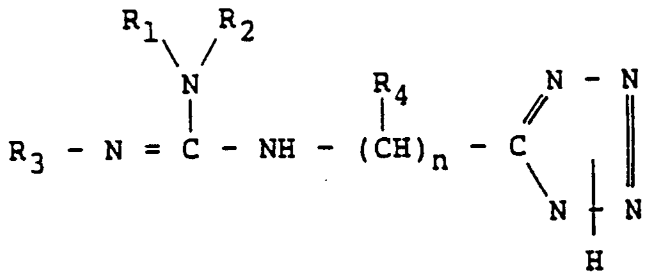

wherein $R_3$ is optionally substituted pyridines, thiazoles, indoles, quinolines, naphthyridines, cinnolines, pteridines, thiophenes, benzothiophenes, naphthothiophenes, thianthrenes, furans, pyrans, isobenzofurans, chromenes, xanthenes, phenoxathins, pyrroles, indoles, isoindoles, indolizines, pyridazines, pyrimidines, pyrazines, pyrazoles, imidazoles, pyrroles, indazoles, purines, quinolizines, isoquinolines, quinolines, phthalazines, quinoxalines, quinazolines, carbazoles, carbolines, phenanthridines, acridines, pyrimidines, phenanthrolines, phenazines, phenarsazines, isothiazoles, phenothiazines, isoxazoles, thiazoles, furazans and heterocyclics of the following formulas:

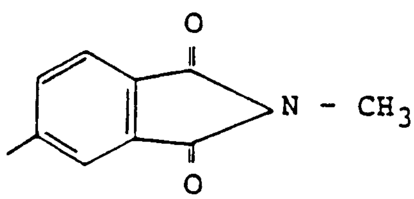

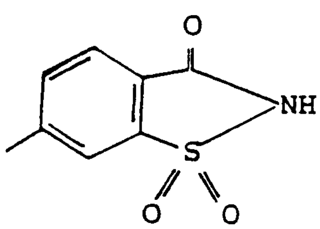

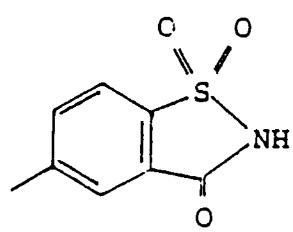

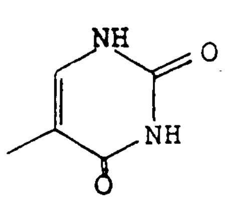

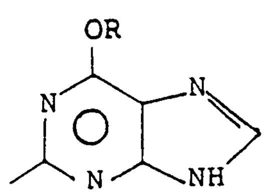

wherein R is H or $C_1$-$C_6$ alkyl and wherein heterocyclic moieties may be optionally substituted with one or more substituents selected from $C_1$-$C_6$ alkyl, halogen, $NO_2$, CN, trihalomethyl, dihalomethyl, hydroxyl or hydroxyalkyl, or an optionally substituted phenyl corresponding to

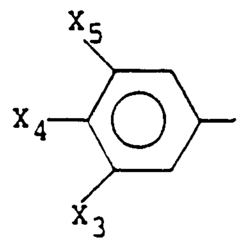

wherein $X_3$, $X_4$ and $X_5$ are the same or different and are selected from the group consisting of

H,
Br,
$CF_3$,
$CF_2CF_3$,
$CH_2CF_3$,
$C_1$-$C_4$ alkyl,
$CH=NOCH_3$,
$CH=NOH$,
CHO,
$CH_2OCH_3$,
$CH_2OH$,
Cl,
CN,
$COCF_3$,
$COC_1$-$C_3$ alkyl,
$CONH_2$,
$CONHC_1$-$C_3$ alkyl,
$CON(C_1$-$C_3$ alkyl$)_2$,
$COOC_1$-$C_3$ alkyl,
COOH,
F,
I,
$NH_2$,
$NHC_1$-$C_3$ alkyl,
$N(C_1$-$C_3$ alkyl$)_2$,
NHCHO,
$NHCOCH_3$,
$NHCONH_2$,
$NHSO_2CH_3$,
$NO_2$,
$OC_1$-$C_3$ alkyl,
$C(O)OCH_3$,
OH,
$SC_1$-$C_3$ alkyl,
$SOC_1$-$C_3$ alkyl,
$SO_2C_1$-$C_3$ alkyl,
$SO_2NH_2$,
$SO_2NHC_1$-$C_3$ alkyl,
$SO_2N(C_1$-$C_3$ alkyl$)_2$, and
$SO_3H$,

or wherein substituents at $X_4$ and $X_5$ form a fused ring;
wherein $R_1$ is an atom of hydrogen or $R_1$ is a $C_1$ to $C_4$ saturated, unsaturated, acyclic, cyclic or mixed hydrocarbyl or modified hydrocarbyl group and wherein, in the modified hydrocarbyl group,

1 to 2 atoms of carbon may be replaced by 1 to 2 of the same or different heteroatoms selected from the group consisting of N, O, S, Cl, Br and I,

and 1 to 3 atoms of hydrogen may be replaced by 1 to 3 atoms of fluorine, oxygen, or nitrogen;
wherein $R_2$ is hydrogen, CN, $NO_2$ or a $C_1$ to $C_{20}$ saturated, unsaturated, acyclic, cyclic or mixed hydrocarbyl or modified hydrocarbyl group and wherein, in the modified hydrocarbyl group,
1 to 4 atoms of carbon may be replaced by 1 to 4 of the same or different heteroatoms selected from the group consisting of N, O, S, Cl, Br and I,
and 1 to 5 atoms of hydrogen may be replaced by 1 to 5 atoms of fluorine, oxygen, or nitrogen;
n is 0, 1, 2, or 3;
wherein $R_1$ and $R_2$ can be fused;
wherein $R_4$ is H or $C_1$-$C_6$ alkyl with the proviso that $R_4$ can only be alkyl on a single carbon atom when n = 2 or 3; and
tautomers and physiologically acceptable salts thereof.

**2.** The compound of Claim 1 wherein $R_1$ is H.

**3.** The compound of Claim 1 wherein $R_3$ is an optionally substituted phenyl and $R_1$ is H.

**4.** The compound of Claim 3 wherein $R_4$ is H and n is 1.

**5.** The compound of Claim 1, 2, 3 or 4 wherein $X_3$ and $X_5$ are selected from the group consisting of

H,
Br,
$CF_3$,
$CH_3$,
Cl and
F.

**6.** The compound of Claim 1, 2, 3 or 4 wherein $X_4$ is selected from the group consisting of

H,
CN,
$C(O)OCH_3$,
F and
$NO_2$.

**7.** The compound of Claim 1, 2, 3 or 4 wherein $R_2$ is selected from the group consisting of
Normal alk(en)(yn)yl $C_2$-$C_{13}$,
Branched alk(en)(yn)yl $C_3$-$C_{13}$,
Cycloalk(en)yl $C_3$-$C_{13}$,
Alk(en)yl cycloalk(en)yl $C_4$-$C_{13}$,
Cycloalk(en)yl alk(en)yl $C_4$-$C_{13}$,
Alk(en)yl cycloalk(en)yl alk(en)yl $C_5$-$C_{13}$,
Alk(en)yl bicycloalk(en)yl $C_7$-$C_{13}$,
Fused bicycloalk(en)yl $C_7$-$C_{13}$,
Alk(en)yl fused bicycloalk(en)yl $C_8$-$C_{13}$,
Fused bicycloalk(en)yl alk(en)yl $C_8$-$C_{13}$,
Alkenyl fused bicycloalk(en)yl alk(en)yl $C_9$-$C_{13}$,
Fused tricycloalk(en)yl $C_{10}$-$C_{13}$,
Alk(en)yl fused tricycloalk(en)yl $C_{11}$-$C_{13}$,
Fused tricycloalk(en)yl alk(en)yl $C_{11}$-$C_{13}$ and
Alk(en)yl fused tricycloalk(en)yl alk(en)yl $C_{11}$-$C_{13}$.

**8.** The compound of Claim 7 wherein $R_2$ is selected from the group consisting of
n-$C_5H_{11}$, n-$C_6H_{13}$, n-$C_7H_{15}$,
$CH(CH_3)(CH_2)_2CH_3$, $CH(CH_3)(CH_2)_3CH_3$, $(CH_2)_3CH(CH_3)_2$, $(CH_2)_4CH(CH_3)_2$, $(CH_2)_5CH(CH_3)_2$,
$C_6H_5$, $C_6$-$C_{10}$ cycloalkyl, $(CH_3CH_2CH_2CH_2)C_6$-$C_{10}$ cycloalkyl,
$CH_2C_6H_5$, $CH_2$-c-$C_6H_{11}$, $CH(CH_3)C_6H_5$, $CH(C_6H_5)C_6H_5$, $CH(CH_3)$-c-$C_6H_{11}$, $(CH_2)_2C_6H_5$, $(CH_2)_2$-c-$C_6H_{11}$, $CH(CH_3)CH_2C_6H_5$, $CH(CH_3)CH_2$-c-$C_6H_{11}$,

$C_6H_4(CH_3)$, $C_6$-$C_{10}$ cycloalkyl($CH_3$),
$CH_2C_6H_4(CH_3)$, $CH_2$-c-$C_6H_{10}(CH_3)$, $CH(CH_3)C_6H_4(CH_3)$, $CH(CH_3)$-c-$C_6H_{10}(CH_3)$,
$(CH_2)_2CH(c\text{-}C_3H_5)_2$, $(CH_2)_3CH(c\text{-}C_3H_5)_2$, $CH(CH_3)CH_2CH(c\text{-}C_3H_5)_2$, $CH(CH_3)(CH_2)_2CH(c\text{-}C_3H_5)_2$,
naphthyl, 5,6,7,8-tetrahydronaphthyl, perhydronaphthyl, indenyl, indanyl,
naphthyl($CH_3$), 5,6,7,8-tetrahydronaphthyl($CH_3$), perhydronaphthyl($CH_3$), indenyl($CH_3$), indanyl-($CH_3$), fenchyl,
$CH_2$-naphthyl, $CH_2$-5,6,7,8-tetrahydronaphthyl, $CH_2$-perhydronaphthyl, $CH_2$-indenyl, $CH_2$-indanyl,
$CH_2$-naphthyl($CH_3$), $CH_2$-5,6,7,8-tetrahydronaphthyl($CH_3$), $CH_2$-perhydronaphthyl($CH_3$), $CH_2$-indenyl($CH_3$), $CH_2$-indanyl($CH_3$),
adamantyl, endo-norbornyl, exo-norbornyl,
$CH_2$-adamantyl, $CH(CH_3)$adamantyl, tetralyl, decalyl, and
$CH_2$-adamantyl($CH_3$).

**9.** The compound of Claim 1, 2, 3 or 4 wherein $R_2$ is a modified hydrocarbyl group wherein up to four carbon atoms may be replaced by the same or different heteroatoms selected from a group consisting of
S to replace C or $CH_2$,
N to replace CH or $CH_3$,
NH and O to replace $CH_2$ and
Cl, Br and I to replace $CH_3$,
wherein up to 5 atoms of hydrogen may be substituted by fluorine atoms.

**10.** The compound of Claim 9 wherein $R_2$ is selected from the group consisting of
pyridinyl, pyridinyl methyl, piperidyl, homopiperidyl, indolyl, indolinyl, isoindolinyl, quinolyl, isoquinolyl, pyrazinyl, pyrimidyl, indazolyl, quinoxalinyl, quinazolinyl, purinyl,
pyranyl, tetrahydropyranyl, benzofuranyl, methoxyphenyl, methyloxycarbonylphenyl, 3,4-methylenedioxyphenyl, 6-oxo-cis-hydrindane, morpholinyl, benzoxazolyl, acetamidophenyl, cyano, nitro,
thienyl, thienylmethyl, benzothienyl, tetrahydro-3-thienyl 1,1, dioxide, 2,2,4,4-tetramethylthiacyclobut-3-yl, thiazolyl, isothiazolyl, $SO_2C_6H_5$, $SO_2$c-$C_6H_{11}$, $SO_2$c-$C_7H_{13}$,
chlorophenyl,
fluorophenyl and
trifluoromethylphenyl.

**11.** The compound of Claim 1 having the formula

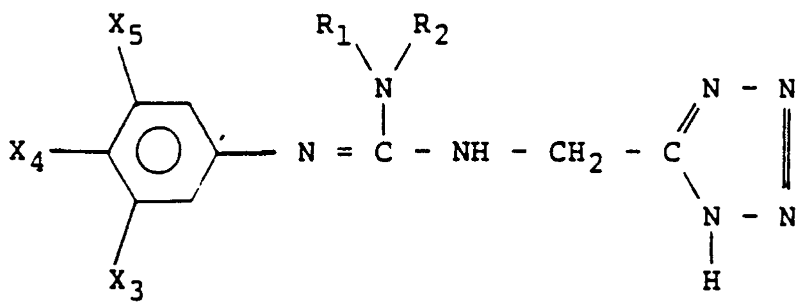

**12.** The compound of Claim 11 wherein $R_1$ is $CH_3$.

**13.** The compound of Claim 11 wherein $X_3$ and $X_5$ are H and $X_4$ is CN.

**14.** The compound of Claim 11 wherein $X_3$ and $X_5$ are selected from the group consisting of $CF_3$, $CH_3$, Cl and F and wherein $X_4$ is selected from the group consisting of H and CN.

**15.** The compound of Claim 11 wherein $R_2$ is selected from the group consisting of (S)$CH(CH_3)C_6H_5$, $CH_2C_6H_5$, $CH(CH_3)$-c-$C_6H_{11}$, $CH(C_6H_5)_2$, c-$C_6H_{11}$, c-$C_7H_{13}$, c-$C_8H_{15}$, c-$C_9H_{17}$, c-$C_{10}H_{19}$, 2-decalyl, endo-norbornyl, 1-naphthyl, $SO_2C_6H_5$ and $SO_2$c-$C_7H_{13}$.

**16.** The compound of Claim 11 wherein $R_1$ is selected from the group consisting of H and $CH_3$, wherein $X_3$ and $X_5$ are H and $X_4$ is CN or wherein $X_3$ and $X_5$ are selected from the group consisting of $CF_3$, $CH_3$, Cl, H and F and wherein $X_4$ is selected from the group consisting of H and CN; and wherein $R_2$ is selected from the group consisting of (S)$CH(CH_3)C_6H_5$, $CH_2C_6H_5$, endo-norbornyl, 1-naphthyl, CH-($CH_3$)-c-$C_6H_{11}$, $CH(C_6H_5)_2$, c-$C_6H_{11}$, c-$C_7H_{13}$, c-$C_8H_{15}$, c-$C_9H_{17}$, c-$C_{10}H_{19}$, 2-decalyl, $SO_2C_6H_5$ and $SO_2$c-$C_7H_{13}$.

**17.** The compound of Claim 16 wherein $R_1$, $X_3$ and $X_5$ are H, $X_4$ is CN and $R_2$ is c-$C_9H_{17}$, including tautomeric forms thereof and physiologically acceptable salts thereof.

**18.** The compound of Claim 16 wherein $R_1$, $X_3$ and $X_5$ are H, $X_4$ is CN and $R_2$ is c-$C_8H_{15}$, including tautomeric forms thereof and physiologically acceptable salts thereof.

**19.** The compound of Claim 16 wherein $R_1$ and $X_4$ are H, $X_3$ and $X_5$ are Cl and $R_2$ is (S)$CH(CH_3)C_6H_5$, including tautomeric forms thereof and physiologically acceptable salts thereof.

**20.** The compound of Claim 16 wherein $R_1$ and $X_5$ are H, $X_3$ is Cl, $X_4$ is CN and $R_2$ is c-$C_8H_{15}$, including tautomeric forms thereof and physiologically acceptable salts thereof.

**21.** The compound of Claim 16 wherein $R_1$ and $X_5$ are H, $X_3$ is $CH_3$, $X_4$ is CN and $R_2$ is c-$C_8H_{15}$, including tautomeric forms thereof and physiologically acceptable salts thereof.

**22.** The compound of Claim 16 wherein $R_1$ and $X_4$ are H, $X_3$ and $X_5$ are Cl and $R_2$ is $CH_2C_6H_5$, including tautomeric forms thereof and physiologically acceptable salts thereof.

**23.** The compound of Claim 16 wherein $R_1$ is $CH_3$, $X_4$ is H, $X_3$ and $X_5$ are Cl and $R_2$ is $CH_2C_6H_5$, including tautomeric forms thereof and physiologically acceptable salts thereof.

**24.** The compound of Claim 16 wherein $R_1$ and $X_4$ are H, $X_3$ and $X_5$ are Cl and $R_2$ is (S)$CH(CH_3)$-c-$C_6H_{11}$, including tautomeric forms thereof and physiologically acceptable salts thereof.

**25.** The compound of Claim 16 wherein $R_1$, $X_3$ and $X_5$ are H, $X_4$ is CN and $R_2$ is (S)$CH(CH_3)C_6H_5$, including tautomeric forms thereof and physiologically acceptable salts thereof.

**26.** The compound of Claim 16 wherein $R_1$, $X_3$, and $X_5$ are H, $X_4$ is CN and $R_2$ is $CH(C_6H_5)_2$, including tautomeric forms thereof and physiologically acceptable salts thereof.

**27.** The compound of Claim 16 wherein $R_1$ and $X_4$ are H, $X_3$ and $X_5$ are Cl and $R_2$ is c-$C_7H_{13}$, including tautomeric forms thereof and physiologically acceptable salts thereof.

**28.** The compound of Claim 16 wherein $X_4$ is CN, $R_1$, $X_3$ and $X_5$ are H, and $R_2$ is c-$C_{10}H_{17}$, including tautomeric forms thereof and physiologically acceptable salts thereof.

**29.** The compound of Claim 1 wherein the compound is selected from the group of physiologically acceptable salts consisting of hydrochloride, phosphate, citrate, sulfate, sodium, potassium, ammonium, calcium and magnesium salts.

**30.** A process for sweetening edible products including foods, beverages, confections, chewing gums, pharmaceuticals, veterinary preparations and toilet, cosmetic and hygiene products, characterized in that it comprises adding to the substance an effective sweetening amount of a compound of Claim 1.

**31.** Edible products sweetened according to the process of Claim 30.

**32.** Sweetening compositions characterized in that they comprise an effective sweetening amount of a compound of Claim 1 and a physiologically acceptable carrier therefor.

**33.** The sweetening compositions of Claim 32 wherein the carrier is a bulking agent.

**34.** The sweetening compositions of Claim 32 wherein the carrier is selected from the group consisting of polydextrose, starch, malto-dextrins, cellulose, methylcellulose, carboxymethylcellulose, maltitol, hydroxymethylcellulose, microcrystalline cellulose, sodium alginate, pectins, gums, lactose, maltose, glucose, leucine, glycerol, mannitol, sorbitol, sodium bicarbonate and phosphoric, citric, tartaric, fumaric, benzoic, sorbic, propionic acids and their sodium, potassium and calcium salts and mixtures of any of the above.

**35.** A sweetening composition comprising:
(a) a compound of Claim 1; and
(b) a different sweetening agent.

**36.** The sweetening composition of Claim 35 further comprising a bulking agent.

**37.** The sweetening composition of Claim 36 wherein the different sweetening agent is selected from the group consisting of sucrose, corn syrups, fructose, aspartame, alitame, neohesperidine dihydrochalcone, high fructose corn syrup, hydrogenated isomaltulose, stevioside, L-sugars, glycyrrhizin, xylitol, acesulfam-K, saccharin (sodium, potassium or calcium salt), cyclamic acid (sodium, potassium or calcium salt), trichlorogalactosucrose, monellin and thaumatin and mixtures thereof.

**38.** A compound of the formula

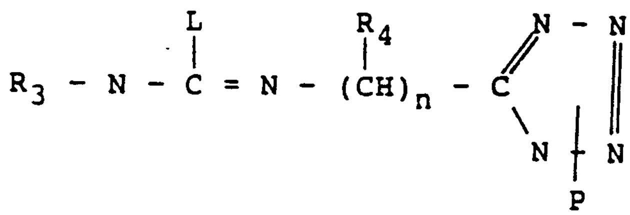

wherein L is S-alkyl, O-alkyl, $OSO_2$-aryl, $SO_3H$ or halogen, P is a protecting group and, $R_3$, $R_4$ and n are as defined in Claim 1.

**39.** The compound of Claim 38 wherein L is S-methyl.

**40.** The compound of Claim 38 wherein $R_3$ is 4-cyanophenyl, 3,5-dichlorophenyl, 3,5-dimethylphenyl, 6-indazolylimino or phenyl.

**41.** The compound of Claim 38 wherein P is

H,

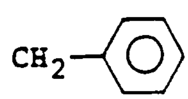 ,

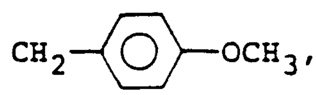 ,

$CH_2OCO\text{-}t\text{-}C_4H_9$ or
$CH_2CH_2COOC_2H_5$

**42.** The compound of Claim 40 wherein $R_4$ is H.

**43.** The compound of Claim 42 wherein n is 1.

**44.** The compound of Claim 42 wherein n is 0.

**45.** A compound of the formula

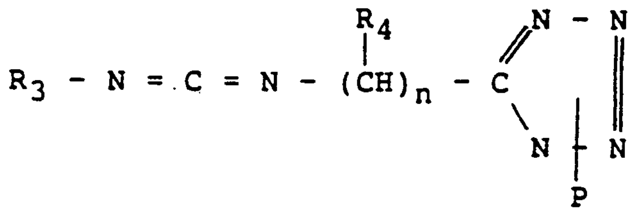

wherein P is a protecting group and $R_3$, $R_4$ and n are as defined in Claim 1.

**46.** The compound of Claim 45 wherein $R_4$ is H.

**47.** The compound of Claim 46 wherein $R_3$ is substituted phenyl.

**48.** The compound of Claim 47 wherein $R_3$ is 4-cyanophenyl 3,5-dichlorophenyl, 3,5-dimethylphenyl or phenyl.

**49.** The compound of Claim 45 wherein P is

H,

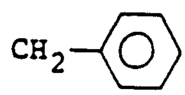

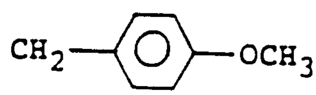

$CH_2OCO\text{-}t\text{-}C_4H_9$ or

$CH_2CH_2COOC_2H_5$

**50.** The compound of Claim 45 wherein n is 0.

**51.** The compound of Claim 45 wherein n is 1.

**52.** The compound of Claim 48 wherein $R_4$ and P are H and n is 1.

**53.** A process comprising:
(a) reacting a compound of the formula

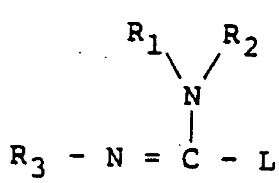

with a compound of the formula

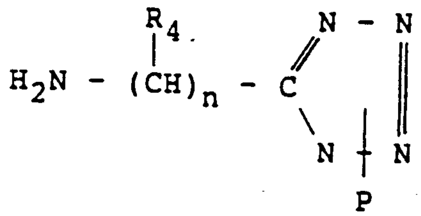

wherein L is S-alkyl, O-alkyl, $OSO_2$-aryl, $SO_3H$ or halogen and P is a protecting group and $R_1$, $R_2$, $R_3$, $R_4$ and n are as defined in Claim 1 under conditions sufficient to form the corresponding guanidine and
(b) recovering the guanidine compound formed in (a) above.

**54.** The process of Claim 53 wherein P is

H,

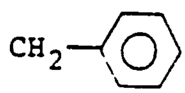

,

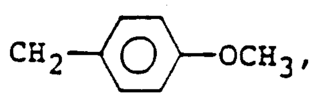

,

$CH_2OCO\text{-}t\text{-}C_4H_9$ or
$CH_2CH_2COOC_2H_5$

**55.** The process of Claim 53 wherein L is -S-$CH_3$.

**56.** The process of Claim 53 wherein $R_3$ is a substituted phenyl and $R_1$ and $R_4$ are H.

**57.** The process of Claim 56 wherein $R_2$ is cyclohexyl, cyclooctyl, alpha-phenethyl, diphenyl methyl, benzyl, phenyl, endo-norbornyl, 1-naphthyl, or 2-decalyl.

**58.** A process comprising:
(a) reacting a carbodiimide of the formula

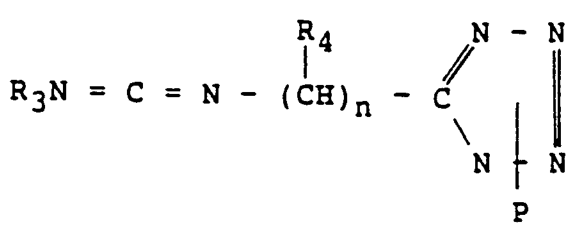

with an amine of the formula

$HNR_1R_2$

wherein P is a protective group, $R_1$, $R_2$, $R_3$, $R_4$ and n are as defined in Claim 1 under conditions sufficient to form the corresponding guanidine and
(b) recovering the guanidine compound formed in (a) above.

**59.** The process of Claim 58 wherein P is

H,

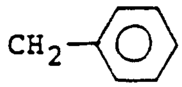 ,

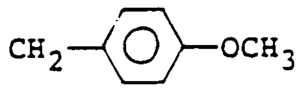

$CH_2OCO\text{-}t\text{-}C_4H_9$ or
$CH_2CH_2COOC_2H_5$

**60.** The process of Claim 58 wherein $R_1$ and $R_4$ are H.

**61.** The process of Claim 60 wherein $R_3$ is a substituted phenyl.

**62.** The process of Claim 61 wherein $R_3$ is 4-cyanophenyl, 3,5-dichlorophenyl, 3,5-dimethylphenyl or phenyl.

**63.** The process of Claim 60 wherein $R_2$ is cyclohexyl, cyclooctyl, alpha-phenethyl, diphenyl methyl, benzyl, phenyl, endo-norbornyl, 1-naphthyl, or 2-decalyl.

**64.** The process of Claim 63 wherein $R_3$ is a substituted phenyl.

**65.** The process of Claim 64 wherein $R_3$ is 4-cyanophenyl, 3,5-dichlorophenyl, 3,5-dimethylphenyl or phenyl.

**66.** A process comprising
(a) reacting a carbodiimide compound of the formula

$R_3 - N = C = N - R_2$

with an amine of the formula

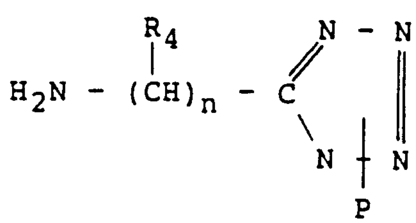

wherein P is a protecting group, $R_2$, $R_3$, $R_4$ and n are as defined in Claim 1 under conditions sufficient to form the corresponding guanidine compound and
(b) recovering the guanidine compound formed in (a) above.

**67.** The process of Claim 66 wherein P is

H,

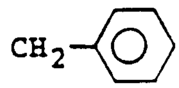 ,

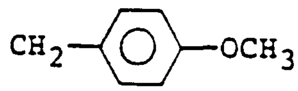 ,

$CH_2OCO\text{-}t\text{-}C_4H_9$ or

$CH_2CH_2COOC_2H_5$

**68.** The process of Claim 66 wherein $R_4$ and P are H.

**69.** The process of Claim 68 wherein $R_3$ is a substituted phenyl.

**70.** The process of Claim 69 wherein $R_3$ is 4-cyanophenyl, 3,5-dichlorophenyl, 3,5-dimethylphenyl.

**71.** The process of Claim 68 wherein $R_2$ is phenyl, benzyl, cyclohexyl, cyclooctyl, endo-norbornyl, 1-naphthyl, 2-decalyl, alpha-phenethyl or diphenyl methyl.

**72.** The process of Claim 71 wherein $R_3$ is a substituted phenyl.

**73.** The process of Claim 72 wherein $R_3$ is 4-cyanophenyl, 3,5-dichlorophenyl, 3,5-dimethylphenyl or phenyl.

**74.** The compound of Claim 1 having the formula

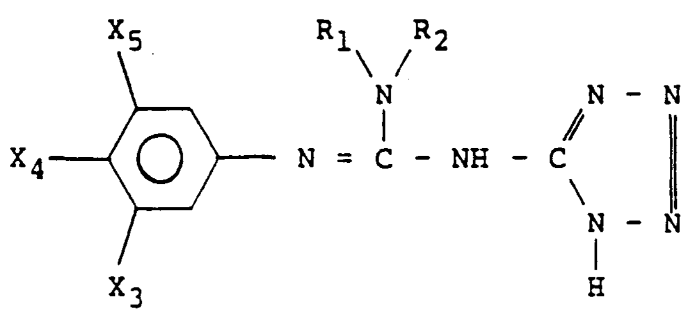

**75.** The compound of Claim 74 wherein $R_1$ is H.

**76.** The compound of Claim 75 wherein $X_3$ and $X_5$ are H and $X_4$ is CN.

**77.** The compound of Claim 76, wherein $R_2$ is phenyl, benzyl, cyclohexyl, cyclooctyl, cyclononyl, 4-pyranyl, 2-decalyl, endo-norbornyl, 1-naphthyl, alpha-phenethyl, or benzhydryl.

**78.** The compound of Claim 75 wherein $X_4$ is H and $X_3$ and $X_5$ are Cl or $CH_3$.

**79.** The compound of Claim 78 wherein $R_2$ is phenyl, benzyl, cyclohexyl, cyclooctyl, cyclononyl, 4-pyranyl, 2-decalyl, endo-norbornyl, 1-naphthyl, alpha-phenethyl, or benzhydryl.

**Claims for the following Contracting State : ES**

**1.** A process for preparing a compound of the formula

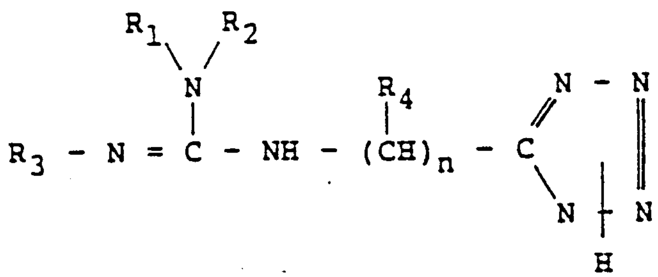

wherein $R_3$ is
optionally substituted pyridines, thiazoles, indoles, quinolines, naphthyridines, cinnolines, pteridines, thiophenes, benzothiophenes, naphthothiophenes, thianthrenes, furans, pyrans, isobenzofurans, chromenes, xanthenes, phenoxathins, pyrroles, indoles, isoindoles, indolizines, pyridazines, pyrimidines, pyrazines, pyrazoles, imidazoles, pyrroles, indazoles, purines, quinolizines, isoquinolines, quinolines, phthalazines, quinoxalines, quinazolines, carbazoles, carbolines, phenanthridines, acridines, pyrimidines, phenanthrolines, phenazines, phenarsazines, isothiazoles, phenothiazines, isoxazoles, thiazoles, furazans and heterocyclics of the following formulas:

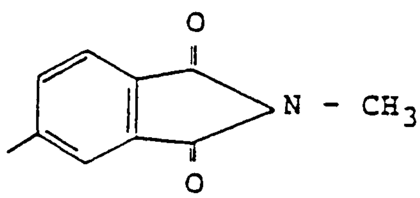

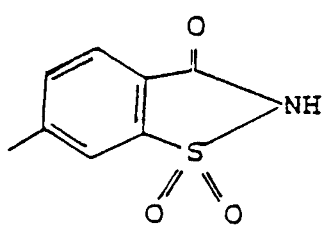

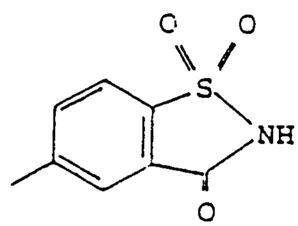

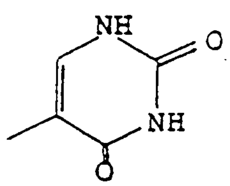

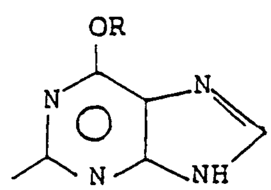

wherein R is H or $C_1$-$C_6$ alkyl and wherein heterocyclic moieties may be optionally substituted with one or more substituents selected from $C_1$-$C_6$ alkyl, halogen, $NO_2$, CN, trihalomethyl, dihalomethyl, hydroxyl or hydroxyalkyl, or an optionally substituted phenyl corresponding to

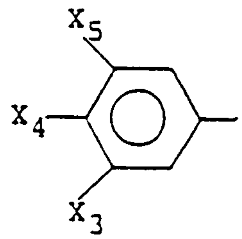

wherein $X_3$, $X_4$ and $X_5$ are the same or different and are selected from the group consisting of

H,
Br,
$CF_3$,
$CF_2CF_3$,
$CH_2CF_3$,
$C_1$-$C_4$ alkyl,
$CH=NOCH_3$,
$CH=NOH$,
CHO,
$CH_2OCH_3$,
$CH_2OH$,
Cl,
CN,
$COCF_3$,
$COC_1$-$C_3$ alkyl,
$CONH_2$,
$CONHC_1$-$C_3$ alkyl,
$CON(C_1$-$C_3$ alkyl$)_2$,
$COOC_1$-$C_3$ alkyl,
COOH,
F,
I,
$NH_2$,
$NHC_1$-$C_3$ alkyl,
$N(C_1$-$C_3$ alkyl$)_2$,
NHCHO,
$NHCOCH_3$,
$NHCONH_2$,
$NHSO_2CH_3$,
$NO_2$,
$OC_1$-$C_3$ alkyl,
$C(O)OCH_3$,
OH,
$SC_1$-$C_3$ alkyl,
$SOC_1$-$C_3$ alkyl,
$SO_2C_1$-$C_3$ alkyl,
$SO_2NH_2$,
$SO_2NHC_1$-$C_3$ alkyl,
$SO_2N(C_1$-$C_3$ alkyl$)_2$, and
$SO_3H$,

or wherein substituents at $X_4$ and $X_5$ form a fused ring;
wherein $R_1$ is an atom of hydrogen or $R_1$ is a $C_1$ to $C_4$ saturated, unsaturated, acyclic, cyclic or mixed hydrocarbyl or modified hydrocarbyl group and wherein, in the modified hydrocarbyl group,

1 to 2 atoms of carbon may be replaced by 1 to 2 of the same or different heteroatoms selected from the group consisting of N, O, S, Cl, Br and I,

and 1 to 3 atoms of hydrogen may be replaced by 1 to 3 atoms of fluorine, oxygen, or nitrogen;
wherein $R_2$ is hydrogen, CN, $NO_2$ or a $C_1$ to $C_{20}$ saturated, unsaturated, acyclic, cyclic or mixed hydrocarbyl or modified hydrocarbyl group and wherein, in the modified hydrocarbyl group,
1 to 4 atoms of carbon may be replaced by 1 to 4 of the same or different heteroatoms selected from the group consisting of N, O, S, Cl, Br and I,
and 1 to 5 atoms of hydrogen may be replaced by 1 to 5 atoms of fluorine, oxygen, or nitrogen;
n is 0, 1, 2, or 3;
wherein $R_1$ and $R_2$ can be fused;
wherein $R_4$ is H or $C_1$-$C_6$ alkyl with the proviso that $R_4$ can only be alkyl on a single carbon atom when n = 2 or 3; and
tautomers and physiologically acceptable salts thereof
characterized in the
(a) reacting a compound of the formula

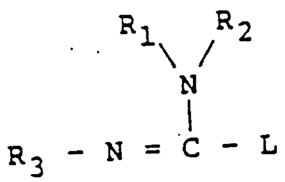

with a compound of the formula

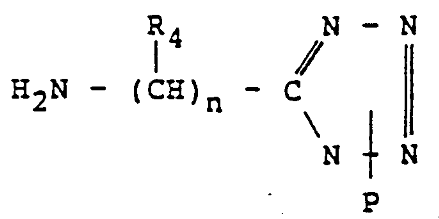

wherein L is S-alkyl, O-alkyl, $OSO_2$-aryl, $SO_3H$ or halogen and P is a protecting group and
$R_1$, $R_2$, $R_3$, $R_4$ and n are as defined in Claim 1 under conditions sufficient to form the corresponding guanidine and
(b) recovering the guanidine compound formed in (a) above
and optionally transforming the obtained compound in a known manner into the respective physiologically acceptable salts thereof.

**2.** A process for preparing a compound according to Claim 1 characterized in the
(a) reacting a carbodiimide compound of the formula

$R_3 - N = C = N - R_2$

with an amine of the formula

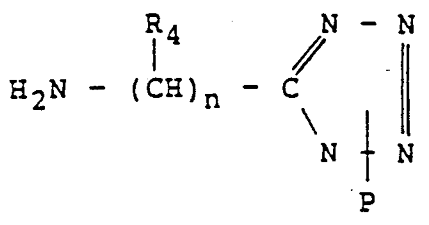

wherein P is a protecting group, $R_2$, $R_3$, $R_4$ and n are as defined in Claim 1 under conditions sufficient to form the corresponding guanidine compound and
(b) recovering the guanidine compound formed in (a) above,
and optionally transforming the obtained compound in a known manner into the respective physiologically acceptable salts thereof.

**3.** A process for preparing a compound according to Claim 1 characterized in the
(a) reacting a carbodiimide of the formula

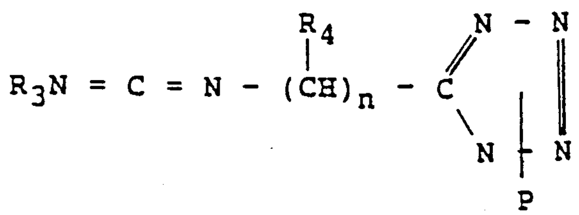

with an amine of the formula

$HNR_1R_2$

wherein P is a protective group, $R_1$, $R_2$, $R_3$, $R_4$ and n are as defined in Claim 1 under conditions sufficient to form the corresponding guanidine and
(b) recovering the guanidine compound formed in (a) above,
and optionally transforming the obtained compound in a known manner into the respective physiologically acceptable salts thereof.

**4.** A process according to any of Claims 1 to 3 characterized in that the physiologically acceptable salts are prepared by transforming the obtained products into the hydrochloride, phosphate, citrate, sulfate, sodium, potassium, ammonium, calcium or magnesium salts.

**5.** A process for sweetening edible products including foods, beverages, confections, chewing gums, pharmaceuticals, veterinary preparations and toilet, cosmetic and hygiene products, characterized in that it comprises adding to the substance an effective sweetening amount of a compound as prepared according to Claim 1.

**6.** Use of an effective sweetening amount of a compound as prepared according to Claim 1 and a physiologically acceptable carrier therefor for preparing sweetening compositions.

**7.** Use according to Claim 6 wherein the carrier is a bulking agent.

**8.** Use according to Claim 6 wherein the carrier is selected from the group consisting of polydextrose, starch, malto-dextrins, cellulose, methylcellulose, carboxymethylcellulose, maltitol, hydroxymethylcellulose, microcrystalline cellulose, sodium alginate, pectins, gums, lactose, maltose, glucose, leucine, glycerol, mannitol, sorbitol, sodium bicarbonate and phosphoric, citric, tartaric, fumaric, benzoic, sorbic, propionic acids and their sodium, potassium and calcium salts and mixtures of any of the above.

**9.** Use of
(a) a compound as prepared according to Claim 1; and
(b) a different sweetening agent
for preparing a sweetening composition.

**10.** Use according to Claim 9 wherein the composition further comprises a bulking agent.

**11.** Use according to Claim 10 wherein the different sweetening agent is selected from the group consisting of sucrose, corn syrups, fructose, aspartame, alitame, neohesperidine dihydrochalcone, high fructose corn syrup, hydrogenated isomaltulose, stevioside, L-sugars, glycyrrhizin, xylitol, acesulfam-K, saccharin (sodium, potassium or calcium salt), cyclamic acid (sodium, potassium or calcium salt), trichlorogalactosucrose, monellin and thaumatin and mixtures thereof.

**Patentansprüche**
**Patentansprüche für folgende Vertragsstaaten : AT, BE, CH, DE, FR, GB, GR, IT, LI, LU, NL, SE**

1. Verbindung gemäß der Formel

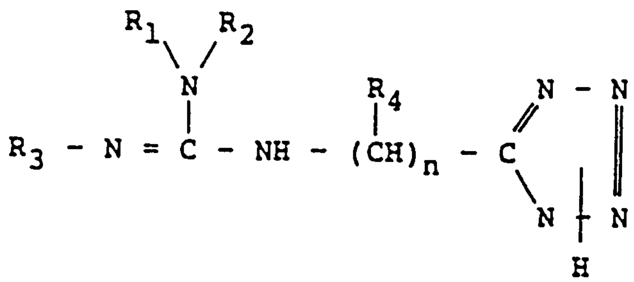

worin $R_3$ für wahlweise substituierte Pyridine, Thiazole, Indole, Chinoline, Naphthyridine, Cinnoline, Pteridine, Thiophene, Benzothiophene, Naphthothiophene, Thianthrene, Furane, Pyrane, Isobenzofurane, Chromene, Xanthene, Phenoxathine, Pyrrole, Indole, Isoindole, Indolizine, Pyridazine, Pyrimidine, Pyrazine, Pyrazole, Imidazole, Pyrrole, Indazole, Purine, Chinolizine, Isochinoline, Chinoline, Phthalazine, Chinoxaline, Chinazoline, Carbazole, Carboline, Phenanthridine, Acridine, Pyrimidine, Phenanthroline, Phenazine, Phenarsazine, Isothiazole, Phenothiazine, Isoxazole, Thiazole, Furazane und Heterocyclen der folgenden Formeln

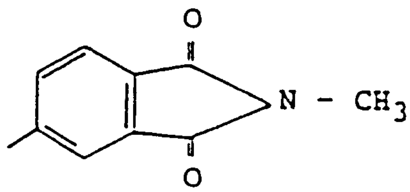

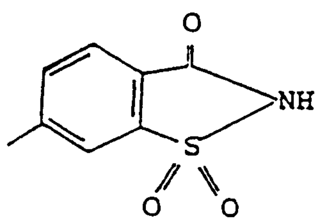

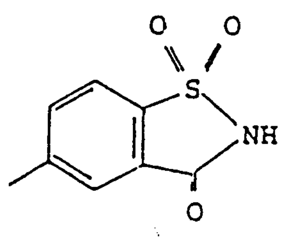

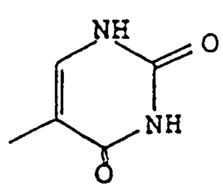

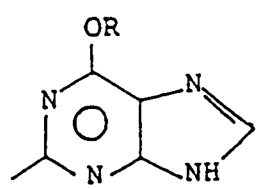

worin R H oder $C_1$-$C_6$-Alkyl ist und worin heterocyclische Einheiten wahlweise substituiert sein können mit einem oder mehreren Substituenten, ausgewählt aus $C_1$-$C_6$-Alkyl, Halogen, $NO_2$, CN, Trihalogenmethyl, Dihalogenmethyl, Hydroxyl oder Hydroxyalkyl, oder für wahlweise substituiertes Phenyl entsprechend

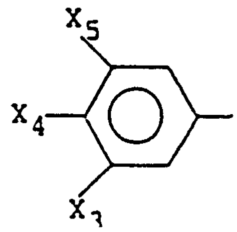

worin $X_3$, $X_4$ und $X_5$ gleich oder verschieden sind und ausgewählt werden aus der Gruppe bestehend aus H, Br, $CF_3$, $CF_2CF_3$, $CH_2CF_3$, $C_1$-$C_4$-Alkyl, $CH=NOCH_3$, $CH=NOH$, CHO, $CH_2OCH_3$, $CH_2OH$, Cl, CN, $COCF_3$, $COC_1$-$C_3$-Alkyl, $CONH_2$, $CONHC_1$-$C_3$-Alkyl, $CON(C_1$-$C_3$-Alkyl$)_2$, $COOC_1$-$C_3$-Alkyl, COOH, F, I, $NH_2$, $NHC_1$-$C_3$-Alkyl, $N(C_1$-$C_3$-Alkyl$)_2$, NHCHO, $NHCOCH_3$, $NHCONH_2$, $NHSO_2CH_3$, $NO_2$, $OC_1$-$C_3$-Alkyl, $C(O)OCH_3$, OH, $SC_1$-$C_3$-Alkyl, $SOC_1$-$C_3$-Alkyl, $SO_2C_1$-$C_3$-Alkyl, $SO_2NH_2$, $SO_2NHC_1$-$C_3$-Alkyl, $SO_2N(C_1C_3$-Alkyl$)_2$ und $SO_3H$, oder worin Substituenten an $X_4$ und $X_5$ einen kondensierten Ring bilden, steht; worin $R_1$ ein Wasserstoffatom ist oder $R_1$ eine $C_1$ bis $C_4$ gesättigte, ungesättigte, acyclische, cyclische oder gemischte Hydrocarbyl- oder modifizierte Hydrocarbylgruppe ist und worin in der modifizierten Hydrocarbylgruppe 1 bis 2 Kohlenstoffatome durch ein bis zwei gleiche oder verschiedene Heteroatome, ausgewählt aus der aus N, O, S, Cl, Br und I bestehenden Gruppe, ersetzt sein können und 1 bis 3 Wasserstoffatome durch 1 bis 3 Fluor-, Sauerstoff- oder Stickstoffatome ersetzt sein können; worin $R_2$ Wasserstoff, CN, $NO_2$ oder eine $C_1$ bis $C_{20}$ gesättigte, ungesättigte, acyclische, cyclische oder gemischte Hydrocarbyl- oder modifizierte Hydrocarbylgruppe ist und worin in der modifizierten Hydrocarbylgruppe 1 bis 4 Kohlenstoffatome durch 1 bis 4 gleiche oder verschiedene Heteroatome, ausgewählt aus der aus N, O, S, Cl, Br und I bestehenden Gruppe, ersetzt sein können und 1 bis 5 Wasserstoffatome durch 1 bis 5 Fluor-, Sauerstoff- oder Stickstoffatome ersetzt sein können; n gleich 0, 1, 2 oder 3 ist; worin $R_1$ und $R_2$ kondensiert sein können; worin $R_4$ H oder $C_1$-$C_6$-Alkyl ist, mit der Maßgabe, daß $R_4$ nur Alkyl an einem einzigen Kohlenstoffatom sein kann, wenn $n=2$ oder 3 ist; und Tautomere und physiologisch verträgliche Salze davon.

**2.** Verbindung nach Anspruch 1, worin $R_1$ H ist.

**3.** Verbindung nach Anspruch 1, worin $R_3$ wahlweise substituiertes Phenyl und $R_1$ H ist.

**4.** Verbindung nach Anspruch 3, worin $R_4$ H und n gleich 1 ist.

**5.** Verbindung nach Anspruch 1, 2, 3 oder 4, worin $X_3$ und $X_5$ aus der aus H, Br, $CF_3$, $CH_3$, Cl und F bestehenden Gruppe ausgewählt werden.

**6.** Verbindung nach Anspruch 1, 2, 3 oder 4, worin $X_4$ aus der aus H, CN, $C(O)OCH_3$, F und $NO_2$ bestehenden Gruppe ausgewählt wird.

**7.** Verbindung nach Anspruch 1, 2, 3 oder 4, worin $R_2$ aus der aus normalem Alk(en)(in)yl $C_2$-$C_{13}$, verzweigtem Alk(en)(in)yl $C_3$-$C_{13}$, Cycloalk(en)yl $C_3$-$C_{13}$, Alk(en)yl-cycloalk(en)yl $C_4$-$C_{13}$, Cycloalk(en)-yl-alk(en)yl $C_4$-$C_{13}$, Alk(en)yl-cycloalk(en)yl-alk(en)yl $C_5$-$C_{13}$, Alk(en)yl-bicycloalk(en)yl $C_7$-$C_{13}$, kondensiertem Bicycloalk(en)yl $C_7$-$C_{13}$, Alk(en)yl-kondensiertem Bicycloalk(en)yl $C_8$-$C_{13}$, kondensiertem Bicycloalk(en)yl-alk(en)yl $C_8$-$C_{13}$, Alkenyl-kondensiertem Bicycloalk(en)yl-alk(en)yl $C_9$-$C_{13}$, kondensiertem Tricycloalk(en)yl $C_{10}$-$C_{13}$, Alk(en)yl-kondensiertem Tricycloalk(en)yl $C_{11}$-$C_{13}$, kondensiertem Tricycloalk(en)yl-alk(en)yl $C_{11}$-$C_{13}$ und Alk(en)yl-kondensiertem Tricycloalk(en)yl-alk(en)yl $C_{11}$-$C_{13}$ bestehenden Gruppe ausgewählt wird.

**8.** Verbindung nach Anspruch 7, worin $R_2$ aus der aus n-$C_5H_{11}$, n-$C_6H_{13}$, n-$C_7H_{15}$, $CH(CH_3)(CH_2)_2CH_3$, $CH(CH_3)(CH_2)_3CH_3$, $(CH_2)_3CH(CH_3)_2$, $(CH_2)_4CH(CH_3)_2$, $(CH_2)_5CH(CH_3)_2$, $C_6H_5$, $C_6$-$C_{10}$-Cycloalkyl, $(CH_3CH_2CH_2CH_2)C_6$-$C_{10}$-Cycloalkyl, $CH_2C_6H_5$, $CH_2$-c-$C_6H_{11}$, $CH(CH_3)C_6H_5$, $CH(C_6H_5)C_6H_5$, $CH(CH_3)$-c-$C_6H_{11}$, $(CH_2)_2C_6H_5$, $(CH_2)_2$-c-$C_6H_{11}$, $CH(CH_3)CH_2C_6H_5$, $CH(CH_3)CH_2$-c-$C_6H_{11}$, $C_6H_4(CH_3)$, $C_6$-$C_{10}$-Cycloalkyl$(CH_3)$, $CH_2C_6H_4(CH_3)$, $CH_2$-c-$C_6H_{10}(CH_3)$, $CH(CH_3)C_6H_4(CH_3)$, $CH(CH_3)$-c-$C_6H_{10}(CH_3)$, $(CH_2)_2CH(c$-$C_3H_5)_2$, $(CH_2)_3CH(c$-$C_3H_5)_2$, $CH(CH_3)CH_2CH(c$-$C_3H_5)_2$, $CH(CH_3)(CH_2)_2CH(c$-$C_3H_5)_2$, Naphthyl, 5,6,7,8-Tetrahydronaphthyl, Perhydronaphthyl, Indenyl, Indanyl, Naphthyl$(CH_3)$, 5,6,7,8-Tetrahydronaphthyl$(CH_3)$, Perhydronaphthyl$(CH_3)$, Indenyl$(CH_3)$, Indanyl$(CH_3)$, Fenchyl, $CH_2$-Naphthyl,

$CH_2$-5,6,7,8-Tetrahydronaphthyl, $CH_2$-Perhydronaphthyl,$CH_2$-Indenyl, $CH_2$-Indanyl, $CH_2$-Naphthyl($CH_3$), $CH_2$-5,6,7,8-Tetrahydronaphthyl($CH_3$), $CH_2$-Perhydronaphthyl($CH_3$), $CH_2$-Indenyl($CH_3$), $CH_2$-Indanyl-($CH_3$), Adamantyl, endo-Norbornyl, exo-Norbornyl, $CH_2$-Adamantyl, $CH(CH_3)$Adamantyl, Tetralyl, Decalyl und $CH_2$-Adamantyl($CH_3$) bestehenden Gruppe ausgewählt wird.

**9.** Verbindung nach Anspruch 1, 2, 3 oder 4, worin $R_2$ eine modifizierte Hydrocarbylgruppe ist, worin bis zu vier Kohlenstoffatome durch gleiche oder verschiedene Heteroatome ersetzt sein können, welche ausgewählt werden aus der Gruppe bestehend aus S anstelle von C oder $CH_2$, N anstelle von CH oder $CH_3$, NH und O anstelle von $CH_2$ und Cl, Br und I anstelle von $CH_3$, worin bis zu 5 Wasserstoffatome durch Fluoratome ersetzt werden können.

**10.** Verbindung nach Anspruch 9, worin $R_2$ aus der aus Pyridinyl, Pyridinylmethyl, Piperidyl, Homopiperidyl, Indolyl, Indolinyl, Isoindolinyl, Chinolyl, Isochinolyl, Pyrazinyl, Pyrimidyl, Indazolyl, Chinoxalinyl, Chinazolinyl, Purinyl, Pyranyl, Tetrahydropyranyl, Benzofuranyl, Methoxyphenyl, Methyloxycarbonylphenyl, 3,4-Methylendioxyphenyl, 6-Oxo-cis-hydrindan, Morpholinyl, Benzoxazolyl, Acetamidophenyl, Cyano, Nitro, Thienyl, Thienylmethyl, Benzothienyl, Tetrahydro-3-thienyl-1,1-dioxid, 2,2,4,4-Tetramethylthiacyclobut-3-yl, Thiazolyl, Isothiazolyl, $SO_2C_6H_5$, $SO_2c\text{-}C_6H_{11}$, $SO_2c\text{-}C_7H_{13}$, Chlorphenyl, Fluorphenyl und Trifluormethylphenyl bestehenden Gruppe ausgewählt wird.

**11.** Verbindung nach Anspruch 1 mit der Formel

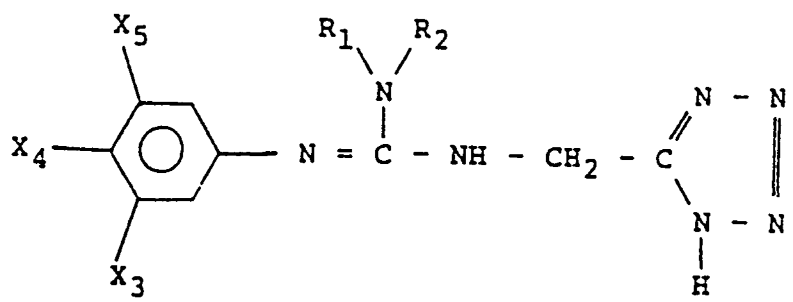

**12.** Verbindung nach Anspruch 11, worin $R_1$ $CH_3$ ist.

**13.** Verbindung nach Anspruch 11, worin $X_3$ und $X_5$ H sind und $X_4$ CN ist.

**14.** Verbindung nach Anspruch 11, worin $X_3$ und $X_5$ aus der aus $CF_3$, $CH_3$, Cl und F bestehenden Gruppe ausgewählt werden und worin $X_4$ aus der aus H und CN bestehenden Gruppe ausgewählt wird.

**15.** Verbindung nach Anspruch 11, worin $R_2$ aus der aus (S)$CH(CH_3)C_6H_5$, $CH_2C_6H_5$, $CH(CH_3)\text{-}c\text{-}C_6H_{11}$, $CH(C_6H_5)_2$, $c\text{-}C_6H_{11}$, $c\text{-}C_7H_{13}$, $c\text{-}C_8H_{15}$, $c\text{-}C_9H_{17}$, $c\text{-}C_{10}H_{19}$, 2-Decalyl, endo-Norbornyl, 1-Naphthyl, $SO_2C_6H_5$ und $SO_2c\text{-}C_7H_{13}$ bestehenden Gruppe ausgewählt wird.

**16.** Verbindung nach Anspruch 11, worin $R_1$ aus der aus H und $CH_3$ bestehenden Gruppe ausgewählt wird, worin $X_3$ und $X_5$ H sind und $X_4$ CN ist, oder worin $X_3$ und $X_5$ aus der aus $CF_3$, $CH_3$, Cl, H und F bestehenden Gruppe ausgewählt werden und worin $X_4$ aus der aus H und CN bestehenden Gruppe ausgewählt wird; und worin $R_2$ aus der aus (S)$CH(CH_3)C_6H_5$, $CH_2C_6H_5$, endo-Norbornyl, 1-Naphthyl, $CH(CH_3)\text{-}c\text{-}C_6H_{11}$, $CH(C_6H_5)_2$, $c\text{-}C_6H_{11}$, $c\text{-}C_7H_{13}$, $c\text{-}C_8H_{15}$, $c\text{-}C_9H_{17}$, $c\text{-}C_{10}H_{19}$, 2-Decalyl, $SO_2C_6H_5$ und $SO_2c\text{-}C_7H_{13}$ bestehenden Gruppe ausgewählt wird.

**17.** Verbindung nach Anspruch 16, worin $R_1$, $X_3$ und $X_5$ H sind, $X_4$ CN ist und $R_2$ $c\text{-}C_9H_{17}$ ist, einschließlich tautomerer Formen davon und physiologisch verträglicher Salze davon.

**18.** Verbindung nach Anspruch 16, worin $R_1$, $X_3$ und $X_5$ H sind, $X_4$ CN ist und $R_2$ $c\text{-}C_8H_{15}$ ist, einschließlich tautomerer Formen davon und physiologisch verträglicher Salze davon.

**19.** Verbindung nach Anspruch 16, worin $R_1$ und $X_4$ H sind, $X_3$ und $X_5$ Cl sind und $R_2$ (S)$CH(CH_3)C_6H_5$ ist, einschließlich tautomerer Formen davon und physiologisch verträglicher Salze davon.

**20.** Verbindung nach Anspruch 16, worin $R_1$ und $X_5$ H sind, $X_3$ Cl ist, $X_4$ CN ist und $R_2$ c-$C_8H_{15}$ ist, einschließlich tautomerer Formen davon und physiologisch verträglicher Salze davon.

**21.** Verbindung nach Anspruch 16, worin $R_1$ und $X_5$ H sind, $X_3$ $CH_3$ ist, $X_4$ CN und $R_2$ c-$C_8H_{15}$ ist, einschließlich tautomerer Formen davon und physiologisch verträglicher Salze davon.

**22.** Verbindung nach Anspruch 16, worin $R_1$ und $X_4$ H sind, $X_3$ und $X_5$ Cl sind und $R_2$ $CH_2C_6H_5$ ist, einschließlich tautomerer Formen davon und physiologisch verträglicher Salze davon.

**23.** Verbindung nach Anspruch 16, worin $R_1$ $CH_3$ ist, $X_4$ H ist, $X_3$ und $X_5$ Cl sind und $R_2$ $CH_2C_6H_5$ ist, einschließlich tautomerer Formen davon und physiologisch verträglicher Salze davon.

**24.** Verbindung nach Anspruch 16, worin $R_1$ und $X_4$ H sind, $X_3$ und $X_5$ Cl sind und $R_2$ (S)$CH(CH_3)$-c-$C_6H_{11}$ ist, einschließlich tautomerer Formen davon und physiologisch verträgliche Salze davon.

**25.** Verbindung nach Anspruch 16, worin $R_1$, $X_3$ und $X_5$ H sind, $X_4$ CN ist und $R_2$ (S)$CH(CH_3)C_6H_5$ ist, einschließlich tautomerer Formen davon und physiologisch verträglicher Salze davon.

**26.** Verbindung nach Anspruch 16, worin $R_1$, $X_3$ und $X_5$ H sind, $X_4$ CN ist und $R_2$ $CH(C_6H_5)_2$ ist, einschließlich tautomerer Formen davon und physiologisch verträglicher Salze davon.

**27.** Verbindung nach Anspruch 16, worin $R_1$ und $X_4$ H sind, $X_3$ und $X_5$ Cl sind und $R_2$ c-$C_7H_{13}$ ist, einschließlich tautomerer Formen davon und physiologisch verträglicher Salze davon.

**28.** Verbindung nach Anspruch 16, worin $X_4$ CN ist, $R_1$, $X_3$ und $X_5$ H sind und $R_2$ c-$C_{10}H_{17}$ ist, einschließlich tautomerer Formen davon und physiologisch verträglicher Salze davon.

**29.** Verbindung nach Anspruch 1, worin die Verbindung aus der Gruppe der physiologisch verträglichen Salze, bestehend aus Hydrochlorid, Phosphat, Citrat, Sulfat, Natrium-, Kalium-, Ammonium-, Calcium- und Magnesiumsalzen, ausgewählt wird.

**30.** Verfahren zum Süßen eßbarer Produkte, darunter Nahrungsmittel, Getränke, Konditorwaren, Kaugummi, Pharmazeutika, Veterinärzubereitungen und Toiletten-, Kosmetik- und Hygieneprodukte, dadurch gekennzeichnet, daß es das Zusetzen einer wirksamen süßenden Menge einer Verbindung nach Anspruch 1 zu der Substanz umfaßt.

**31.** Eßbare Produkte, gesüßt nach dem Verfahren von Anspruch 30.

**32.** Süßstoffzusammensetzungen, dadurch gekennzeichnet, daß sie eine wirksame süßende Menge einer Verbindung nach Anspruch 1 und einen physiologisch verträglichen Träger dafür umfassen.

**33.** Süßstoffzusammensetzungen nach Anspruch 32, worin der Träger ein Füllstoff ist.

**34.** Süßstoffzusammensetzungen nach Anspruch 32, worin der Träger aus der aus Polydextrose, Stärke, Maltodextrinen, Cellulose, Methylcellulose, Carboxymethylcellulose, Maltit, Hydroxymethylcellulose, mikrokristalliner Cellulose, Natriumalginat, Pektinen, Gummen, Lactose, Maltose, Glucose, Leucin, Glycerin, Mannit, Sorbit, Natriumbicarbonat und Phosphor-, Citronen-, Wein-, Fumar-, Benzoe-, Sorbin-, Propionsäure und deren Natrium-, Kalium- und Calciumsalzen, und deren Mischungen bestehenden Gruppe ausgewählt wird.

**35.** Süßstoffzusammensetzung, umfassend (a) eine Verbindung nach Anspruch 1 und (b) ein anderes Süßmittel.

**36.** Süßstoffzusammensetzung nach Anspruch 35, die weiters einen Füllstoff umfaßt.

**37.** Süßstoffzusammensetzung nach Anspruch 36, worin das andere Süßmittel aus der Saccharose, Maissirupe, Fructose, Aspartam, Alitam, Neohesperidin-dihydrochalcon, Glucose-Fructose-Maissirup, hydrierte Isomaltulose, Steviosid, L-Zucker, Glycyrrhizin, Xylit, Acesulfam-K, Saccharin (Natrium-, Kalium- oder Calciumsalz), Cyclaminsäure (Natrium-, Kalium- oder Calciumsalz), Trichlorgalactosaccharose, Monellin und Thaumatin und deren Gemische umfassenden Gruppe ausgewählt wird.

**38.** Verbindung der Formel

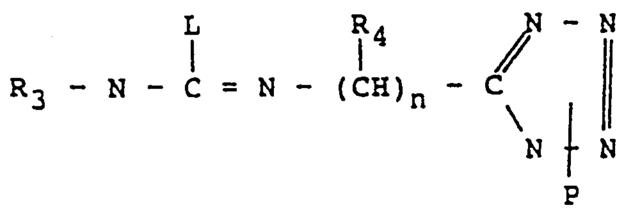

worin L S-Alkyl, O-Alkyl, $OSO_2$-Aryl, $SO_3H$ oder Halogen ist, P eine Schutzgruppe darstellt und $R_3$, $R_4$ und n wie im Anspruch 1 definiert sind.

**39.** Verbindung nach Anspruch 38, worin L S-Methyl ist.

**40.** Verbindung nach Anspruch 38, worin $R_3$ 4-Cyanophenyl, 3,5-Dichlorphenyl, 3,5-Dimethylphenyl, 6-Indazolylimino oder Phenyl ist.

**41.** Verbindung nach Anspruch 38, worin P H,

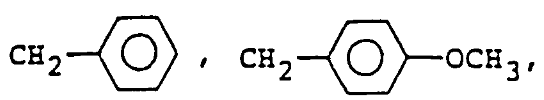

$CH_2OCO$-t-$C_4H_9$ oder $CH_2CH_2COOC_2H_5$ ist.

**42.** Verbindung nach Anspruch 40, worin $R_4$ H ist.

**43.** Verbindung nach Anspruch 42, worin n 1 ist.

**44.** Verbindung nach Anspruch 42, worin n 0 ist.

**45.** Verbindung der Formel

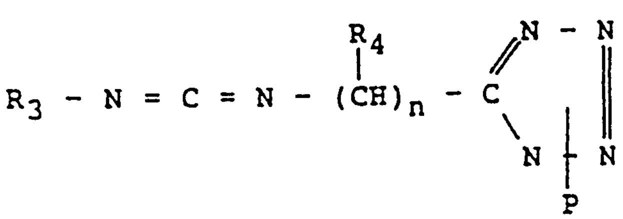

worin P eine Schutzgruppe darstellt und $R_3$, $R_4$ und n wie im Anspruch 1 definiert sind.

**46.** Verbindung nach Anspruch 45, worin $R_4$ H ist.

**47.** Verbindung nach Anspruch 46, worin $R_3$ substituiertes Phenyl ist.

**48.** Verbindung nach Anspruch 47, worin $R_3$ 4-Cyanophenyl, 3,5-Dichlorphenyl, 3,5-Dimethylphenyl oder Phenyl ist.

**49.** Verbindung nach Anspruch 45, worin P H,

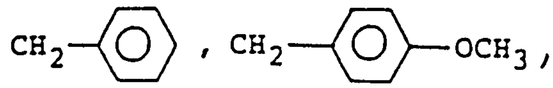

$CH_2OCO$-t-$C_4H_9$ oder $CH_2CH_2COOC_2H_5$ ist.

**50.** Verbindung nach Anspruch 45, worin n 0 ist.

**51.** Verbindung nach Anspruch 45, worin n 1 ist.

**52.** Verbindung nach Anspruch 48, worin $R_4$ und P H sind und n 1 ist.

**53.** Verfahren umfassend (a) die Umsetzung einer Verbindung der Formel

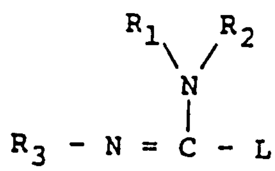

mit einer Verbindung der Formel

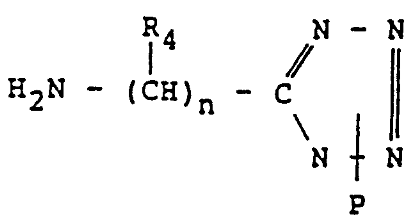

worin L S-Alkyl, O-Alkyl, $OSO_2$-Aryl, $SO_3H$ oder Halogen ist und P für eine Schutzgruppe steht und $R_1$, $R_2$, $R_3$, $R_4$ und n wie im Anspruch 1 definiert sind, unter Bedingungen, die ausreichend sind, um das entsprechende Guanidin herzustellen, und (b) Gewinnung der unter (a) gebildeten Guanidinverbindung.

**54.** Verfahren nach Anspruch 53, worin P H,

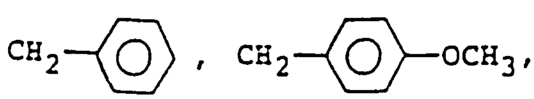

$CH_2OCO$-t-$C_4H_9$ oder $CH_2CH_2COOC_2H_5$ ist.

**55.** Verfahren nach Anspruch 53, worin L für $-S-CH_3$ steht.

**56.** Verfahren nach Anspruch 53, worin $R_3$ substituiertes Phenyl ist und $R_1$ und $R_4$ H sind.

**57.** Verfahren nach Anspruch 56, worin $R_2$ Cyclohexyl, Cyclooctyl, alpha-Phenethyl, Diphenylmethyl, Benzyl, Phenyl, endo-Norbornyl, 1-Naphthyl oder 2-Decalyl ist.

**58.** Verfahren umfassend (a) die Umsetzung eines Carbodiimids der Formel

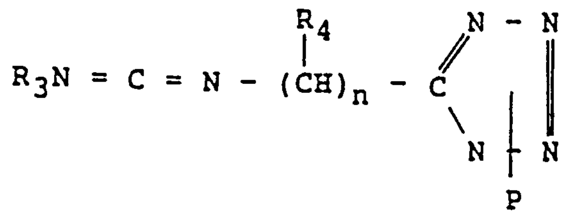

mit einem Amin der Formel $HNR_1R_2$, worin P eine Schutzgruppe ist, $R_1$, $R_2$, $R_3$, $R_4$ und n wie im Anspruch 1 definiert sind, unter Bedingungen, die ausreichend sind, um das entsprechende Guanidin zu bilden, und (b) Gewinnung der unter (a) gebildeten Guanidinverbindung.

**59.** Verfahren nach Anspruch 58, worin P H,

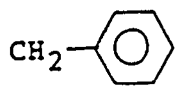 , 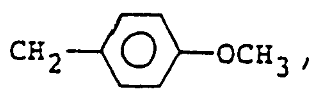 ,

$CH_2OCO\text{-}t\text{-}C_4H_9$ oder $CH_2CH_2COOC_2H_5$ ist.

**60.** Verfahren nach Anspruch 58, worin $R_1$ und $R_4$ H sind.

**61.** Verfahren nach Anspruch 60, worin $R_3$ substituiertes Phenyl ist.

**62.** Verfahren nach Anspruch 61, worin $R_3$ 4-Cyanophenyl, 3,5-Dichlorphenyl, 3,5-Dimethylphenyl oder Phenyl ist.

**63.** Verfahren nach Anspruch 60, worin $R_2$ Cyclohexyl, Cyclooctyl, alpha-Phenethyl, Diphenylmethyl, Benzyl, Phenyl, endo-Norbornyl, 1-Naphthyl oder 2-Decalyl ist.

**64.** Verfahren nach Anspruch 63, worin $R_3$ substituiertes Phenyl ist.

**65.** Verfahren nach Anspruch 64, worin $R_3$ 4-Cyanophenyl, 3,5-Dichlorphenyl, 3,5-Dimethylphenyl oder Phenyl ist.

**66.** Verfahren umfassend (a) die Umsetzung einer Carbodiimidverbindung der Formel $R_3\text{-}N=C=N\text{-}R_2$ mit einem Amin der Formel

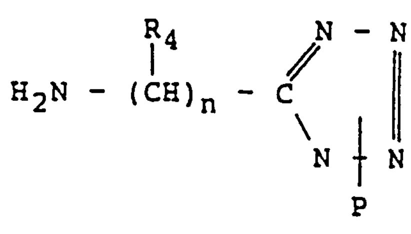

worin P eine Schutzgruppe ist, $R_2$, $R_3$, $R_4$ und n wie im Anspruch 1 definiert sind, unter Bedingungen, die ausreichend sind, um die entsprechende Guanidinverbindung zu bilden, und (b) Gewinnung der unter (a) gebildeten Guanidinverbindung.

**67.** Verfahren nach Anspruch 66, worin P H,

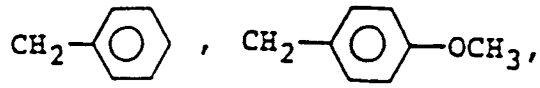

$CH_2OCO$-t-$C_4H_9$ oder $CH_2CH_2COOC_2H_5$ ist.

**68.** Verfahren nach Anspruch 66, worin $R_4$ und P H sind.

**69.** Verfahren nach Anspruch 68, worin $R_3$ substituiertes Phenyl ist.

**70.** Verfahren nach Anspruch 69, worin $R_3$ 4-Cyanophenyl, 3,5-Dichlorphenyl, 3,5-Dimethylphenyl ist.

**71.** Verfahren nach Anspruch 68, worin $R_2$ Phenyl, Benzyl, Cyclohexyl, Cyclooctyl, endo-Norbornyl, 1-Naphthyl, 2-Decalyl, alpha-Phenethyl oder Diphenylmethyl ist.

**72.** Verfahren nach Anspruch 71, worin $R_3$ substituiertes Phenyl ist.

**73.** Verfahren nach Anspruch 72, worin $R_3$ 4-Cyanophenyl, 3,5-Dichlorphenyl, 3,5-Dimethylphenyl oder Phenyl ist.

**74.** Verbindung nach Anspruch 1 mit der Formel

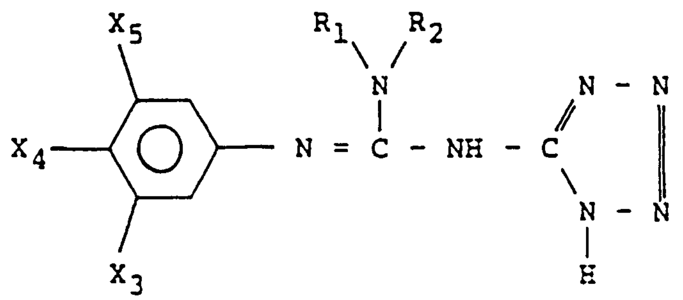

**75.** Verbindung nach Anspruch 74, worin $R_1$ H ist.

**76.** Verbindung nach Anspruch 75, worin $X_3$ und $X_5$ H sind und $X_4$ CN ist.

**77.** Verbindung nach Anspruch 76, worin $R_2$ Phenyl, Benzyl, Cyclohexyl, Cyclooctyl, Cyclononyl, 4-Pyranyl, 2-Decalyl, endo-Norbornyl, 1-Naphthyl, alpha-Phenethyl oder Benzhydryl ist.

**78.** Verbindung nach Anspruch 75, worin $X_4$ H ist und $X_3$ und $X_5$ Cl oder $CH_3$ sind.

**79.** Verbindung nach Anspruch 78, worin $R_2$ Phenyl, Benzyl, Cyclohexyl, Cyclooctyl, Cyclononyl, 4-Pyranyl, 2-Decalyl, endo-Norbornyl, 1-Naphthyl, alpha-Phenethyl oder Benzhydryl ist.

**Patentansprüche für folgenden Vertragsstaat : ES**

1. Verfahren zur Herstellung einer Verbindung der Formel

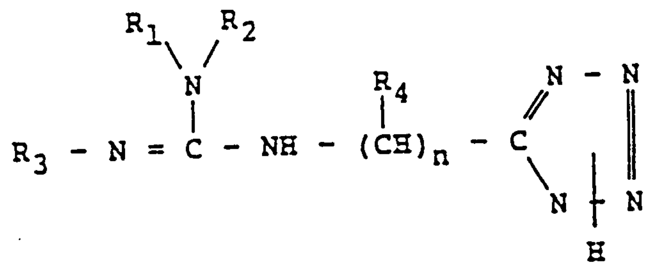

worin $R_3$ für wahlweise substituierte Pyridine, Thiazole, Indole, Chinoline, Naphthyridine, Cinnoline, Pteridine, Thiophene, Benzothiophene, Naphthothiophene, Thianthrene, Furane, Pyrane, Isobenzofurane, Chromene, Xanthene, Phenoxathine, Pyrrole, Indole, Isoindole, Indolizine, Pyridazine, Pyrimidine, Pyrazine, Pyrazole, Imidazole, Pyrrole, Indazole, Purine, Chinolizine, Isochinoline, Chinoline, Phthalazine, Chinoxaline, Chinazoline, Carbazole, Carboline, Phenanthridine, Acridine, Pyrimidine, Phenanthroline, Phenazine, Phenarsazine, Isothiazole, Phenothiazine, Isoxazole, Thiazole, Furazane und Heterocyclen der folgenden Formeln

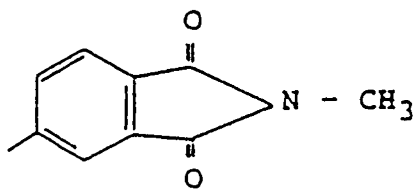

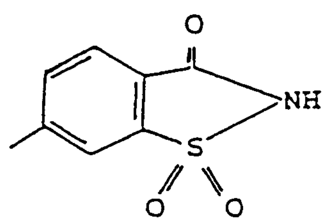

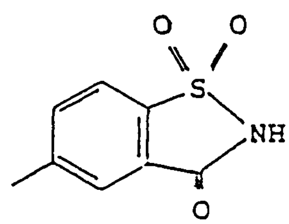

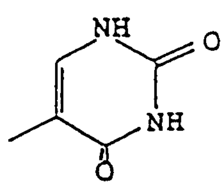

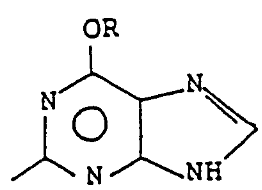

worin R H oder $C_1$-$C_6$-Alkyl ist und worin heterocyclische Einheiten wahlweise substituiert sein können mit einem oder mehreren Substituenten, ausgewählt aus $C_1$-$C_6$-Alkyl, Halogen, $NO_2$, CN, Trihalogenmethyl, Dihalogenmethyl, Hydroxyl oder Hydroxyalkyl, oder für wahlweise substituiertes Phenyl der Formel

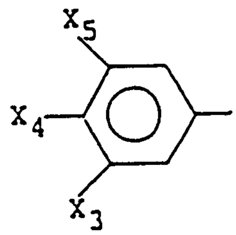

worin $X_3$, $X_4$ und $X_5$ gleich oder verschieden sind und ausgewählt werden aus der Gruppe bestehend aus H, Br, $CF_3$, $CF_2CF_3$, $CH_2CF_3$, $C_1$-$C_4$-Alkyl, $CH=NOCH_3$, $CH=NOH$, CHO, $CH_2OCH_3$, $CH_2OH$, Cl, CN, $COCF_3$, $COC_1$-$C_3$-Alkyl, $CONH_2$, $CONHC_1$-$C_3$-Alkyl, $CON(C_1$-$C_3$-Alkyl$)_2$, $COOC_1$-$C_3$-Alkyl, COOH, F, I, $NH_2$, $NHC_1$-$C_3$-Alkyl, $N(C_1$-$C_3$-Alkyl$)_2$, NHCHO, $NHCOCH_3$, $NHCONH_2$, $NHSO_2CH_3$, $NO_2$, $OC_1$-$C_3$-Alkyl, $C(O)OCH_3$, OH, $SC_1$-$C_3$-Alkyl, $SOC_1$-$C_3$-Alkyl, $SO_2C_1$-$C_3$-Alkyl, $SO_2NH_2$, $SO_2NHC_1$-$C_3$-Alkyl, $SO_2N(C_1$-$C_3$-Alkyl$)_2$ und $SO_3H$, oder worin Substituenten an $X_4$ und $X_5$ einen kondensierten Ring bilden, steht; worin $R_1$ ein Wasserstoffatom ist oder $R_1$ eine $C_1$ bis $C_4$ gesättigte, ungesättigte, acyclische, cyclische oder gemischte Hydrocarbyl- oder modifizierte Hydrocarbylgruppe ist und worin in der modifizierten Hydrocarbylgruppe 1 bis 2 Kohlenstoffatome durch ein bis zwei gleiche oder verschiedene Heteroatome, ausgewählt aus der aus N, O, S, Cl, Br und I bestehenden Gruppe, ersetzt sein können und 1 bis 3 Wasserstoffatome durch 1 bis 3 Fluor-, Sauerstoff- oder Stickstoffatome ersetzt sein können; worin $R_2$ Wasserstoff, CN, $NO_2$ oder eine $C_1$ bis $C_{20}$ gesättigte, ungesättigte, acyclische, cyclische oder gemischte Hydrocarbyl- oder modifizierte Hydrocarbylgruppe ist und worin in der modifizierten Hydrocarbylgruppe 1 bis 4 Kohlenstoffatome durch 1 bis 4 gleiche oder verschiedene Heteroatome, ausgewählt aus der aus N, O, S, Cl, Br und I bestehenden Gruppe, ersetzt sein können und 1 bis 5 Wasserstoffatome durch 1 bis 5 Fluor-, Sauerstoff- oder Stickstoffatome ersetzt sein können; n gleich 0, 1, 2 oder 3 ist; worin $R_1$ und $R_2$ kondensiert sein können; worin $R_4$ H oder $C_1$-$C_6$-Alkyl ist, mit der Maßgabe, daß $R_4$ nur Alkyl an einem einzigen Kohlenstoffatom sein kann, wenn n = 2 oder 3 ist; und Tautomere und physiologisch verträglicher Salze davon, gekennzeichnet durch (a) die Umsetzung einer Verbindung der Formel

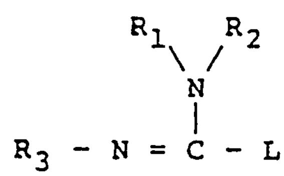

mit einer Verbindung der Formel

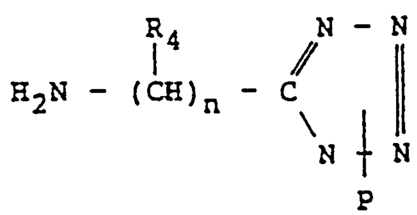

worin L S-Alkyl, O-Alkyl, $OSO_2$-Aryl, $SO_3H$ oder Halogen ist und P für eine Schutzgruppe steht und $R_1$, $R_2$, $R_3$, $R_4$ und n wie im Anspruch 1 definiert sind, unter Bedingungen, die ausreichend sind, um das entsprechende Guanidin herzustellen, und (b) Gewinnung der unter (a) gebildeten Guanidinverbindung sowie wahlweise Überführen der erhaltenen Verbindung in bekannter Weise in die jeweiligen physiologisch verträglichen Salze davon.

**2.** Verfahren zur Herstellung einer Verbindung nach Anspruch 1, gekennzeichnet durch (a) die Umsetzung einer Carbodiimidverbindung der Formel $R_3$-N=C=N-$R_2$ mit einem Amin der Formel

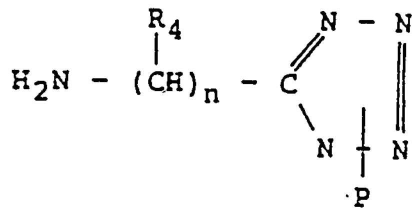

worin P eine Schutzgruppe ist, $R_2$, $R_3$, $R_4$ und n wie im Anspruch 1 definiert sind, unter Bedingungen, die ausreichend sind, um die entsprechende Guanidinverbindung zu bilden, und (b) Gewinnung der unter (a) gebildeten Guanidinverbindung sowie wahlweise Überführen der erhaltenen Verbindung in bekannter Weise in die jeweiligen physiologisch verträglichen Salze davon.

**3.** Verfahren zur Herstellung einer Verbindung nach Anspruch 1, gekennzeichnet durch (a) die Umsetzung eines Carbodiimids der Formel

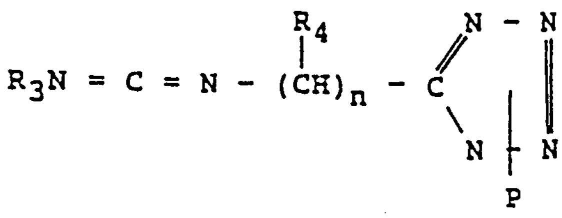

mit einem Amin der Formel $HNR_1R_2$, worin P eine Schutzgruppe ist, $R_1$, $R_2$, $R_3$, $R_4$ und n wie im Anspruch 1 definiert sind, unter Bedingungen, die ausreichend sind, um das entsprechende Guanidin zu bilden, und (b) Gewinnung der unter (a) gebildeten Guanidinverbindung sowie wahlweise Überführen der erhaltenen Verbindung in bekannter Weise in die jeweiligen physiologisch verträglichen Salze davon.

**4.** Verfahren nach irgendeinem der Ansprüche 1 bis 3, dadurch gekennzeichnet, daß die physiologisch verträglichen Salze durch Überführen der erhaltenen Produkte in Hydrochlorid, Phosphat, Citrat, Sulfat, Natrium-, Kalium-, Ammonium-, Calcium- oder Magnesiumsalze hergestellt werden.

**5.** Verfahren zum Süßen eßbarer Produkte, darunter Nahrungsmittel, Getränke, Konditorwaren, Kaugummi, Pharmazeutika, Veterinärzubereitungen und Toiletten-, Kosmetik- und Hygieneprodukte, dadurch gekennzeichnet, daß es das Zusetzen einer wirksamen süßenden Menge einer nach Anspruch 1 hergestellten Verbindung zu der Substanz umfaßt.

**6.** Verwendung einer wirksamen süßenden Menge einer nach Anspruch 1 hergestellten Verbindung und eines physiologisch verträglichen Trägers zur Herstellung von Süßstoffzusammensetzungen.

**7.** Verwendung nach Anspruch 6, worin der Träger ein Füllstoff ist.

**8.** Verwendung nach Anspruch 6, worin der Träger aus der aus Polydextrose, Stärke, Maltodextrinen, Cellulose, Methylcellulose, Carboxymethylcellulose, Maltit, Hydroxymethylcellulose, mikrokristalliner Cellulose, Natriumalginat, Pektinen, Gummen, Lactose, Maltose, Glucose, Leucin, Glycerin, Mannit, Sorbit, Natriumbicarbonat und Phosphor-, Citronen-, Wein-, Fumar-, Benzoe-, Sorbin-, Propionsäure und deren Natrium-, Kalium- und Calciumsalzen, und deren Mischungen bestehenden Gruppe ausgewählt wird.

**9.** Verwendung (a) einer nach Anspruch 1 hergestellten Verbindung und (b) eines anderen Süßmittels zur Herstellung einer Süßstoffzusammensetzung.

**10.** Verwendung nach Anspruch 9, worin die Zusammensetzung weiters einen Füllstoff enthält.

**11.** Verwendung nach Anspruch 10, worin das andere Süßmittel aus der Saccharose, Maissirupe, Fructose, Aspartam, Alitam, Neohesperidin-dihydrochalcon, Glucose-Fructose-Maissirup, hydrierte Isomaltulose, Steviosid, L-Zucker, Glycyrrhizin, Xylit, Acesulfam-K, Saccharin (Natrium-, Kalium- oder Calciumsalz), Cyclaminsäure (Natrium-, Kalium- oder Calciumsalz), Trichlorgalactosaccharose, Monellin und Thaumatin und deren Gemische umfassenden Gruppe ausgewählt wird.

**Revendications**
**Revendications pour les Etats contractants suivants : AT, BE, CH, DE, FR, GB, GR, IT, LI, LU, NL, SE**

**1.** Composé répondant à la formule :

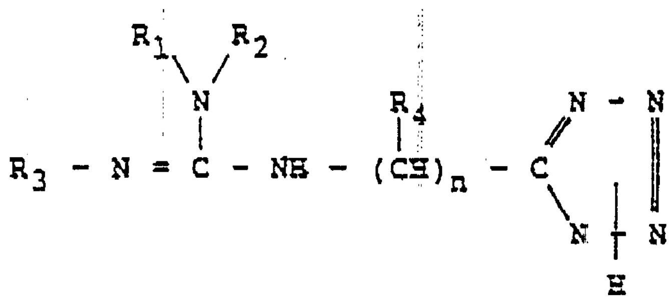

dans laquelle $R_3$ représente des pyridines, thiazoles, indoles, quinoléines, naphtyridines, cinnolines, ptéridines, thiophènes, benzothiophènes, naphtothiophènes, thianthrènes, furannes, pyrannes, isobenzofurannes, chromènes, xanthènes, phénoxathines, pyrroles, indoles, isoindoles, indolizines, pyridazines, pyrimidines, pyrazines, pyrazoles, imidazoles, pyrroles, indazoles, purines, quinolizines, isoquinoléines, quinoléines, phtalazines, quinoxalines, quinazolines, carbazoles, carbolines, phénanthridines, acridines, pyrimidines, phénanthrolines, phénazines, phénarsazines, isothiazoles, phénothiazines, isothiazoles, thiazoles, furazannes (portant éventuellement des substituants) et des hétérocycliques répondant aux formules suivantes :

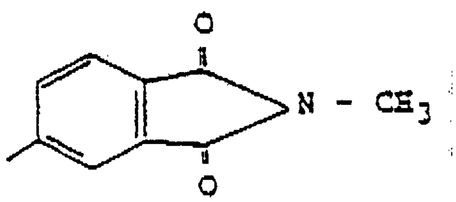

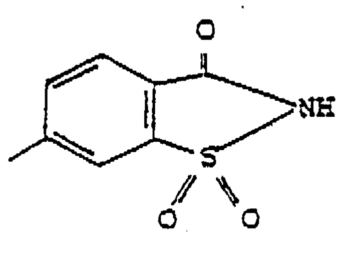

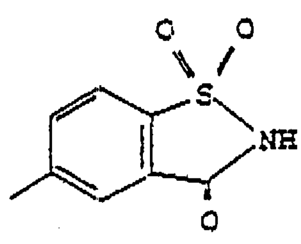

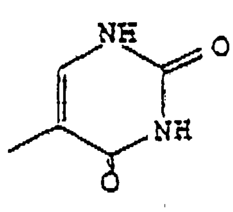

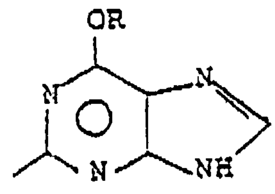

dans lesquels R représente H ou un groupe alkyle en $C_1$ à $C_6$, et les fragments hétérocycliques peuvent être éventuellement substitués par un ou plusieurs substituants choisis parmi un groupe alkyle en $C_1$ à $C_6$, un atome d'halogène, un groupe $NO_2$, CN, trihalogénométhyle, dihalogénométhyle, hydroxyle ou hydroxyalkyle, ou bien un groupe phényle éventuellement substitué répondant à la formule :

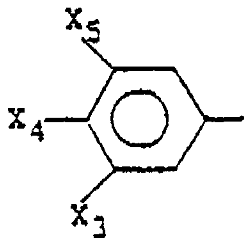

dans laquelle $X_3$, $X_4$ et $X_5$ sont identiques ou différents et sont choisis dans l'ensemble consistant en :

H,
Br,
$CF_3$,
$CF_2CF_3$,
$CH_2CF_3$,
Alkyle en $C_1$ à $C_4$,
$CH=NOCH_3$,
CH=NOH,
CHO,
$CH_2OCH_3$,
$CH_2OH$,
Cl,
CN,
$COCF_3$,
CO alkyle (en $C_1$ à $C_3$),
$CONH_2$,
CONH alkyle (en $C_1$ à $C_3$),
CON (chaque alkyle en $C_1$ à $C_3$),
COO alkyle (en $C_1$ à $C_3$),
COOH,
F,
I,
$NH_2$,
NH alkyle (en $C_1$ à $C_3$),
N $(alkyle)_2$, chaque alkyle de $C_1$ à $C_3$,
NHCHO,
$NHCOCH_3$,
$NHCONH_2$,
$NHSO_2CH_3$,
$NO_2$,
O alkyle (en $C_1$ à $C_3$),
$C(O)OCH_3$,
OH,
S alkyle (en $C_1$ à $C_3$),
SO alkyle (en $C_1$ à $C_3$),
$SO_2$ alkyle (en $C_1$ à $C_3$),
$SO_2NH_2$,
$SO_2NH$ alkyle en ($C_1$ à $C_3$),
$SO_2N$ $(alkyle)_2$, (chaque alkyle en $C_1$ à $C_3$),
$SO_3H$,

ou dans laquelle les substituants sur les positions représentées par $X_4$ et $X_5$ forment un noyau condensé ;

$R_1$ représente un atome d'hydrogène ou bien $R_1$ représente un groupe hydrocarbyle en $C_1$ à $C_4$, saturé, insaturé, acyclique, cyclique ou hydrocarbyle mixte ou hydrocarbyle modifié et, dans le groupe hydrocarbyle modifié,

1 à 2 atomes de carbone peuvent être remplacés par 1 à 2 hétéroatomes, identiques ou différents, choisis chacun dans l'ensemble consistant en N, O, S, Cl, Br et I,

et 1 à 3 atomes d'hydrogène peuvent être remplacés par 1 à 3 atomes de fluor, d'oxygène ou d'azote,

$R_2$ représente un atome d'hydrogène, un groupe CN, $NO_2$, ou un groupe hydrocarbyle en $C_1$ à $C_{20}$, saturé, insaturé, acyclique, cyclique ou mixte ou un groupe hydrocarbyle modifié et, dans le groupe hydrocarbyle modifié,

1 à 4 atomes de carbone peuvent être remplacés par 1 à 4 hétéroatomes, identiques ou différents, choisis chacun dans l'ensemble consistant en N, O, S, Cl, Br et I,

et 1 à 5 atomes d'hydrogène peuvent être remplacés par 1 à 5 atomes de fluor, d'oxygène ou d'azote,

n vaut 0, 1, 2 ou 3,

$R_1$ et $R_2$ peuvent être condensés, fusionnés,

$R_4$ représente H ou un groupe alkyle en $C_1$ à $C_6$, à la condition que $R_4$ ne puisse représenter un groupe alkyle sur un seul atome de carbone que lorsque n vaut 2 ou 3, et

leurs tautomères et sels physiologiquement acceptables.

**2.** Composé selon la revendication 1, dans lequel $R_1$ représente H.

**3.** Composé selon la revendication 1, dans lequel $R_3$ représente un groupe phényle éventuellement substitué, et $R_1$ représente H.

**4.** Composé selon la revendication 3, dans lequel $R_4$ représente H et n vaut 1.

**5.** Composé selon la revendication 1, 2, 3 ou 4, dans lequel $X_3$ et $X_5$ sont choisis dans l'ensemble consistant en :

H,
Br,
$CF_3$,
$CH_3$,
Cl et
F.

**6.** Composé selon la revendication 1, 2, 3 ou 4, dans lequel $X_4$ est choisi dans l'ensemble consistant en :

H,
CN,
$C(O)OCH_3$,
F et
$NO_2$.

**7.** Composé selon la revendication 1, 2, 3 ou 4, dans lequel $R_2$ est choisi dans l'ensemble consistant en un groupe :

alc(en)(yn)yle normal en $C_2$ à $C_{13}$,
alc(en)(yn)yle ramifié en $C_3$ à $C_{13}$,
cycloalc(en)yle en $C_3$ à $C_{13}$,
alc(en)yl cycloalc(en)yle en $C_4$ à $C_{13}$,
cycloalc(en)yl alc(en)yle en $C_4$ à $C_{13}$,
alc(en)yl cycloalc(en)yl alc(en)yle en $C_5$ à $C_{13}$,
alc(en)yl bicycloalc(en)yle en $C_7$ à $C_{13}$,
bicycloalc(en)yle condensé en $C_7$ à $C_{13}$,

alc(en)yl bicycloalc(en)yle condensé en $C_8$ à $C_{13}$,
bicycloalc(en)yl alc(en)yle condensé en $C_8$ à $C_{13}$,
alcényl bicycloalc(en)yle condensé alc(en)yle en $C_9$ à $C_{13}$,
tricycloalc(en)yle condensé en $C_{10}$ à $C_{13}$,
alc(en)yl tricycloalc(en)yle condensé en $C_{11}$ à $C_{13}$,
tricycloalc(en)yl alc(en)yle condensé en $C_{11}$ à $C_{13}$ et
alc(en)yl tricycloalc(en)yl condensé alc(en)yle en $C_{11}$ à $C_{13}$.

**8.** Composé selon la revendication 7, dans lequel $R_2$ est choisi dans l'ensemble consistant en :
n-$C_5H_{11}$, n-$C_6H_{13}$, n-$C_7H_{15}$,
$CH(CH_3)(CH_2)_2CH_3$, $CH(CH_3)(CH_2)_3CH_3$, $(CH_2)_3CH(CH_3)_2$, $(CH_2)_4CH(CH_3)_2$, $(CH_2)_5CH(CH_3)_2$,
$C_6H_5$, cycloakyle en $C_6$-$C_{10}$, $(CH_3CH_2CH_2CH_2)$ cycloalkyle en $C_6$-$C_{10}$,
$CH_2C_6H_5$, $CH_2$-c-$C_6H_{11}$, $CH(CH_3)C_6H_5$, $CH(C_6H_5)C_6H_5$, $CH(CH_3)$-c-$C_6H_{11}$, $(CH_2)_2C_6H_5$, $(CH_2)_2$-c-$C_6H_{11}$, $CH(CH_3)CH_2C_6H_5$, $CH(CH_3)CH_2$-c-$C_6H_{11}$,
$C_6H_4$ $(CH_3)$, $C_6$-$C_{10}$ cycloalkyl$(CH_3)$,
$CH_2C_6H_4(CH_3)$, $CH_2$-c-$C_6H_{10}(CH_3)$, $CH(CH_3)C_6H_4(CH_3)$, $CH(CH_3)$-c-$C_6H_{10}(CH_3)$,
$(CH_2)_2CH(c\text{-}C_3\text{-}H_5)_2$, $(CH_2)_3CH(c\text{-}C_3H_5)_2$, $CH(CH_3)CH_2CH(c\text{-}C_3H_5)_2$, $CH(CH_3)(CH_2)_2CH(c\text{-}C_3H_5)_2$,
naphtyle, 5,6,7,8-tétrahydronaphtyle, perhydronaphtyle, indényle, indanyle,
naphtyl$(CH_3)$, 5,6,7,8-tétrahydronapthyl$(CH_3)$, perhydronapthyl $(CH_3)$, indényl$(CH_3)$, indanyl$(CH_3)$, fenchyle,
$CH_2$-napthyle, $CH_2$-5,6,7,8-tétrahydronaphtyle, $CH_2$-perhydronapthyle, $CH_2$-indényle, $CH_2$-indanyle,
$CH_2$-napthyl$(CH_3)$, $CH_2$-5,6,7,8-tétrahydronapthyl$(CH_3)$, $CH_2$-perhydronapthyl$(CH_3)$, $CH_2$-indényl-$(CH_3)$, $CH_2$-indanyl$(CH_3)$,
adamantyle, endo-norbornyle, exo-norbornyle,
$CH_2$-adamantyle, $CH(CH_3)$adamantyle, tétralyle, décalyle et
$CH_2$-adamantyl$(CH_3)$.

**9.** Composé selon la revendication 1, 2, 3 ou 4, dans lequel $R_2$ représenteun groupe hydrocarbyle modifié dans lequel jusqu'à 4 atomes de carbone peut ou peuvent être remplacés par des hétéroatomes, identiques ou différents, choisis dans un ensemble consistant en :
S pour remplacer C ou $CH_2$,
N pour remplacer CH ou $CH_3$,
NH et O pour remplacer $CH_2$, et
Cl, Br et I pour remplacer $CH_3$,
et jusqu'à 5 atomes d'hydrogène peut ou peuvent être remplacés par des atomes de fluor.

**10.** Composé selon la revendications 9, dans lequel $R_2$ est choisi dans l'ensemble consistant en un groupe :
pyridinyle, pyridinylméthyle, pipéridyle, homopipéridyle, indolyle, indolinyle, isoindolinyle, quinolyle, isoquinolyle, pyrazinyle, pyrimidyle, indazolyle, quinoxalinyle, quinazolinyle, purinyle,
pyrannyle, tétrahydropyrannyle, benzofurannyle, méthoxyphényle, méthyloxycarbonylphényle, 3,4-méthylènedioxyphényle, 6-oxo-cis-hydrindane, morpholinyle, benzoxazolyle, acétamidophényle, cyano, nitro,
thiényle, thiénylméthyle, benzothiényle, 1,1-dioxyde de tétrahydro-3-thiényle, 2,2,4,4-tétraméthylthiocyclobut-3-yle, thiazolyle, isothiazolyle, $SO_2C_6H_5$, -$SO_2$c-$C_6H_{11}$, $SO_2$c-$C_7H_{13}$,
chlorophényle,
fluorophényle et
trifluorométhylphényle.

11. Composé selon la revendication 1, répondant à la formule :

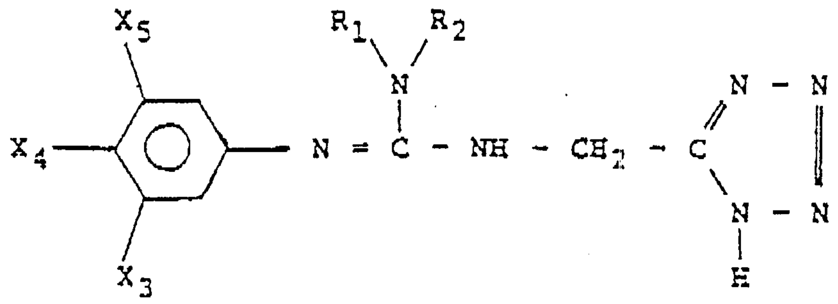

12. Composé selon la revendication 11, dans lequel $R_1$ représente $CH_3$.

13. Composé selon la revendication 11, dans lequel $X_3$ et $X_5$ représentent chacun H et $X_4$ représente CN.

14. Composé selon la revendication 11, dans lequel $X_3$ et $X_5$ sont choisis parmi $CF_3$, $CH_3$, Cl et F et $X_4$ est choisi parmi H et CN.

15. Composé selon la revendication 11, dans lequel $R_2$, est choisi dans l'ensemble consistant en un groupe $(S)CH(CH_3)C_6H_5$, $CH_2C_6H_5$, $CH(CH_3)$-c-$C_6H_{11}$, $CH(C_6H_5)_2$, c-$C_6H_{11}$, c-$C_7H_{13}$, c-$C_8H_{15}$, c-$C_9H_{17}$, c-$C_{10}H_{19}$, 2-décalyle, endo-norbornyle, 1-naphtyle, $SO_2C_6H_5$ et $SO_2$c-$C_7H_{13}$.

16. Composé selon la revendication 11, dans lequel $R_1$ est choisi dans l'ensemble consistant en H et $CH_3$ ; $X_3$ et $X_5$ représentent chacun H et $X_4$ représente CN, ou bien $X_3$ et $X_5$ sont choisis dans l'ensemble consistant en $CF_3$, $CH_3$, Cl, H et F, et $X_4$ est choisi dans l'ensemble consistant en H et CN; et $R_2$ est choisi dans l'ensemble consistant en un coupe $(S)CH(CH_3)C_6H_5$, $CH_2C_6H_5$, endo-norbornyle, 1-naphtyle, $CH(CH_3)$-c-$C_6H_{11}$, $CH(C_6H_5)_2$, c-$C_6H_{11}$, c-$C_7H_{13}$, c-$C_8H_{15}$, c-$C_9H_{17}$, c-$C_{10}H_{19}$, 2-décalyle, $SO_2C_6H_5$ et $SO_2$c-$C_7H_{13}$.

17. Composé selon la revendication 16, dans lequel $R_1$, $X_3$ et $X_5$ représentent chacun H; $X_4$ représente CN et $R_2$ représente un groupe c-$C_9H_{17}$, y compris leurs formes tautomères et leurs sels physiologiquement acceptables.

18. Composé selon la revendication 16, dans lequel $R_1$, $X_3$ et $X_5$ représentent chacun H ; $X_4$ représente CN et $R_2$ représente un groupe c-$C_8H_{15}$, y compris leurs formes tautomères et leurs sels physiologiquement acceptables.

19. Composé selon la revendication 16, dans lequel $R_1$ et $X_4$ représentent H, $X_3$ et $X_5$ représentent Cl et $R_2$ représente $(S)CH(CH_3)C_6H_5$, y compris leurs formes tautomères et leurs sels physiologiquement acceptables.

20. Composé selon la revendication 16, dans lequel $R_1$ et $X_5$ représentent chacun H ; $X_3$ représente Cl; $X_4$ représente CN et $R_2$ représente un groupe c-$C_8H_{15}$, y compris leurs formes tautomères et leurs sels physiologiquement acceptables.

21. Composé selon la revendication 16, dans lequel $R_1$ et $X_5$ représentent chacun H ; $X_3$ représente $CH_3$ ; $X_4$ représente CN et $R_2$ représente un groupe c-$C_8H_{15}$, y compris leurs formes tautomères et leurs sels physiologiquement acceptables.

22. Composé selon la revendication 16, dans lequel $R_1$ et $X_4$ représentent H, $X_3$ et $X_5$ représentent cha cun Cl et $R_2$ représente un groupe $CH_2C_6H_5$, y compris leurs formes tautomères et leurs sels physiologiquement acceptables.

23. Composé selon la revendication 16, dans lequel $R_1$ représente $CH_3$ ; $X_4$ représente H; $X_3$ et $X_5$ représentent chacun Cl, et $R_2$ représente $CH_2C_6H_5$, y compris leurs formes tautomères et leurs sels

physiologiquement acceptables.

**24.** Composé selon la revendication 16, dans lequel $R_1$ et $X_4$ représentent chacun H, $X_3$ et $X_5$ représentent chacun Cl et $R_2$ représentent (S)$CH(CH_3)$-c-$C_6H_{11}$, y compris leurs formes tautomères et leurs sels physiologiquement acceptables.

**25.** Composé selon la revendication 16, dans lequel $R_1$, $X_3$ et $X_5$ représentent chacun H, $X_4$ représente CN et $R_2$ représente (S)$CH(CH_3)C_6H_5$, y compris leurs formes tautomères et leurs sels physiologiquement acceptables.

**26.** Composé selon la revendication 16, dans lequel $R_1$, $X_3$ et $X_5$ représentent chacun H ; $X_4$ représente CN et $R_2$ représente $CH(C_6H_5)_2$, y compris leurs formes tautomères et leurs sels physiologiquement acceptables.

**27.** Composé selon la revendication 16, dans lequel $R_1$ et $X_4$ représentent H, $X_3$ et $X_5$ représentent Cl et $R_2$ représente un groupe c-$C_7H_{13}$, y compris leurs formes tautomères et leurs sels physiologiquement acceptables.

**28.** Composé selon la revendication 16, dans lequel $X_4$ représente CN ; $R_1$, $X_3$ et $X_5$ représentent chacun H, et $R_2$ représente un groupe c-$C_{10}H_{17}$, y compris leurs formes tautomères et leurs sels physiologiquement acceptables.

**29.** Composé selon la revendication 1, ce composé étant choisi dans l'ensemble formé par les sels physiologiquement acceptables consistant en un chlorhydrate, un phosphate, un citrate, un sulfate de sodium, de potassium, d'ammonium, de calcium et de magnésium.

**30.** Procédé pour édulcorer des produits comestibles, comprenant des aliments, des boissons, des produits de confiserie, des pâtes à mâcher ou chewing-gums, des produits pharmaceutiques, des préparations vétérinaires et des produits pour la toilette, des produits cosmétiques et d'hygiène, procédé caractérisé en ce qu'il comprend l'addition, à la substance, d'une quantité, efficace pour édulcorer, d'un composé selon la revendication 1.

**31.** Produits comestibles édulcorés selon le procédé de la revendication 30.

**32.** Compositions édulcorantes, caractérisées en ce qu'elles comprennent une quantité, efficace pour l'édulcoration, d'un composé selon la revendication 1 ainsi qu'un support, véhicule ou excipient physiologiquement acceptable.

**33.** Compositions édulcorantes selon la revendication 32, dans lesquelles l'excipient ou support est un agent volumique.

**34.** Compositions édulcorantes selon la revendication 32, dans lesquelles le support est choisi dans l'ensemble consistant en du polydextrose, de l'amidon ou de la fécule, des maltodextrins, de la cellulose, de la méthylcellulose, de la carboxyméthylcellulose, du maltitol, de l'hydroxyméthylcellulose, de la cellulose microcristalline, de l'alginate de sodium, des pectines, des gommes, du lactose, du maltose, du glucose, de la leucine, du glycérol, du mannitol, du sorbitol, du bicarbonate de sodium et les acides phosphorique, citrique, tartrique, fumarique, benzoïque, sorbique, propionique et leurs sels de sodium, de potassium et de calcium et des mélanges de deux ou plusieurs quelconques d'entre eux.

**35.** Composition édulcorante, comprenant :
(a) un composé selon la revendication 1 ; et
(b) un agent édulcorant différent.

**36.** Composition édulcorante selon la revendication 35, comprenant en outre un agent donnant du corps ou du volume, ou agent volumique.

**37.** Composition édulcorante selon la revendication 36, dans laquelle l'agent édulcorant différent est choisi dans l'ensemble consistant en du saccharose, des sirops de maïs, du fructose, de l'aspartame, de l'alitame, une dihydrochalcone de néohespéridine, du sirop de maïs à forte teneur en fructose, de l'isomaltulose hydrogéné, du stévioside, des sucres L, de la glycyrizine, du xylitol, de lacésulfame-K, de la saccharine (sel de sodium, de potassium ou de calcium), de l'acide cyclamique (sel de sodium, de potassium ou de calcium), du trichlorogalactosaccharose, de la monelline et de la thaumatine, et leurs mélanges.

**38.** Composé de formule :

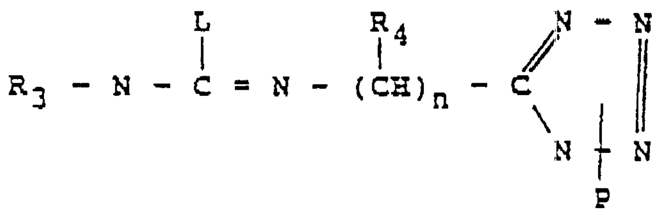

dans laquelle L représente un groupe S-alkyle, O-alkyle, $OSO_2$-aryle, $SO_3H$ ou un atome d'halogène ; P est un groupe protecteur, et $R_3$, $R_4$ et n sont tels que définis à la revendication 1.

**39.** Composé selon la revendication 38, dans lequel L représente un groupe S-méthyle.

**40.** Composé selon la revendication 38, dans lequel $R_3$ représente un groupe 4-cyanophényle, 3,5-dichlorophényle, 3,5-diméthylphényle, 6-indazolylimino ou phényle.

**41.** Composé selon la revendication 38, dans lequel P représente :

H,

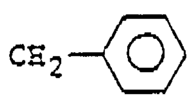 ,

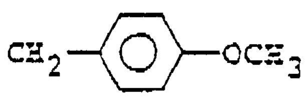 ,

$CH_2OCO\text{-}t\text{-}C_4H_9$ ou

$CH_2CH_2COOC_2H_5$.

**42.** Composé selon la revendication 40, dans lequel $R_4$ représente H.

**43.** Composé selon la revendication 42, dans lequel n vaut 1.

**44.** Composé selon la revendication 42, dans lequel n est nul.

**45.** Composé de formule :

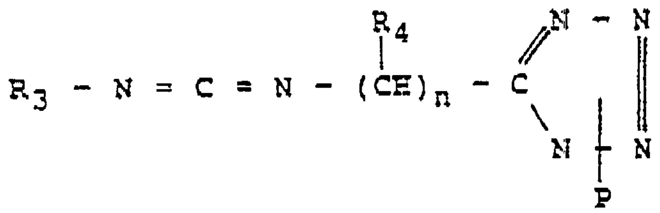

dans laquelle P représente un groupe protecteur et $R_3$, $R_4$ et n sont tels que définis à la revendication 1.

**46.** Composé selon la revendication 45, dans lequel $R_4$ représente H.

**47.** Composé selon la revendication 46, dans lequel $R_3$ représente un groupe phényle substitué.

**48.** Composé selon la revendication 47, dans lequel $R_3$ représente un groupe 4-cyanophényle, 3,5-dichlorophényle, 3,5-diméthylphényle ou phényle.

**49.** Composé selon la revendication 45, dans lequel P représente :

H,

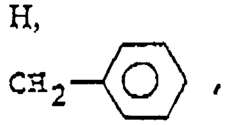

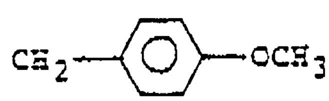

$CH_2OCO$-t-$C_4H_9$ ou

$CH_2CH_2COOC_2H_5$.

**50.** Composé selon la revendication 45, dans lequel n est nul.

**51.** Composé selon la revendication 45, dans lequel n vaut 1.

**52.** Composé selon la revendication 48, dans lequel $R_4$ et P représentent chacun H et n vaut 1.

**53.** Procédé comprenant :

(a) la réaction d'un composé de formule :

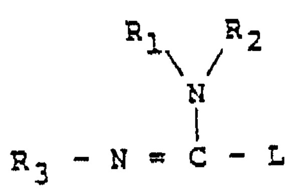

avec un composé de formule;

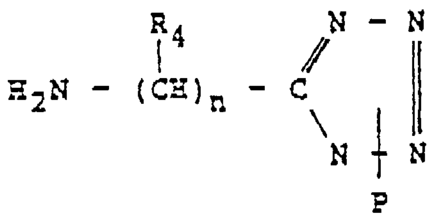

dans lesquelles L représente un groupe S-alkyle, O-alkyle, $OSO_2$-aryle, $SO_3H$ ou halogéno, et P est un groupe protecteur, et

$R_1$, $R_2$, $R_3$, $R_4$ et n sont tels que définis à la revendication 1, dans des conditions suffisantes pour former la guanidine correspondante, et

(b) la récupération de la guanidine formée en (a) ci-dessus.

**54.** Procédé selon la revendication 53, dans lequel P représente :

H,

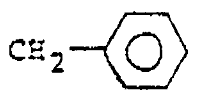 ,

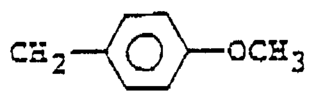 ,

$CH_2OCO$-t-$C_4H_9$ ou

$CH_2CH_2COOC_2H_5$.

**55.** Procédé selon la revendication 53, dans lequel L représente -S-$CH_3$.

**56.** Procédé selon la revendication 53, dans lequel $R_3$ représente un groupe phényle substitué et $R_1$ et $R_4$ représentent chacun H.

**57.** Procédé selon la revendication 56, dans lequel $R_2$ représente un groupe cyclohexyle, cyclooctyle, alpha-phénéthyle, diphénylméthyle, benzyle, phényle, endo-norbornyle, 1-naphtyle ou 2-décalyle.

**58.** Procédé comprenant :

(a) la réaction d'un carbodiimide de formule :

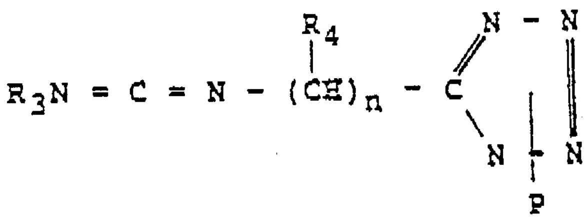

avec une amine de formule :

$HNR_1R_2$

formules dans lesquelles P représente un groupe protecteur; $R_1$, $R_2$, $R_3$, $R_4$ et n sont tels que définis à la revendication 1, dans des conditions suffisantes pour former la guanidine correspondante, et
(b) la récupération de la guanidine formée en (a) ci-dessus.

**59.** Procédé selon la revendication 58, dans lequel P représente :

H,

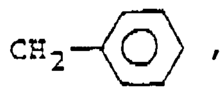

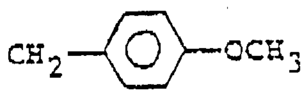

$CH_2OCO$-t-$C_4H_9$ ou
$CH_2CH_2COOC_2H_5$.

**60.** Procédé selon la revendication 58, dans lequel $R_1$ et $R_4$ représentent chacun H.

**61.** Procédé selon la revendication 60, dans lequel $R_3$ représente un groupe phényle substitué.

**62.** Procédé selon la revendication 61, dans lequel $R_3$ représente un groupe 4-cyanophényle, 3,5-dichlorophényle, 3,5-diméthylphényle ou phényle.

**63.** Procédé selon la revendication 60, dans lequel $R_2$ représente un groupe cyclohexyle, cyclooctyle, alpha-phénétyle, diphénylméthyle, benzyle, phényle, endo-norbornyle, 1-naphtyle ou 2-décalyle.

**64.** Procédé selon la revendication 63, dans lequel $R_3$ représente un groupe phényle substitué.

**65.** Procédé selon la revendication 64, dans lequel $R_3$ représente un groupe 4-cyanophényle, 3,5-dichlorophényle, 3,5-diméthylphényle ou phényle.

**66.** Procédé comprenant :
(a) la réaction d'un carbodiimide de formule :

$R_3$-N = C = N-$R_2$

avec une amine de formule :

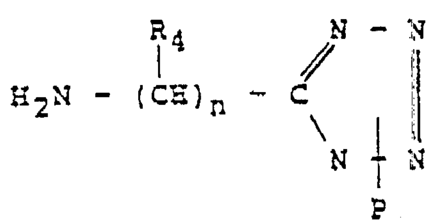

dans laquelle P représente un coupe protecteur ; $R_2$, $R_3$, $R_4$ et n sont tels que définis à la revendication 1, dans des conditions suffisantes pour former la guanidine correspondante, et
(b) la récupération de la guanidine formée en (a) ci-dessus.

**67.** Procédé selon la revendication 66, dans lequel P représente :

H,

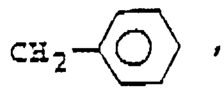

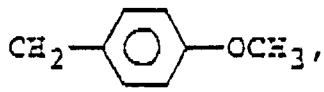

$CH_2OCO\text{-}t\text{-}C_4H_9$ ou
$CH_2CH_2COOC_2H_5$.

**68.** Procédé selon la revendication 66, dans lequel $R_4$ et P représentent chacun H.

**69.** Procédé selon la revendication 68, dans lequel $R_3$ représentent un groupe phényle substitué.

**70.** Procédé selon la revendication 69, dans lequel $R_3$ représente un groupe 4-cyanophényle, 3,5-dichlorophényle, 3,5-diméthylphényle.

**71.** Procédé selon la revendication 68, dans lequel $R_2$ représente un groupe phényle, benzyle, cyclohexyle, cyclooctyle, endo-norbornyle, 1-naphtyle, 2-décalyle, alpha-phénétyle ou diphénylméthyle.

**72.** Procédé selon la revendication 71, dans lequel $R_3$ représente un groupe phényle substitué.

**73.** Procédé selon la revendication 72, dans lequel $R_3$ représente un groupe 4-cyanophényle, 3,5-dichlorophényle, 3,5-diméthylphényle ou phényle.

**74.** Composé selon la revendication 1, répondant à la formule :

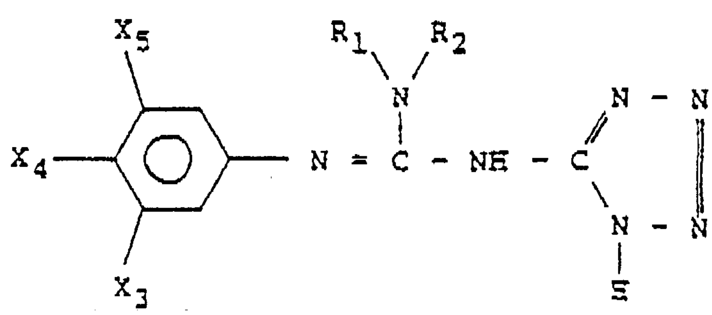

**75.** Composé selon la revendication 74, dans lequel $R_1$ représente H.

**76.** Composé selon la revendication 75, dans lequel $X_3$ et $X_5$ représentent chacun H, et $X_4$ représente CN.

**77.** Composé selon la revendication 76, dans lequel $R_2$ représente un groupe phényle, benzyle, cyclohexyle, cyclooctyle, cyclononyle, 4-pyrannyle, 2-décalyle, endobornyle, 1-naphtyle, alpha-phénétyle ou benzhydryle.

**78.** Composé selon la revendication 75, dans lequel $X_4$ représente H et $X_3$ et $X_5$ représentent chacun Cl ou $CH_3$.

**79.** Composé selon la revendication 78, dans lequel $R_2$ représente un coupe phényle, benzyle, cyclohexyle, cyclooctyle, cyclononyle, 4-pyrannyle, 2-décalyle, endo-norbornyle, 1-naphtyle, alpha-phénétyle ou benzhydryle.

**Revendications pour l'Etat contractant suivant : ES**

**1.** Procédé pour préparer un composé de formule :

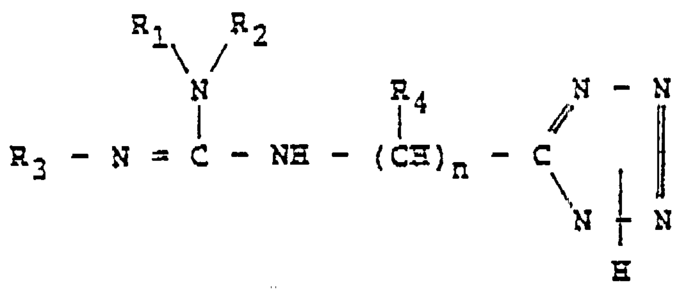

dans laquelle $R_3$ représente des pyridines, thiazoles, indoles, quinoléines, naphtyridines, cinnolines, ptéridines, thiophènes, benzothiophènes, naphtothiophènes, thianthrènes, furannes, pyrannes, isobenzofurannes, chromènes, xanthènes, phénoxathines, pyrroles, indoles, isoindoles, indolizines, pyridazines, pyrimidines, pyrazines, pyrazoles, imidazoles, pyrroles, indazoles, purines, quinolizines, isoquinoléines, quinoléines, phtalazines, quinoxalines quinazolines, carbazoles, carbolines, phénanthridines, acridines, pyrimidines, phénanthrolines, phénazines, phénarsazines, isothiazoles, phénothiazines, isothiazoles, thiazoles, furazannes (portant éventuellement des substituants) et des hétérocycliques répondant aux formules suivantes :

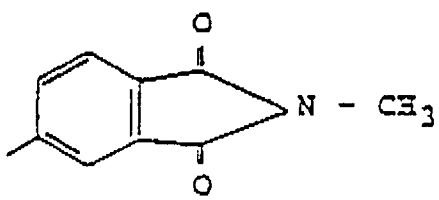

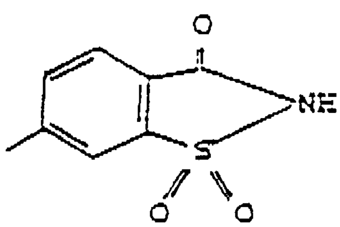

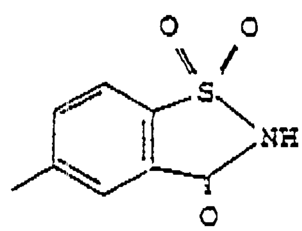

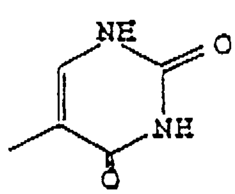

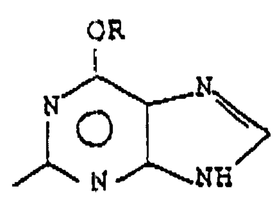

dans lesquels R représente H ou un groupe alkyle en $C_1$ à $C_6$, et les fragments hétérocycliques peuvent être éventuellement substitués par un ou plusieurs substituants choisis parmi un groupe alkyle en $C_1$ à $C_6$, un atome d'halogène, un groupe $NO_2$, CN, trihalogénométhyle, dihalogénométhyle,

hydroxyle ou hydroxyalkyle, ou bien un groupe phényle éventuellement substitué répondant à la formule :

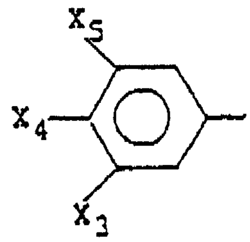

dans laquelle $X_3$, $X_4$ et $X_5$ sont identiques ou différents et sont choisis dans l'ensemble consistant en :

H,
Br,
$CF_3$,
$CF_2CF_3$,
$CH_2CF_3$,
Alkyle en $C_1$ à $C_4$,
$CH = NOCH_3$,
$CH = NOH$,
CHO,
$CH_2OCH_3$,
$CH_2OH$,
Cl,
CN,
$COCF_3$,
CO alkyle (en $C_1$ à $C_3$),
$CONH_2$,
CONH alkyle (en $C_1$ à $C_3$),
CON (chaque alkyle en $C_1$ à $C_3$),
COOC alkyle (en $C_1$ à $C_3$),
COOH,
F,
I,
$NH_2$,
NH alkyle (en $C_1$ à $C_3$)
N (alkyle)$_2$, chaque alkyle de $C_1$ à $C_3$,
NHCHO,
$NHCOCH_3$,
$NHCONH_2$,
$NHSO_2CH_3$,
$NO_2$,
O alkyle (en $C_1$ à $C_3$),
$C(O)CH_3$,
OH,
S alkyle (en $C_1$ à $C_3$),
SO alkyle (en $C_1$ à $C_3$),
$SO_2$ alkyle (en $C_1$ à $C_3$),
$SO_2NH_2$,
$SO_2NH$ alkyle en ($C_1$ à $C_3$),
$SO_2N$ (alkyle)$_2$, (chaque alkyle en $C_1$ à $C_3$),
$SO_3H$,

ou dans laquelle les substituants sur les positions représentées par $X_4$ et $X_5$ forment un noyau condensé ;

$R_1$ représente un atome d'hydrogène ou bien $R_1$ représente un groupe hydrocarbyle en $C_1$ à $C_4$, saturé, insaturé acyclique, cyclique ou hydrocarbyle mixte ou hydrocarbyle modifié et, dans le groupe hydrocarbyle modifié,

1 à 2 atomes de carbone peuvent être remplacés par 1 à 2 hétéroatomes, identiques ou différents, choisis chacun dans l'ensemble consistant en N, O, S, Cl, Br et I,

et 1 à 3 atomes d'hydrogène peuvent être remplacés par 1 à 3 atomes de fluor, d'oxygène ou d'azote,

$R_2$ représente un atome d'hydrogène, un groupe CN, $NO_2$, ou un groupe hydrocarbyle en $C_1$ à $C_{20}$, saturé, insaturé, acyclique, cyclique ou mixte ou un groupe hydrocarbyle modifié et, dans le groupe hydrocarbyle modifié,

1 à 4 atomes de carbone peuvent être remplacés par 1 à 4 hétéroatomes, identiques ou différents, choisis chacun dans l'ensemble consistant en N, O, S, Cl, Br et I,

et 1 à 5 atomes d'hydrogène peuvent être remplacés par 1 à 5 atomes de fluor, d'oxygène ou d'azote,

n vaut 0, 1, 2 ou 3,

$R_1$ et $R_2$ peuvent être condensés, fusionnés,

$R_4$ représente H ou un groupe alkyle en $C_1$ à $C_6$, à la condition que $R_4$ ne puisse représenter un groupe alkyle sur un seul atome de carbone que lorsque n vaut 2 ou 3, et

leurs tautomères et sels physiologiquement acceptables,

procédé caractérisé en ce que :

(a) on fait réagir un composé de formule :

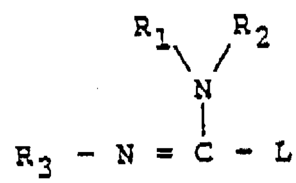

avec un composé de formule :

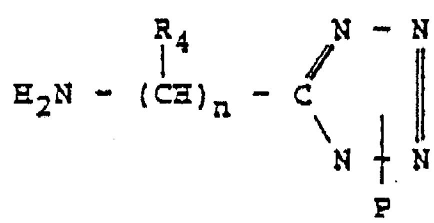

formule dans lesquels L représente un groupe S-alkyle, O-alkyle, $OSO_2$-aryle, $SO_3H$ ou un atome d'halogène et P représente un groupe protecteur, et

$R_1$, $R_2$, $R_3$, $R_4$ et n sont tels que définis à la revendication 1, dans des conditions suffisantes pour former la guanidine correspondante, et

(b) on récupère la guanidine formée en (a) ci-dessus,

et l'on transforme éventuellement le composé ainsi obtenu, de façon connue, en ses sels respectifs, physiologiquement acceptables.

**2.** Procédé pour préparer un composé selon la revendication 1, caractérisé en ce que :

(a) on fait réagir un carbodiimide de formule :

$R_3$-N = C = N-$R_2$

avec une amine de formule :

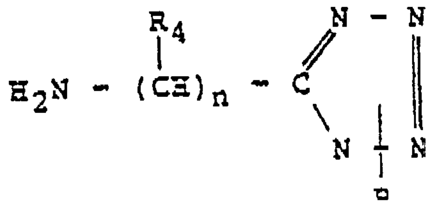

formule dans lesquels P représente un groupe protecteur; $R_2$, $R_3$, $R_4$ et n sont tels que définis à la revendication 1, dans des conditions suffisantes pour former la guanidine correspondante, et

(b) on récupère la guanidine formée en (a) ci-dessus,

et éventuellement on transforme de façon connue le composé ainsi obtenu en ses sels respectifs physiologiquement acceptables.

**3.** Procédé pour préparer un composé selon la revendication 1, caractérisé en ce que :

(a) on fait réagir un carbodiimide de formule :

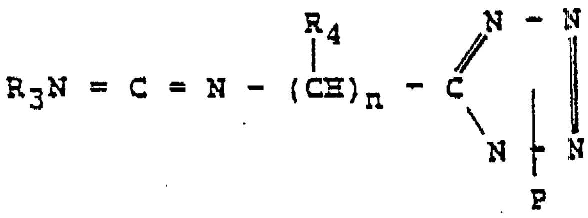

avec une amine de formule :

$HNR_1R_2$

formule dans lesquels P représente un groupe protecteur ; $R_1$, $R_2$, $R_3$, $R_4$ sont tels que définis à la revendication 1, dans des conditions suffisantes pour former la guanidine correspondante, et

(b) on récupère la guanidine formée en (a) ci-dessus,

et éventuellement on transforme de façon connue le composé ainsi obtenu en ses sels respectifs, physiologiquement acceptables.

**4.** Procédé selon l'une quelconque des revendications 1 à 3, caractérisé en ce que l'on prépare les sels physiologiquement acceptables en transformant les produits obtenus en le chlorhydrate, le phosphate, le citrate, le sulfate de sodium, de potassium, d'ammonium, de calcium ou de magnésium.

**5.** Procédé pour édulcorer des produits comestibles, comprenant des aliments, des boissons, des produits de confiserie, des chewing-gums, des préparations pharmaceutiques, vétérinaires et des produits de toilette, cosmétiques et d'hygiène, procédé caractérisé en ce qu'il comprend l'addition, à la substance, d'une quantité efficace pour une édulcoration, d'un composé tel que préparé selon la revendication 1.

**6.** Utilisation d'une quantité édulcorante efficace d'un composé tel que préparé à la revendication 1 et d'un excipient, véhicule ou support physiologiquement acceptable de ce composé, pour préparer des compositions édulcorantes.

**7.** Utilisation selon la revendication 6, dans laquelle le support ou véhicule est un agent volumique.

**8.** Utilisation selon la revendication 6, dans laquelle le support ou véhicule est choisi dans l'ensemble consistant en du polydextrose, de la fécule ou de l'amidon, des maltodextrines, de la cellulose, de la méthylcellulose, de la carboxyméthylcellulose, du maltitol, de l'hydroxyméthylcellulose, de la cellulose microcristalline, de l'alginate de sodium, des pectines, des gommes, du lactose, du maltose, du glucose, de la leucine, du glycérol, du mannitol, du sorbitol, du bicarbonate de sodium et des acides phosphorique, citrique, tartrique, fumarique, benzoïque, sorbique, propionique, et leurs sels de sodium,

de potassium et de calcium, et des mélanges de n'importe quel support précité.

**9.** Utilisation de :
(a) un composé tel que préparé selon la revendication 1 ; et
(b) un agent édulcorant différent,
pour préparer une composition édulcorante.

**10.** Utilisation selon la revendication 9, dans laquelle la composition comprend en outre un agent donnant du corps ou du volume (agent volumique).

**11.** Utilisation selon la revendication 10, dans laquelle l'agent édulcorant différent est choisi dans l'ensemble consistant en le saccharose, des sirops de maïs, du fructose, de l'aspartame, de l'alitame, de la dihydrochalcone de néohespéridine, du sirop de maïs à forte teneur en fructose, de l'isomaltulose hydrogéné, du stévioside, des sucres L, de la glycyrrhizine, du xylitol, de l'acésulfame K, de la saccharine (sel de sodium, de potassium ou de calcium), de l'acide cyclamique (sel de sodium, de potassium ou de calcium), du trichlorogalactosusaccharose, de la monelline et de la thaumatine, et leurs mélanges.

FIGURE 1

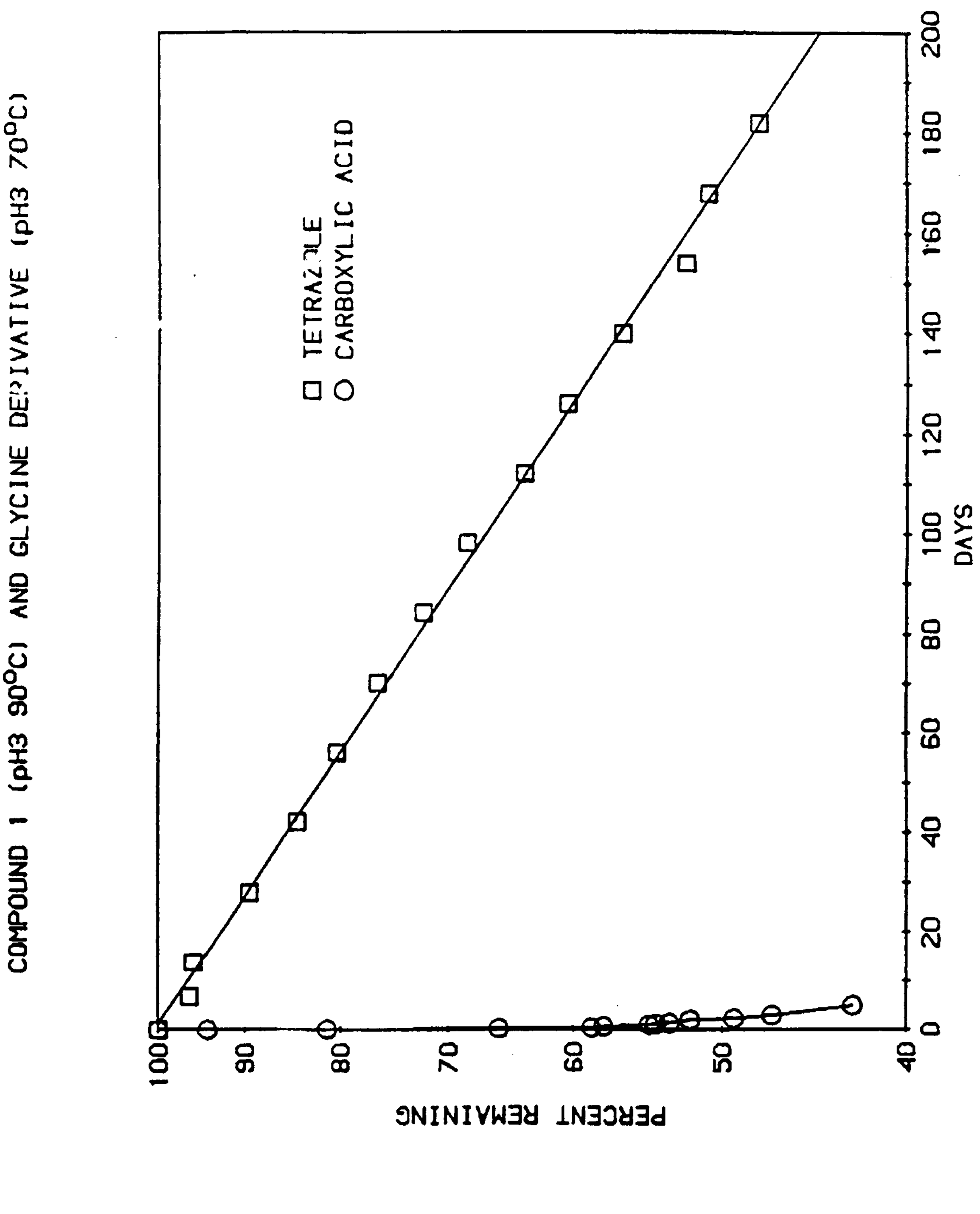
COMPOUND 1 (pH3 90°C) AND GLYCINE DERIVATIVE (pH3 70°C)
TETRAZOLE
CARBOXYLIC ACID
PERCENT REMAINING
100
90
80
70
60
50
40
DAYS
0
20
40
60
80
100
120
140
160
180
200